# EUROPEAN PATENT SPECIFICATION

(11) **EP 4 270 008 B1**
(45) Date of publication and mention of the grant of the patent: **04.02.2026**
(21) Application number: 23182542.3
(22) Date of filing: 01.12.2020
(51) Int. Cl.: G01N 33/487, G01N 33/68

(54) **METHOD OF CHARACTERISING A TARGET POLYPEPTIDE USING A NANOPORE**
VERFAHREN ZUR CHARAKTERISIERUNG EINES ZIELPOLYPEPTIDS UNTER VERWENDUNG EINER NANOPORE
PROCÉDÉ DE CARACTÉRISATION D'UN POLYPEPTIDE CIBLE À L'AIDE D'UN NANOPORE

(30) Priority: 02.12.2019 GB 201917599; 30.09.2020 GB 202015479
(43) Date of publication of application: 01.11.2023
(62) Divisional of application: 20825190.0
(73) Proprietor: Oxford Nanopore Technologies PLC, Oxford OX4 4DQ (GB)
(72) Inventor: HERON, Andrew John, Oxford, OX4 4DQ (GB); GRAHAM, James Edward, Oxford, OX4 4DQ (GB); STRYCHARSKA, Melania Slawa, Oxford, OX4 4DQ (GB)
(74) Representative: J A Kemp LLP

(56) References cited:
- EP-A1- 2 886 663
- WO-A1-2013/016486
- WO-A1-2014/013260
- WO-A1-2015/051378
- WO-A2-2013/123379
- RESTREPO-P�REZ LAURA ET AL: "Paving the way to single-molecule protein sequencing", NATURE NANOTECHNOLOGY, NATURE PUB. GROUP, INC, LONDON, vol. 13, no. 9, 6 September 2018 (2018-09-06), pages 786 - 796, XP036583049, ISSN: 1748-3387, [retrieved on 20180906], DOI: 10.1038/S41565-018-0236-6
- JONATHAN M CRAIG ET AL: "Determining the effects of DNA sequence on Hel308 helicase translocation along single-stranded DNA using nanopore tweezers", NUCLEIC ACIDS RESEARCH, vol. 47, no. 5, 15 January 2019 (2019-01-15), GB, pages 2506 - 2513, XP055660955, ISSN: 0305-1048, DOI: 10.1093/nar/gkz004

## Description

### Field

The present disclosure relates to methods of characterising a target polypeptide by forming a conjugate of the target polypeptide with a polynucleotide and using a polynucleotide-handling protein which is modified by covalently closing a polynucleotide-unbinding opening through which a polynucleotide strand may unbind to control the movement of the conjugate with respect to a nanopore. The disclosure also relates to kits for carrying out such methods.

### Background

The characterisation of biological molecules is of increasing importance in biomedical and biotechnological applications. For example, sequencing of nucleic acids allows the study of genomes and the proteins they encode and, for example, allows correlation between nucleic acid mutations and observable phenomena such as disease indications. Nucleic acid sequencing can be used in evolutionary biology to study the relationship between organisms. Metagenomics involves identifying organisms present in samples, for example microbes in a microbiome, with nucleic acid sequencing allowing the identification of such organisms. Whilst techniques to characterise (e.g. sequence) polynucleotides have been extensively developed, techniques to characterise polypeptides are less advanced, despite being of very significant biotechnological importance. For example, knowledge of a protein sequence can allow structure-activity relationships to be established and has implications in rational drug development strategies for developing ligands for specific receptors. Identification of post-translational modifications is also key to understanding the functional properties of many proteins. For example, typically 30-50% of protein species are phosphorylated in eukaryotes. Some proteins may have multiple phosphorylation sites, serving to activate or inactivate a protein, promote its degradation, or modulate interactions with protein partners. There is thus a pressing need for methods to characterise proteins and other polypeptides.

Known methods of characterising polypeptides include mass spectrometry and Edman degradation.

Protein mass spectrometry involves characterising whole proteins or fragments thereof in an ionised form. Known methods of protein mass spectrometry include electrospray ionisation (ESI) and matrix-assisted laser desorption/ionisation (MALDI). Mass spectrometry has some benefits, but results obtained can be affected by the presence of contaminants and it can be difficult to process fragile molecules without their fragmentation. Moreover, mass spectrometry is not a single molecule technique and provides only bulk information about the sample interrogated. Mass spectrometry is unsuitable for characterising differences within a population of polypeptide samples and is unwieldy when seeking to distinguish neighbouring residues.

Edman degradation is an alternative to mass spectrometry which allows the residue-by-residue sequencing of polypeptides. Edman degradation sequences polypeptides by sequentially cleaving the N-terminal amino acid and then characterising the individually cleaved residues using chromatography or electrophoresis. However, Edman sequencing is slow, involves the use of costly reagents, and like mass spectrometry is not a single molecule technique.

As such, there remains a pressing need for new techniques to characterise polypeptides, especially at the single molecule level. Single molecule techniques for characterising biomolecules such as polynucleotides have proven to be particularly attractive due to their high fidelity and avoidance of amplification bias.

One attractive method of single molecule characterization of biomolecules such as polypeptides is nanopore sensing. Nanopore sensing is an approach to analyte detection and characterization that relies on the observation of individual binding or interaction events between the analyte molecules and an ion conducting channel. Nanopore sensors can be created by placing a single pore of nanometre dimensions in an electrically insulating membrane and measuring voltage-driven ion currents through the pore in the presence of analyte molecules. The presence of an analyte inside or near the nanopore will alter the ionic flow through the pore, resulting in altered ionic or electric currents being measured over the channel. The identity of an analyte is revealed through its distinctive current signature, notably the duration and extent of current blocks and the variance of current levels during its interaction time with the pore. Nanopore sensing has the potential to allow rapid and cheap polypeptide characterisation.

Nanopore sensing and characterisation of polypeptides has been proposed in the art. For example, WO 2013/123379 discloses the use of an NTP-driven protein processing unfoldase enzyme to process a protein to be translocated through a nanopore. However, there remains a need for alternative and/or improved methods of characterising polypeptides.

Restrepo-Pérez, L. et al.; Nat Nanotechnol (2018, 13(9):786-796) discloses a method of characterising a protein using measurements obtained at its transition through a nanopore, wherein the driving force for said transition is provided by an oligonucleotide conjugated to the protein.

### Summary

The disclosure relates to methods of characterising a target polypeptide. The methods comprise conjugating the target polypeptide to a polynucleotide to form a polypeptide-polynucleotide conjugate. The methods comprise contacting the conjugate with a polynucleotide-handling protein. The polynucleotide-handling protein is capable of controlling the movement of the polynucleotide with respect to a nanopore. One or more measurements characteristic of the polypeptide are taken as the conjugate moves with respect to the nanopore. In this manner, the target polypeptide which is comprised in the conjugate is characterised.

Accordingly, provided herein is a method of characterising a target polypeptide, comprising
- conjugating the target polypeptide to a polynucleotide to form a polynucleotide-polypeptide conjugate;
- contacting the conjugate with a polynucleotide-handling protein capable of controlling the movement of the polynucleotide with respect to a nanopore; and
- taking one or more measurements characteristic of the polypeptide as the conjugate moves with respect to the nanopore,
thereby characterising the polypeptide;
wherein the polynucleotide-handling protein is modified by covalently closing a polynucleotide-unbinding opening through which a polynucleotide strand may unbind, thereby preventing the polynucleotide binding protein from disengaging from the conjugate when the polynucleotide-handling protein contacts a portion of the conjugate comprising a polypeptide.

In some embodiments, the nanopore has a constriction region. In some embodiments the nanopore is modified to extend the distance between the polynucleotide-handling protein and a constriction region of the nanopore. In some embodiments the polynucleotide-handling protein is separated from the nanopore using a displacer unit, thereby extending the distance between the active site of the polynucleotide-handling protein and the nanopore. In some embodiments, the displacer unit comprises one or more proteins. In some embodiments, the polynucleotide-handling protein is modified to extend the distance from the active site of the polynucleotide-handling protein to the nanopore.

In some embodiments the polynucleotide-handling protein is capable of remaining bound to the conjugate when the portion of the conjugate in contact with the active site of the polynucleotide-handling protein comprises a polypeptide. In some embodiments the polynucleotide-handling protein is a helicase.

In some embodiments the conjugate comprises a plurality of polypeptide sections and/or a plurality of polynucleotide sections.

In some embodiments the polypeptide has a length of from 2 to about 50 peptide units. In some embodiments the polypeptide is held in a linearized form.

In some embodiments the polynucleotide has a length of from about 10 to about 1000 nucleotides. In some embodiments one or more adapters and/or one or more tethers and/or one or more anchors are attached to the polynucleotide in the conjugate.

In some embodiments of the disclosed methods,
i) the polynucleotide-handling protein is located on the *cis* side of the nanopore and the polynucleotide-handling protein controls the movement of the conjugate from the *cis* side of the nanopore to the *trans* side of the nanopore; or
ii) the polynucleotide-handling protein is located on the *trans* side of the nanopore and the polynucleotide-handling protein controls the movement of the conjugate from the *trans* side of the nanopore to the *cis* side of the nanopore. In some embodiments, the polynucleotide-handling protein is located on the *cis*
side of the nanopore and the polynucleotide-handling protein controls the movement of the polynucleotide from the *cis* side of the nanopore to the *trans* side of the nanopore, thereby controlling the movement of the polypeptide through the nanopore. In some embodiments, the polynucleotide-handling protein is located on the *trans* side of the nanopore and the polynucleotide-handling protein controls the movement of the polynucleotide from the *trans* side of the nanopore to the *cis* side of the nanopore, thereby controlling the movement of the polypeptide through the nanopore.

In some embodiments the conjugate comprises one or more structures of the form L-{P-N}-P*ₘ*, wherein:
- L is a leader, wherein L is optionally an N moiety;
- P is a polypeptide;
- N comprises a polynucleotide; and
- m is 0 or 1;
and the method comprises threading the leader (L) through the nanopore thereby contacting the polypeptide (P) with the nanopore; and
i) the polynucleotide-handling protein is located on the *cis* side of the nanopore and the method comprises allowing the polynucleotide-handling protein to control the movement of the polynucleotide moiety (N) from the *cis* side of the nanopore to the *trans* side of the nanopore, thereby controlling the movement of the polypeptide (P) through the nanopore; or
ii) the polynucleotide-handling protein is located on the *trans* side of the nanopore and the method comprises allowing the polynucleotide-handling protein to control the movement of the polynucleotide moiety (N) from the *trans* side of the nanopore to the *cis* side of the nanopore, thereby controlling the movement of the polypeptide (P) through the nanopore.

In some embodiments the conjugate comprises one or more structures of the form L-P₁-N-{P-N}*ₙ*-P*ₘ* , wherein:
- *n* is a positive integer;
- L is a leader, wherein L is optionally an N moiety;
- each P, which may be the same or different, is a polypeptide;
- each N, which may be the same or different, comprises a polynucleotide; and
- *m* is 0 or 1;
and the method comprises threading the leader (L) through the nanopore thereby contacting polypeptide (P₁) with the nanopore, and
i) the polynucleotide-handling protein is located on the *cis* side of the nanopore and the method comprises allowing the polynucleotide-handling protein to control the movement of each polynucleotide (N) sequentially from the *cis* side of the nanopore to the *trans* side of the nanopore, thereby controlling the movement of each polypeptide (P) sequentially through the nanopore; or
ii) the polynucleotide-handling protein is located on the *trans* side of the nanopore and the method comprises allowing the polynucleotide-handling protein to control the movement of each polynucleotide (N) sequentially from the *trans* side of the nanopore to the *cis* side of the nanopore, thereby controlling the movement of each polypeptide (P) sequentially through the nanopore

In some embodiments of the disclosed methods,
i) the polynucleotide-handling protein is located on the *cis* side of the nanopore and the polynucleotide-handling protein controls the movement of the conjugate from the *trans* side of the nanopore to the *cis* side of the nanopore; or
ii) the polynucleotide-handling protein is located on the *trans* side of the nanopore and the polynucleotide-handling protein controls the movement of the conjugate from the *cis* side of the nanopore to the *trans* side of the nanopore.

In some embodiments the polynucleotide-handling protein is located on the *cis* side of the nanopore and the polynucleotide-handling protein controls the movement of the polynucleotide from the *trans* side of the nanopore to the *cis* side of the nanopore, thereby controlling the movement of the polypeptide through the nanopore. In some embodiments the polynucleotide-handling protein is located on the *trans* side of the nanopore and the polynucleotide-handling protein controls the movement of the polynucleotide from the *cis* side of the nanopore to the *trans* side of the nanopore, thereby controlling the movement of the polypeptide through the nanopore.

In some embodiments the conjugate comprises one or more structures of the form L-{P-N}-P*ₘ*, wherein:
- L is a leader, wherein L is optionally an N moiety;
- P is a polypeptide;
- N comprises a polynucleotide;
- *m* is 0 or 1;
and the method comprises threading the leader (L) through the nanopore thereby contacting the polypeptide (P) with the nanopore, and
i) the polynucleotide-handling protein is located on the *cis* side of the nanopore and the method comprises allowing the polynucleotide-handling protein to control the movement of the polynucleotide (N) from the *trans* side of the nanopore to the *cis* side of the nanopore, thereby controlling the movement of the polypeptide (P) through the nanopore; or
i) the polynucleotide-handling protein is located on the *trans* side of the nanopore and the method comprises allowing the polynucleotide-handling protein to control the movement of the polynucleotide (N) from the *cis* side of the nanopore to the *trans* side of the nanopore, thereby controlling the movement of the polypeptide (P) through the nanopore

In some embodiments the conjugate comprises a blocking moiety attached to the polypeptide via an optional linker, and the method comprises
i) contacting the conjugate with the nanopore such that the blocking moiety is on the opposite side of the nanopore to the polynucleotide-handling protein;
ii) contacting the polynucleotide of the conjugate with the polynucleotide-handling protein;
iii) allowing the polynucleotide-handling protein to control the movement of the polynucleotide with respect to the nanopore thereby controlling the movement of the polypeptide through the nanopore;
iv) when the blocking moiety contacts the nanopore thereby preventing further movement of the conjugate through the nanopore, allowing the polynucleotide-handling protein to transiently unbind from the polynucleotide so that the conjugate moves through the nanopore under an applied force in a direction opposite to the direction of movement controlled by the polynucleotide-handling protein; and
v) optionally repeating steps (ii) to (iv) to oscillate the polypeptide through the nanopore.

In some embodiments the one or more measurements are characteristic of one or more characteristics of the polypeptide selected from (i) the length of the polypeptide, (ii) the identity of the polypeptide, (iii) the sequence of the polypeptide, (iv) the secondary structure of the polypeptide and (v) whether or not the polypeptide is modified.

Also provided is a kit comprising:
- a nanopore comprising a constriction region, wherein the nanopore is a transmembrane protein pore;
- a polynucleotide comprising a reactive functional group for conjugating to a target polypeptide; and
- a polynucleotide-handling protein capable of controlling the movement of the polynucleotide with respect to the nanopore;
wherein the polynucleotide-handling protein is modified by covalently closing a polynucleotide-unbinding opening through which a polynucleotide strand may unbind, thereby preventing the polynucleotide binding protein to prevent it from disengaging from the conjugate when the polynucleotide-handling protein contacts a portion of the conjugate comprising a polypeptide.

In some embodiments, (i) said nanopore is modified to increase the distance between the constriction region and the polynucleotide-handling protein when the polynucleotide-handling enzyme is in contact with the nanopore; and/or (ii) said kit further comprises one or more displacer units for extending the distance between the nanopore and the active site of the polynucleotide-handling protein. In some embodiments the nanopore, polynucleotide and/or polynucleotide-handling protein, and optionally the one or more displacer units if present are as defined herein.

### Brief Description of the Figures

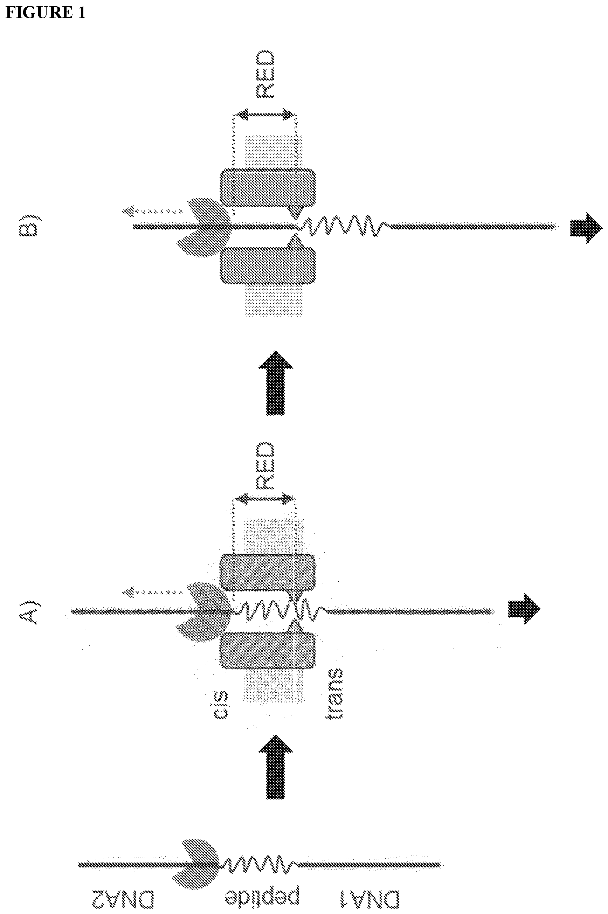

**Figure 1**. Schematic showing a non-limiting example of an embodiment of the disclosed methods in which a polynucleotide-handling protein at the *cis* side of a nanopore controls the movement of a conjugate comprising a polynucleotide (DNA2) conjugated to a polypeptide from the *cis* side of a nanopore to the *trans* side of the nanopore, thus allowing the polypeptide to be characterised as it moves with respect to the nanopore. As shown an optional leader (DNA1) is attached to the conjugate to facilitate the threading of the polypeptide through the nanopore. The RED (discussed herein) is shown as the notional distance between a constriction in the nanopore and the active site of the polynucleotide-handling protein. (A) The substrate can be captured in the nanopore e.g. from the *cis* side of the membrane by the application of e.g. a positive voltage to the trans side of the membrane. A polynucleotide-handling protein moves along the polynucleotide section in the direction shown by the dotted arrow and feeds the substrate into the pore, proceeding to state (B). As the polynucleotide-handling protein moves along the polynucleotide (e.g. in 1 nucleotide fuel-driven steps) it feeds the conjugate into nanopore, and the peptide section passes through the nanopore.

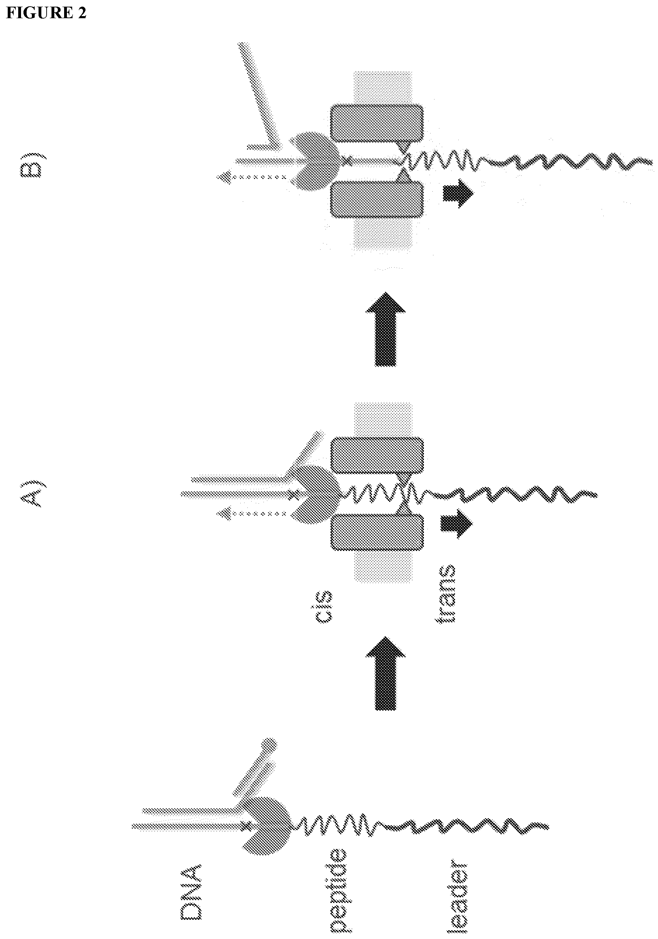

**Figure 2**. Schematic showing an embodiment of the general setup shown in Figure 1. In this non-limiting example, the polynucleotide-handling protein is initially stalled at a spacer (X) in the polynucleotide portion of the conjugate (DNA). An adapter is attached to the polynucleotide portion of the conjugate and has a tether attached thereto to localise the conjugate in the membrane in the region of a nanopore for characterisation. Steps (A) and (B) are as described for Figure 1. As the polynucleotide-handling protein processes the polynucleotide it may displace the adapter.

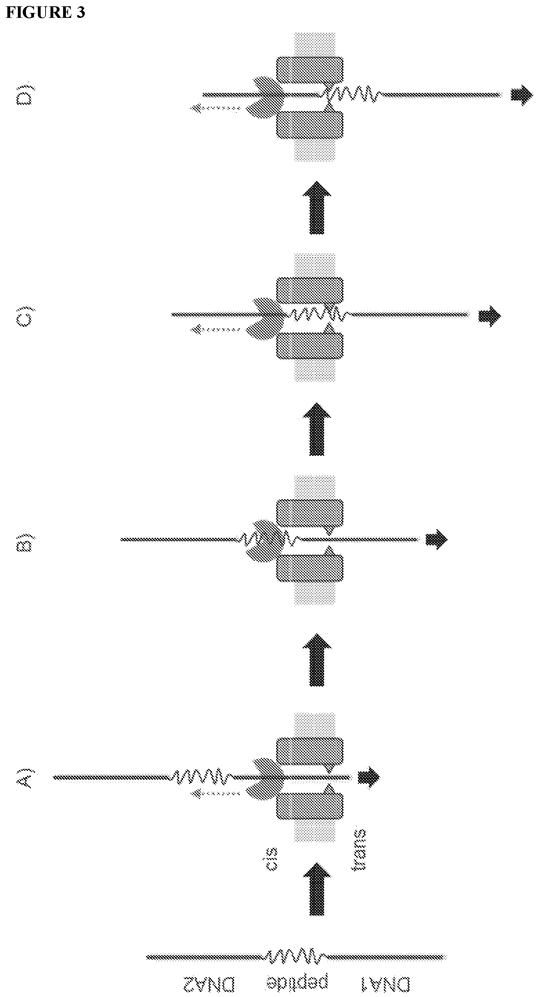

**Figure 3**. Schematic showing a further embodiment of the general setup shown in Figure 1. In this non-limiting example the conjugate comprises multiple polynucleotide and polypeptide sections which are sequentially moved through the nanopore under the control of the polynucleotide-handling protein. As shown the polynucleotide-handling protein is initially loaded onto a first polynucleotide portion of the conjugate (DNA1) and moves that portion of the conjugate through the nanopore. The polynucleotide-handling protein passes the first polypeptide section of the conjugate through the nanopore without dissociating from the conjugate. The polynucleotide-handling protein then contacts a second polynucleotide portion of the conjugate (DNA2) and controls its movement through the nanopore. Further polypeptide and polynucleotide portions (not shown) can be similarly sequentially moved with respect to the nanopore.

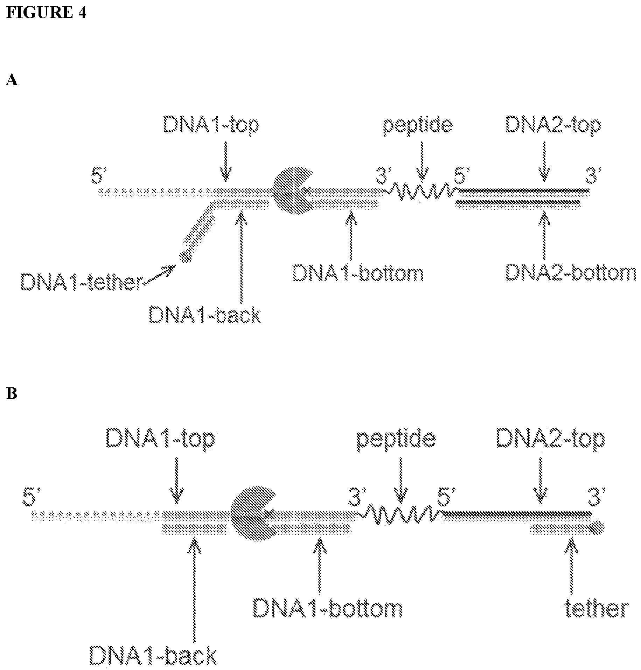

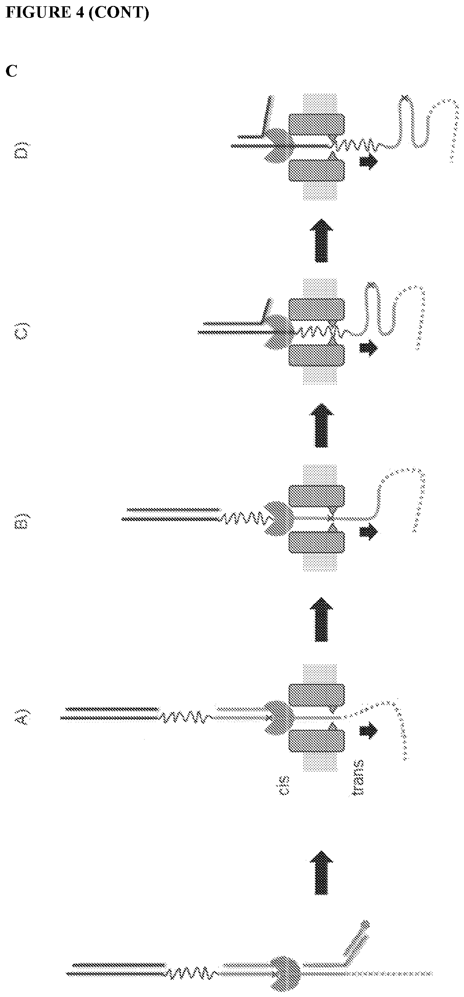

**Figure 4**. Schematic showing a non-limiting example of a substrate for use in the embodiment described in Figure 3. A: A first polynucleotide portion of the conjugate (DNA1) comprises a sequencing Y adapter with a leader (dotted) to facilitate capture in the nanopore; a tether to enable tethering to a membrane for localising the conjugate in the region of the nanopore; and a polynucleotide-handling protein stalled by a spacer (X). As shown the polynucleotide portion of the conjugate comprises double stranded DNA. **B**: A variation of the embodiment of the substrate shown in (A); the tether (or an additional tether) can be located on a second polynucleotide portion of the conjugate (DNA2). The notation "top" and "bottom" is purely for ease of comprehension. **C:** Schematic showing the methods of the invention using the substrate shown in 4(A).

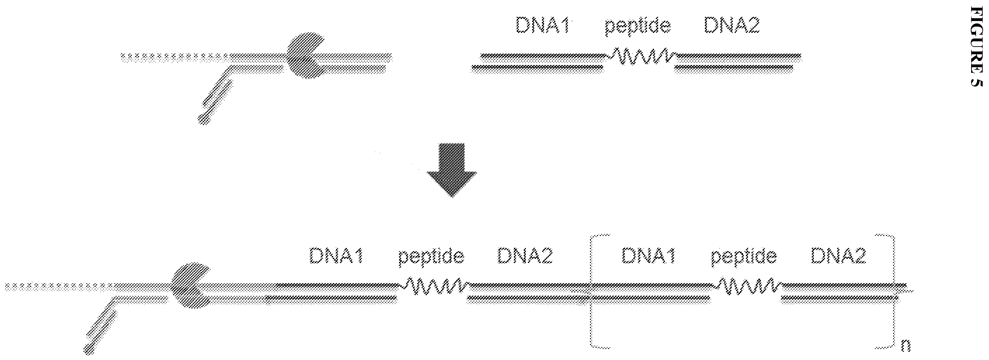

**Figure 5**. Schematic showing further non-limiting examples of substrates for use in the disclosed methods. The conjugate may comprise multiple polynucleotide and polypeptide sections (n>0) which may be sequentially processed by the polynucleotide-handling protein for characterisation by the nanopore as described herein.

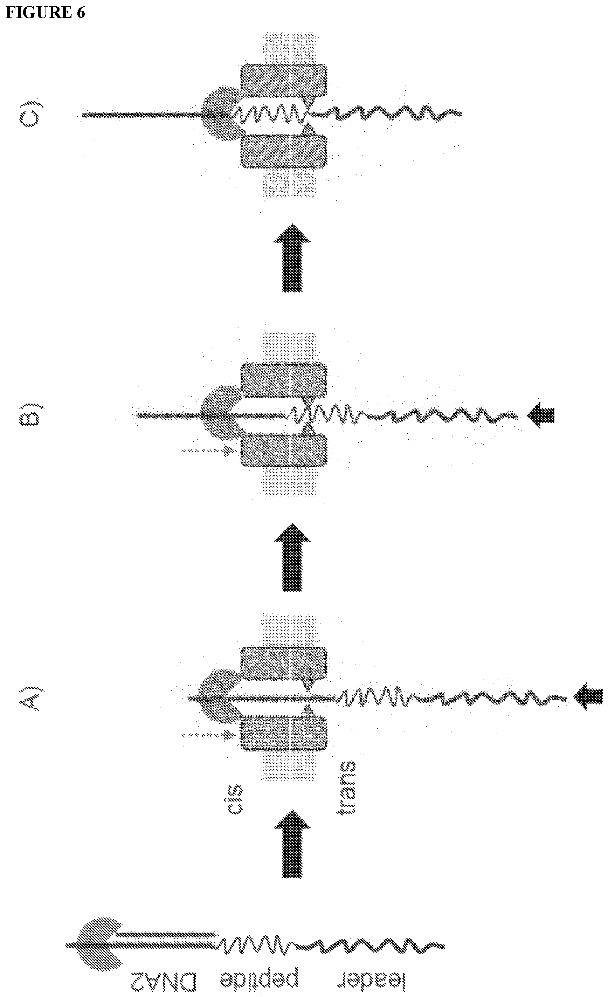

**Figure 6**. Schematic showing a non-limiting example of an embodiment of the disclosed methods in which a polynucleotide-handling protein at the *cis* side of the nanopore controls the movement of a conjugate comprising a polynucleotide (DNA2) conjugated to a polypeptide from the *trans* side of a nanopore to the *cis* side of the nanopore, thus allowing the polypeptide to be characterised as it moves with respect to the nanopore. As shown an optional leader is attached to the conjugate to facilitate the initial threading of the polypeptide through the nanopore. (A) The substrate can be captured in the nanopore e.g. from the *cis* side of the membrane by the application of e.g. a positive voltage to the trans side of the membrane. A polynucleotide-handling protein moves along the polynucleotide section in the direction shown by the dotted arrow to move the substrate out of the pore, proceeding to state (B). As the polynucleotide-handling protein moves along the polynucleotide (e.g. in 1 nucleotide fuel-driven steps) it drives the conjugate out of the nanopore. The polypeptide section of the conjugate thus passes through the nanopore (state C) and is thus characterised.

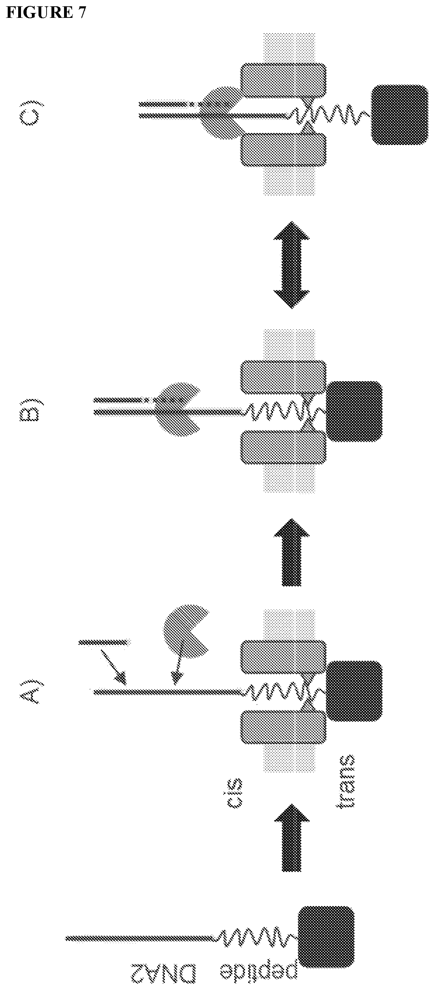

**Figure 7**. Schematic showing a non-limiting example of use of a blocking moiety (black square) which when in contact with the nanopore prevents the movement of the conjugate through the nanopore. In the non-limiting example as shown the polynucleotide-handling protein is a polymerase which can control the movement of the conjugate by extension of the polynucleotide portion of the conjugate. Chain extension can continue until the blocking moiety reaches the nanopore. Dissociation of the newly synthesized strand allows the conjugate to move back through the nanopore from *cis* to *trans* and then the polynucleotide can re-cycle the movement of the conjugate through the nanopore from *trans* to *cis.* In this way the conjugate can be "flossed" through the nanopore. Other polynucleotide-handling proteins can be used in analogous methods.

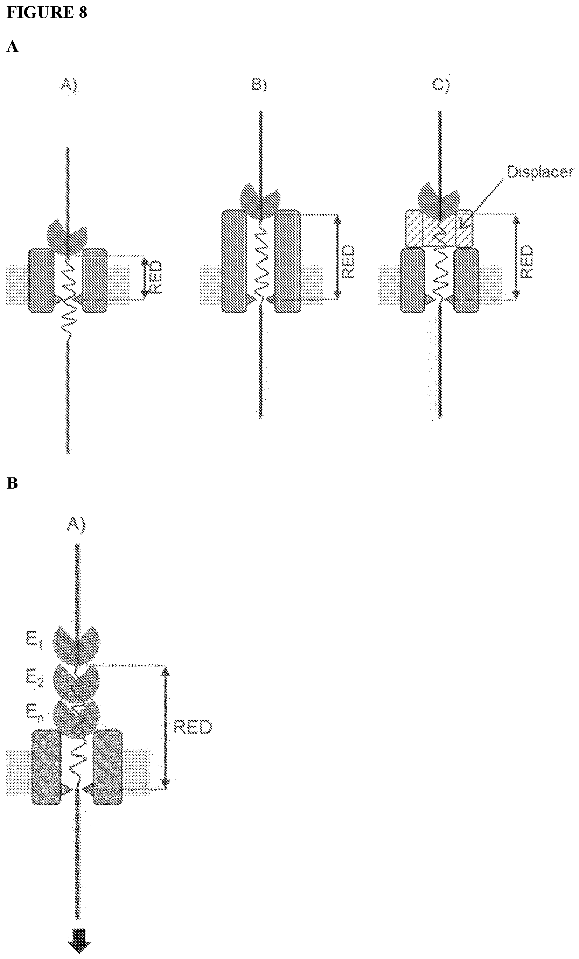

**Figure 8**. Schematic showing non-limiting examples of strategies for increasing the distance between the nanopore (e.g. a constriction within the nanopore) and the active site of the polynucleotide-handling protein used to control the movement of the conjugate with respect to the nanopore. **A:** Schematic of an unmodified pore showing the unmodified RED. **B:** A nanopore can be modified to extend the RED. **C:** A displacer unit can be used to displace the polynucleotide-handling protein from the nanopore thus extending the RED. **D:** Multiple polynucleotide-handling proteins can be used to displace the active polynucleotide-handling protein which controls the movement of the conjugate with respect to the nanopore from the nanopore. These embodiments are described in more detail herein.

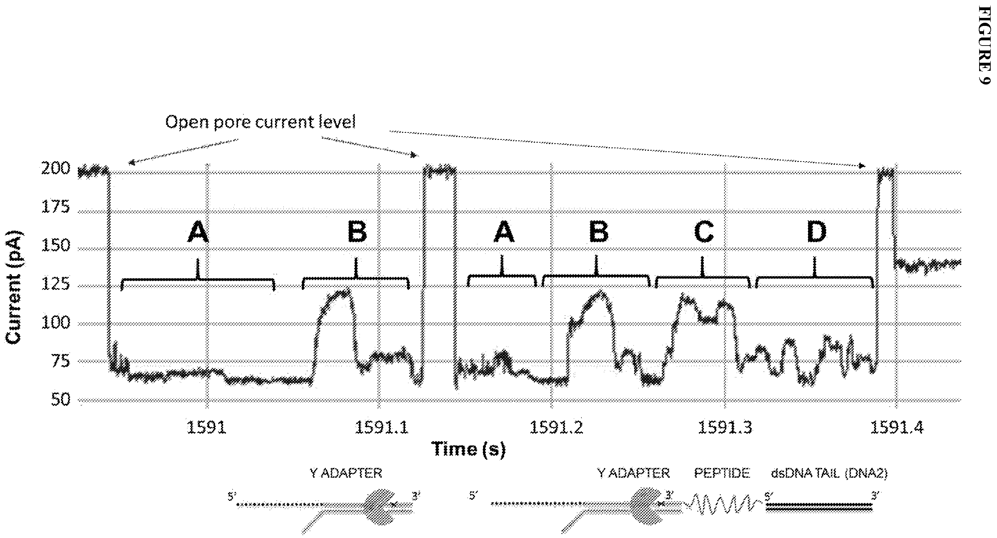

**Figure 9****.** Representative current vs. time traces for Example 1 with cartoons of corresponding constructs for clarity. States A-D correspond to those described in Figure 4F: A - capture of the leader strand by the nanopore, B - translocation of the Y adapter across the nanopore reader head (RED), C - translocation of the polypeptide across RED, D - translocation of the polynucleotide tail (DNA2). First trace represents a partial translocation event of only the Y adapter (states A and B only), while the second trace shows translocation of the entire conjugated polynucleotide-polypeptide across the nanopore. Data obtained as described in Example 1 (this data for polynucleotide-peptide conjugate containing peptide of sequence SEQ ID NO: 20).

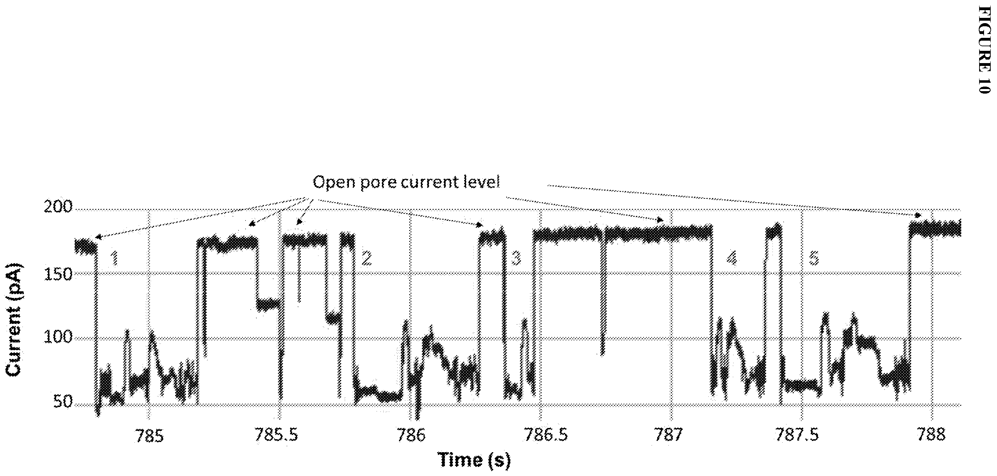

**Figure 10****.** Current *vs.* time trace illustrating the high throughput of data collection; in the period of 3 seconds there are 5 capture events and 4 correspond to the full polynucleotide-polypeptide conjugate (event 3 is a partial translocation of only the Y-adapter). Data described in Example 1 (this data for polynucleotide-peptide conjugate containing peptide of sequence SEQ ID NO: 20).

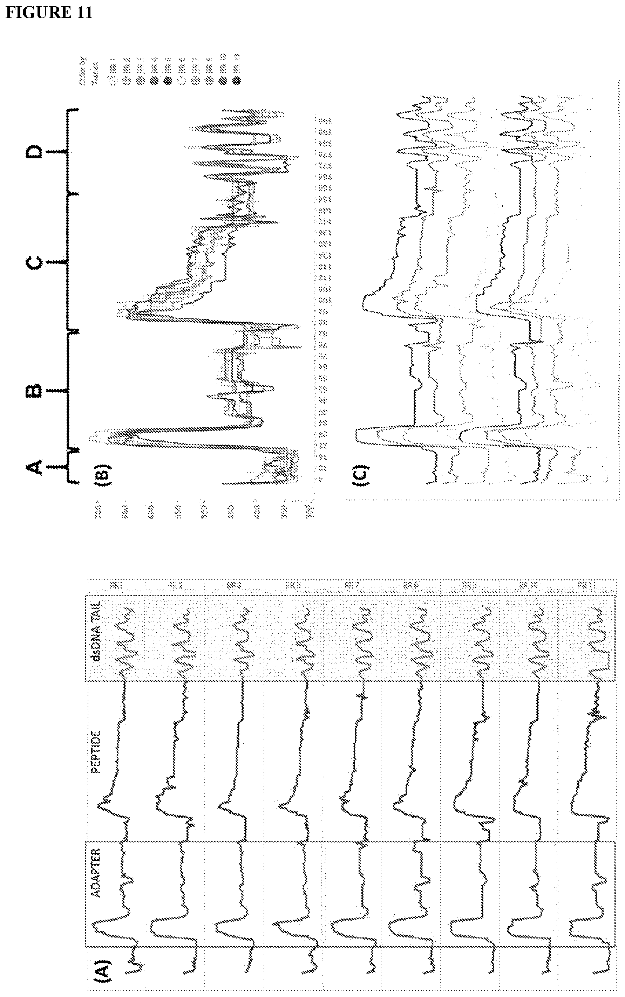

**Figure 11****.** Current traces for translocation of polynucleotide-peptide conjugate described in Figure 4B and Example 1 corresponding to peptide sequence GGSGRRSGSG (SEQ ID NO: 21). **A:** 11 examples of traces aligned with respect to states described in Figures 4 and Figure 9. **B:** Overlay of the same 11 traces. **C:** A stacked plot of the 11 example traces to illustrate normalisation of time axis using a dynamic time warping algorithm to facilitate best alignment of the key trace features.

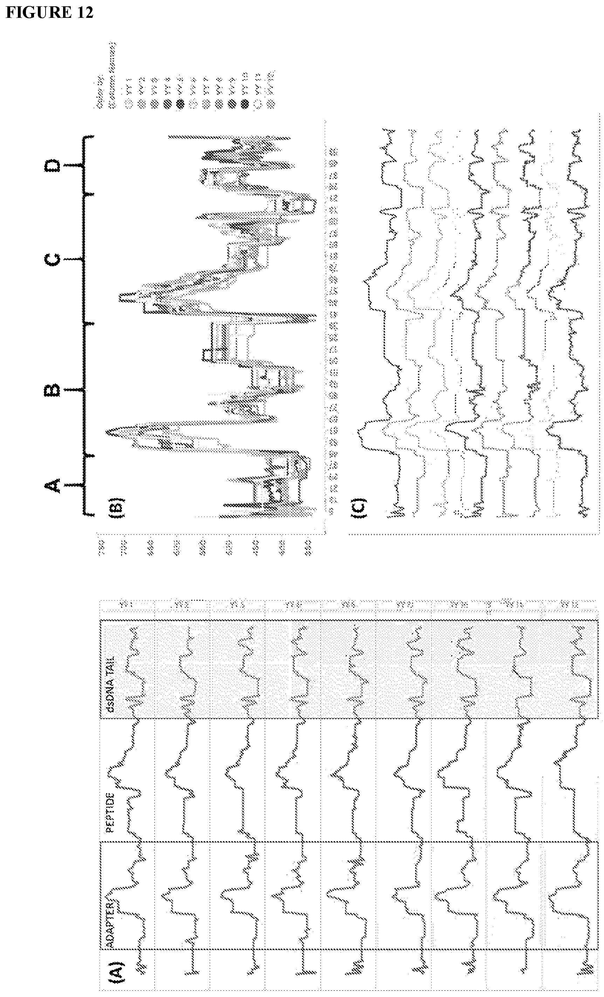

**Figure 12****.** Current traces for translocation of polynucleotide-peptide conjugate described in Figure 4B and Example 1 corresponding to peptide sequence GGSGYYSGSG (SEQ ID NO: 22). **A:** 12 examples of traces aligning with respect to states described in Figures 4 and Figure 9. **B:** Overlay of the same 12 traces. **C:** A stacked plot of the 12 example traces.

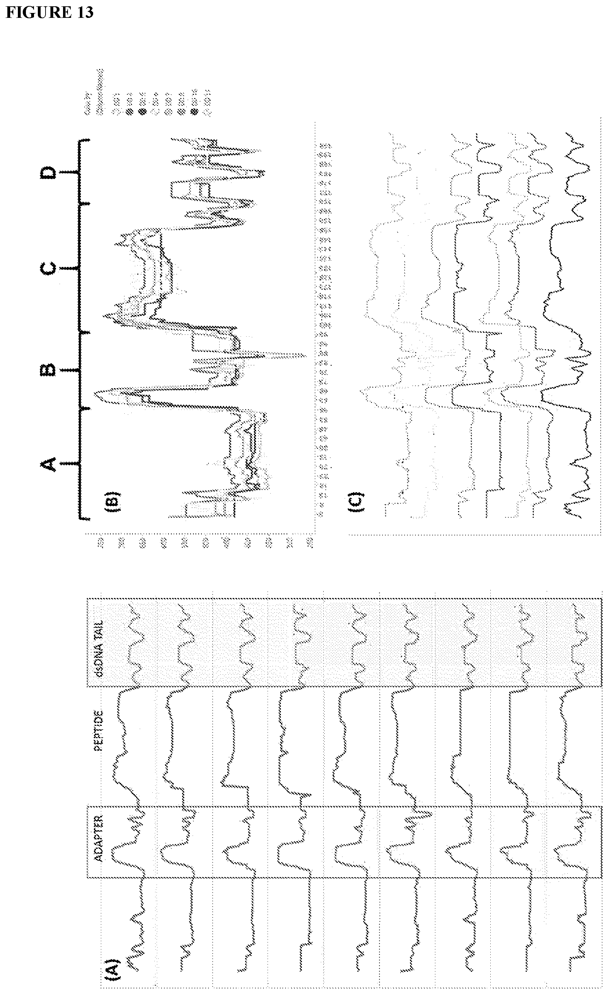

**Figure 13****.** Current traces for translocation of polynucleotide-peptide conjugate described in Figure 4B and Example 1 corresponding to peptide sequence GGSGDDSGSG (SEQ ID NO: 20). **A:** 11 examples of traces aligning with respect to states described in Figures 4 and Figure 9. **B:** Overlay of the same 11 traces. **C:** A stacked plot of the 11 example traces.

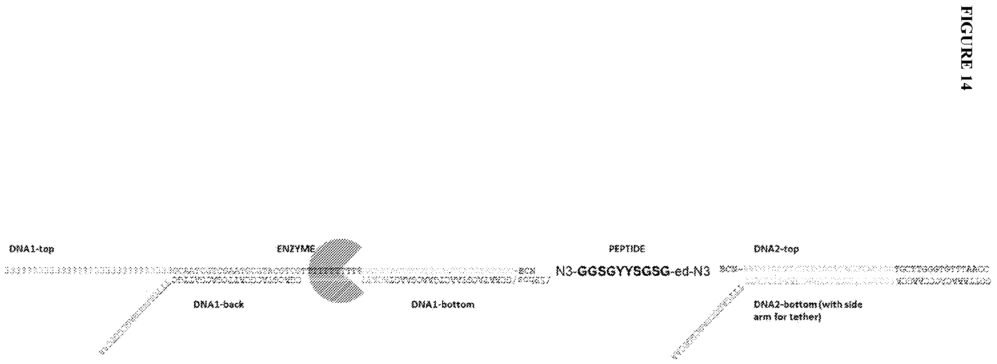

**Figure 14****.** Schematic structure of a construct obtained using peptide of SEQ ID NO: 22; Y adapter comprising polynucleotide strands of SEQ ID NOs: 11, 12 and 13; and polynucleotide tail comprising polynucleotide strands of SEQ ID NOs: 14 and 16 (described in Example 1).

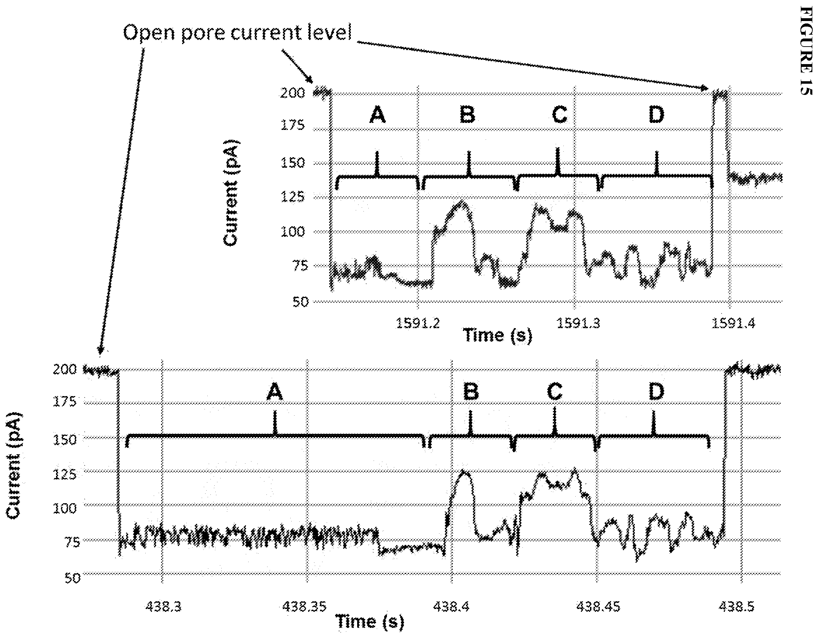

**Figure 15****.** Representative current vs. time traces for Example 2 compared to Example 1. States A-D correspond to those described in Figure 4F: A - capture of the leader strand by the nanopore, B - translocation of the Y adapter across the nanopore reader head (RED), C - translocation of the polypeptide across RED, D - translocation of the polynucleotide tail (DNA2). Trace in the top panel was collected according to the protocol in Example 1 (using a peptide pre-modified during synthesis); trace in the bottom panel shows translocation of polynucleotide-polypeptide conjugated according to the protocol in Example 2 (using unmodified peptide of SEQ ID NO: 23; i.e. the same sequence as in the corresponding trace for Example 1).

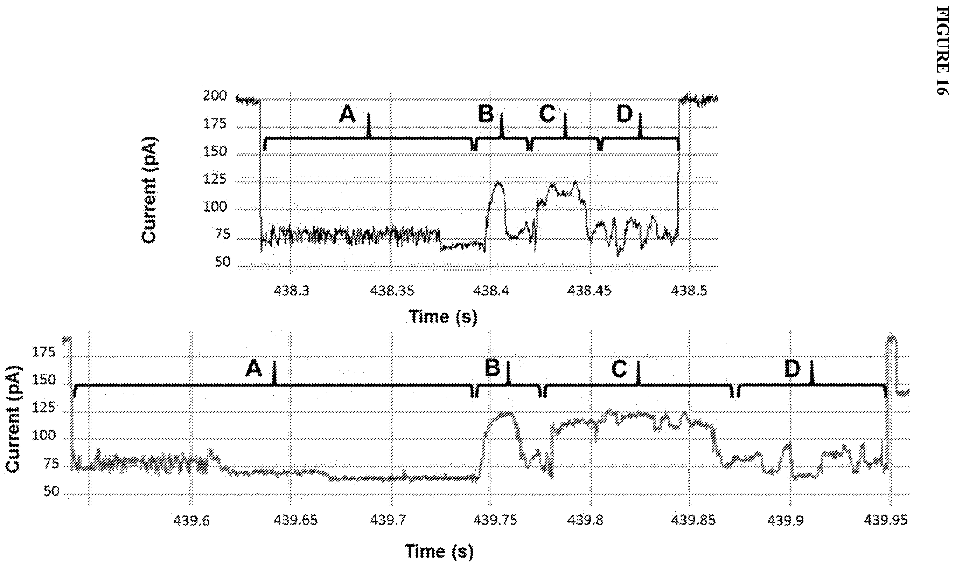

**Figure 16****.** Representative current vs. time traces for translocation of polynucleotide-peptide conjugates of a 10-amino acid peptide (top panel; SEQ ID NO: 20) compared to a 21-amino acid peptide (bottom panel; SEQ ID NO: 24). States A-D correspond to those described in Figure 4F: A - capture of the leader strand by the nanopore, B - translocation of the Y adapter across the nanopore reader head (RED), C - translocation of the polypeptide across RED, D - translocation of the polynucleotide tail. Results are described in Example 3.

### Detailed Description

The present invention will be described with respect to particular embodiments and with reference to certain drawings but the invention is not limited thereto but only by the claims. Any reference signs in the claims shall not be construed as limiting the scope. Of course, it is to be understood that not necessarily all aspects or advantages may be achieved in accordance with any particular embodiment of the invention. Thus, for example those skilled in the art will recognize that the invention may be embodied or carried out in a manner that achieves or optimizes one advantage or group of advantages as taught herein without necessarily achieving other aspects or advantages as may be taught or suggested herein.

The invention, both as to organization and method of operation, together with features and advantages thereof, may best be understood by reference to the following detailed description when read in conjunction with the accompanying drawings. The aspects and advantages of the invention will be apparent from and elucidated with reference to the embodiment(s) described hereinafter. Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Similarly, it should be appreciated that in the description of exemplary embodiments of the invention, various features of the invention are sometimes grouped together in a single embodiment, figure, or description thereof for the purpose of streamlining the disclosure and aiding in the understanding of one or more of the various inventive aspects. This method of disclosure, however, is not to be interpreted as reflecting an intention that the claimed invention requires more features than are expressly recited in each claim. Rather, as the following claims reflect, inventive aspects lie in less than all features of a single foregoing disclosed embodiment.

It should be appreciated that "embodiments" of the disclosure can be specifically combined together unless the context indicates otherwise. The specific combinations of all disclosed embodiments (unless implied otherwise by the context) are further disclosed embodiments of the claimed invention.

In addition as used in this specification and the appended claims, the singular forms "a", "an", and "the" include plural referents unless the content clearly dictates otherwise. Thus, for example, reference to "a polynucleotide" includes two or more polynucleotides, reference to "a motor protein" includes two or more such proteins, reference to "a helicase" includes two or more helicases, reference to "a monomer" refers to two or more monomers, reference to "a pore" includes two or more pores and the like.

### Definitions

Where an indefinite or definite article is used when referring to a singular noun e.g. "a" or "an", "the", this includes a plural of that noun unless something else is specifically stated. Where the term "comprising" is used in the present description and claims, it does not exclude other elements or steps. Furthermore, the terms first, second, third and the like in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein. The following terms or definitions are provided solely to aid in the understanding of the invention. Unless specifically defined herein, all terms used herein have the same meaning as they would to one skilled in the art of the present invention. Practitioners are particularly directed to Sambrook et al., Molecular Cloning: A Laboratory Manual, 4th ed., Cold Spring Harbor Press, Plainsview, New York (2012); and Ausubel et al., Current Protocols in Molecular Biology (Supplement 114), John Wiley & Sons, New York (2016), for definitions and terms of the art. The definitions provided herein should not be construed to have a scope less than understood by a person of ordinary skill in the art.

"About" as used herein when referring to a measurable value such as an amount, a temporal duration, and the like, is meant to encompass variations of ± 20 % or ± 10 %, more preferably ± 5 %, even more preferably ± 1 %, and still more preferably ± 0.1 % from the specified value, as such variations are appropriate to perform the disclosed methods.

"Nucleotide sequence", "DNA sequence" or "nucleic acid molecule(s)" as used herein refers to a polymeric form of nucleotides of any length, either ribonucleotides or deoxyribonucleotides. This term refers only to the primary structure of the molecule. Thus, this term includes double- and single-stranded DNA, and RNA. The term "nucleic acid" as used herein, is a single or double stranded covalently-linked sequence of nucleotides in which the 3' and 5' ends on each nucleotide are joined by phosphodiester bonds. The polynucleotide may be made up of deoxyribonucleotide bases or ribonucleotide bases. Nucleic acids may be manufactured synthetically in vitro or isolated from natural sources. Nucleic acids may further include modified DNA or RNA, for example DNA or RNA that has been methylated, or RNA that has been subject to post-translational modification, for example 5'-capping with 7-methylguanosine, 3'-processing such as cleavage and polyadenylation, and splicing. Nucleic acids may also include synthetic nucleic acids (XNA), such as hexitol nucleic acid (HNA), cyclohexene nucleic acid (CeNA), threose nucleic acid (TNA), glycerol nucleic acid (GNA), locked nucleic acid (LNA) and peptide nucleic acid (PNA). Sizes of nucleic acids, also referred to herein as "polynucleotides" are typically expressed as the number of base pairs (bp) for double stranded polynucleotides, or in the case of single stranded polynucleotides as the number of nucleotides (nt). One thousand bp or nt equal a kilobase (kb). Polynucleotides of less than around 40 nucleotides in length are typically called "oligonucleotides" and may comprise primers for use in manipulation of DNA such as via polymerase chain reaction (PCR).

The term "amino acid" in the context of the present disclosure is used in its broadest sense and is meant to include organic compounds containing amine (NH₂) and carboxyl (COOH) functional groups, along with a side chain (e.g., a R group) specific to each amino acid. In some embodiments, the amino acids refer to naturally occurring L α-amino acids or residues. The commonly used one and three letter abbreviations for naturally occurring amino acids are used herein: A=Ala; C=Cys; D=Asp; E=Glu; F=Phe; G=Gly; H=His; I=Ile; K=Lys; L=Leu; M=Met; N=Asn; P=Pro; Q=Gln; R=Arg; S=Ser; T=Thr; V=Val; W=Trp; and Y=Tyr (Lehninger, A. L., (1975) Biochemistry, 2d ed., pp. 71-92, Worth Publishers, New York). The general term "amino acid" further includes D-amino acids, retro-inverso amino acids as well as chemically modified amino acids such as amino acid analogues, naturally occurring amino acids that are not usually incorporated into proteins such as norleucine, and chemically synthesised compounds having properties known in the art to be characteristic of an amino acid, such as β-amino acids. For example, analogues or mimetics of phenylalanine or proline, which allow the same conformational restriction of the peptide compounds as do natural Phe or Pro, are included within the definition of amino acid. Such analogues and mimetics are referred to herein as "functional equivalents" of the respective amino acid. Other examples of amino acids are listed by Roberts and Vellaccio, The Peptides: Analysis, Synthesis, Biology, Gross and Meiehofer, eds., Vol. 5 p. 341, Academic Press, Inc., N.Y. 1983.

The terms "polypeptide", and "peptide" are interchangeably used herein to refer to a polymer of amino acid residues and to variants and synthetic analogues of the same. Thus, these terms apply to amino acid polymers in which one or more amino acid residues is a synthetic non-naturally occurring amino acid, such as a chemical analogue of a corresponding naturally occurring amino acid, as well as to naturally-occurring amino acid polymers. Polypeptides can also undergo maturation or post-translational modification processes that may include, but are not limited to: glycosylation, proteolytic cleavage, lipidization, signal peptide cleavage, propeptide cleavage, phosphorylation, and such like. A peptide can be made using recombinant techniques, e.g., through the expression of a recombinant or synthetic polynucleotide. A recombinantly produced peptide it typically substantially free of culture medium, e.g., culture medium represents less than about 20 %, more preferably less than about 10 %, and most preferably less than about 5 % of the volume of the protein preparation.

The term "protein" is used to describe a folded polypeptide having a secondary or tertiary structure. The protein may be composed of a single polypeptide, or may comprise multiple polypeptides that are assembled to form a multimer. The multimer may be a homooligomer, or a heterooligmer. The protein may be a naturally occurring, or wild type protein, or a modified, or non-naturally, occurring protein. The protein may, for example, differ from a wild type protein by the addition, substitution or deletion of one or more amino acids.

A "variant" of a protein encompass peptides, oligopeptides, polypeptides, proteins and enzymes having amino acid substitutions, deletions and/or insertions relative to the unmodified or wild-type protein in question and having similar biological and functional activity as the unmodified protein from which they are derived. The term "amino acid identity" as used herein refers to the extent that sequences are identical on an amino acid-by-amino acid basis over a window of comparison. Thus, a "percentage of sequence identity" is calculated by comparing two optimally aligned sequences over the window of comparison, determining the number of positions at which the identical amino acid residue (e.g., Ala, Pro, Ser, Thr, Gly, Val, Leu, Ile, Phe, Tyr, Trp, Lys, Arg, His, Asp, Glu, Asn, Gln, Cys and Met) occurs in both sequences to yield the number of matched positions, dividing the number of matched positions by the total number of positions in the window of comparison (i.e., the window size), and multiplying the result by 100 to yield the percentage of sequence identity.

For all aspects and embodiments of the present invention, a "variant" has at least 50%, 60%, 70%, 80%, 90%, 95% or 99% complete sequence identity to the amino acid sequence of the corresponding wild-type protein. Sequence identity can also be to a fragment or portion of the full length polynucleotide or polypeptide. Hence, a sequence may have only 50 % overall sequence identity with a full length reference sequence, but a sequence of a particular region, domain or subunit could share 80 %, 90 %, or as much as 99 % sequence identity with the reference sequence.

The term "wild-type" refers to a gene or gene product isolated from a naturally occurring source. A wild-type gene is that which is most frequently observed in a population and is thus arbitrarily designed the "normal" or "wild-type" form of the gene. In contrast, the term "modified", "mutant" or "variant" refers to a gene or gene product that displays modifications in sequence (e.g., substitutions, truncations, or insertions), post-translational modifications and/or functional properties (e.g., altered characteristics) when compared to the wild-type gene or gene product. It is noted that naturally occurring mutants can be isolated; these are identified by the fact that they have altered characteristics when compared to the wild-type gene or gene product. Methods for introducing or substituting naturally-occurring amino acids are well known in the art. For instance, methionine (M) may be substituted with arginine (R) by replacing the codon for methionine (ATG) with a codon for arginine (CGT) at the relevant position in a polynucleotide encoding the mutant monomer. Methods for introducing or substituting non-naturally-occurring amino acids are also well known in the art. For instance, non-naturally-occurring amino acids may be introduced by including synthetic aminoacyl-tRNAs in the IVTT system used to express the mutant monomer. Alternatively, they may be introduced by expressing the mutant monomer in *E. coli* that are auxotrophic for specific amino acids in the presence of synthetic (i.e. non-naturally-occurring) analogues of those specific amino acids. They may also be produced by naked ligation if the mutant monomer is produced using partial peptide synthesis. Conservative substitutions replace amino acids with other amino acids of similar chemical structure, similar chemical properties or similar side-chain volume. The amino acids introduced may have similar polarity, hydrophilicity, hydrophobicity, basicity, acidity, neutrality or charge to the amino acids they replace. Alternatively, the conservative substitution may introduce another amino acid that is aromatic or aliphatic in the place of a pre-existing aromatic or aliphatic amino acid. Conservative amino acid changes are well-known in the art and may be selected in accordance with the properties of the 20 main amino acids as defined in Table 1 below. Where amino acids have similar polarity, this can also be determined by reference to the hydropathy scale for amino acid side chains in Table 2.

**Table 1 - Chemical properties of amino acids**

| | | | |
|---|---|---|---|
| Ala | aliphatic, hydrophobic, neutral | Met | hydrophobic, neutral |
| Cys | polar, hydrophobic, neutral | Asn | polar, hydrophilic, neutral |
| Asp | polar, hydrophilic, charged (-) | Pro | hydrophobic, neutral |
| Glu | polar, hydrophilic, charged (-) | Gln | polar, hydrophilic, neutral |
| Phe | aromatic, hydrophobic, neutral | Arg | polar, hydrophilic, charged (+) |
| Gly | aliphatic, neutral | Ser | polar, hydrophilic, neutral |
| His | aromatic, polar, hydrophilic, charged (+) | Thr | polar, hydrophilic, neutral |
| Ile | aliphatic, hydrophobic, neutral | Val | aliphatic, hydrophobic, neutral |
| Lys | polar, hydrophilic, charged(+) | Trp | aromatic, hydrophobic, neutral |
| Leu | aliphatic, hydrophobic, neutral | Tyr | aromatic, polar, hydrophobic |

**Table 2 - Hydropathy scale**

| Side Chain | Hydropathy |
|---|---|
| Ile | 4.5 |
| Val | 4.2 |
| Leu | 3.8 |
| Phe | 2.8 |
| Cys | 2.5 |
| Met | 1.9 |
| Ala | 1.8 |
| Gly | -0.4 |
| Thr | -0.7 |
| Ser | -0.8 |
| Trp | -0.9 |
| Tyr | -1.3 |
| Pro | -1.6 |
| His | -3.2 |
| Glu | -3.5 |
| Gln | -3.5 |
| Asp | -3.5 |
| Asn | -3.5 |
| Lys | -3.9 |
| Arg | -4.5 |

A mutant or modified protein, monomer or peptide can also be chemically modified in any way and at any site. A mutant or modified monomer or peptide is preferably chemically modified by attachment of a molecule to one or more cysteines (cysteine linkage), attachment of a molecule to one or more lysines, attachment of a molecule to one or more non-natural amino acids, enzyme modification of an epitope or modification of a terminus. Suitable methods for carrying out such modifications are well-known in the art. The mutant of modified protein, monomer or peptide may be chemically modified by the attachment of any molecule. For instance, the mutant of modified protein, monomer or peptide may be chemically modified by attachment of a dye or a fluorophore.

### Disclosed Methods

The disclosure relates to methods of characterising polypeptides by forming a conjugate with a polynucleotide and using a polynucleotide-handling protein to control the movement of the conjugate with respect to a nanopore, wherein the polynucleotide-handling protein is modified by covalently closing a polynucleotide-unbinding opening through which a polynucleotide strand may unbind.

In contrast to methods which seek to control the movement of a polypeptide with respect to a nanopore using a poly*peptide*-handling enzyme, the methods of the present disclosure enable the control of the movement of a polypeptide with respect to a nanopore using a poly*nucleotide*-handling enzyme.

The methods disclosed herein exploit the ability of polynucleotide-handling proteins to control the movement of conjugates which do not only comprise polynucleotides. In particular, conjugates which comprise polypeptides can be moved in a controlled manner using polynucleotide-handling proteins, as described herein. Polynucleotide-handling proteins suitable for use in the disclosed methods are described in more detail herein.

Accordingly, provided herein is a method of characterising a target polypeptide, comprising
- conjugating the target polypeptide to a polynucleotide to form a polynucleotide-polypeptide conjugate;
- contacting the conjugate with a polynucleotide-handling protein capable of controlling the movement of the polynucleotide with respect to a nanopore; and
- taking one or more measurements characteristic of the polypeptide as the conjugate moves with respect to the nanopore,
thereby characterising the polypeptide;
wherein the polynucleotide-handling protein is modified by covalently closing a polynucleotide-unbinding opening through which a polynucleotide strand may unbind, thereby preventing the polynucleotide binding protein from disengaging from the conjugate when the polynucleotide-handling protein contacts a portion of the conjugate comprising a polypeptide.

Any suitable polypeptide can be characterised using the methods disclosed herein. In some embodiments the target polypeptide is a protein or naturally occurring polypeptide. In some embodiments the polypeptide is a synthetic polypeptide. Polypeptides which can be characterised in accordance with the disclosed methods are described in more detail herein.

Any suitable polynucleotide can be used in forming the conjugate for use in the methods disclosed herein. In some embodiments the polynucleotide has a length at least as long as a portion of the target polypeptide to be characterised. In some embodiments the polynucleotide has a greater length than the portion of the target polypeptide to be characterised. This is discussed in more detail herein. Polynucleotides suitable for use in the disclosed methods are disclosed in more detail herein.

In the disclosed methods, the target polypeptide can be conjugated to the polynucleotide using any suitable means. Some exemplary means are described in more detail herein.

The conjugate formed in the disclosed methods is contacted with a polynucleotide-handling protein which is capable of controlling the movement of the polynucleotide with respect to a nanopore. Exemplary polynucleotide-handling proteins are described in more detail herein.

The polynucleotide-handling protein controls the movement of the polynucleotide with respect to a nanopore. Thus, the polynucleotide-handling protein controls the movement of the conjugate with respect to the nanopore. Any suitable nanopore can be used in the disclosed methods. Nanopores suitable for use in the disclosed methods are described in more detail herein.

The disclosed methods comprise taking one or more measurements characteristic of the polypeptide as the conjugate moves with respect to the nanopore. The one or more measurements can be any suitable measurements. Typically, the one or more measurements are electrical measurements, e.g. current measurements, and/or are one or more optical measurements. Apparatuses for recording suitable measurements, and the information that such measurements can provide, are described in more detail herein.

### Characterising a target polypeptide

As disclosed herein, a polynucleotide can be used to control the movement of a polypeptide with respect to a nanopore. The movement of the polynucleotide is controlled by the polynucleotide-handling protein. Because the polynucleotide is conjugated to the polypeptide in the conjugate, the movement of the polynucleotide drives the movement of the polypeptide.

The use of a polynucleotide-handling protein to control the movement of the polynucleotide, and thus the movement of the polypeptide, may be associated with advantages compared to methods for characterising polypeptides known in the art. By way of example, polynucleotide-handling proteins are capable of processing the handling of polynucleotides with higher turnover rates compared to polypeptide-handling enzymes. This means that characterisation data may be obtained more rapidly for polypeptides characterised in accordance with the disclosed methods as compared to previously known methods.

These and other advantages will become apparent throughout the present disclosure.

In developing the methods of the present disclose, the inventors have found that the length of the polypeptide which can be characterised is typically improved when nanopores having a longer barrel or channel are used as compared to nanopores which have a shorter barrel or channel. Without being bound by theory, the inventors believe that this may be because pores having a longer barrel or channel, when used in conjunction with a polynucleotide-handling protein as in the disclosed methods, lead to a longer distance between the active site of the polynucleotide-handling protein and a constriction in the nanopore than pores having a shorter barrel or channel. This distance can be referred to as the RED (reader-enzyme distance). Those skilled in the art will appreciate that (as discussed below) the form of the nanopore is not limiting. The nanopore may be a protein nanopore or a solid state nanopore. If the nanopore does not have a narrowing within the channel of the nanopore then the constriction as used herein may, for example, in one embodiment, be identified with an opening of the nanopore.

Without being bound by theory, it is surmised that the length of the portion of the polypeptide within the conjugate which can be characterised by the nanopore may correspond to or be determined by the RED. In other words, the nanopore may comprise a reading head, and the one or more measurements are characteristic of a "read portion" of the polypeptide, wherein the length of the read portion corresponds to or is determined by the distance between the reading head and the active site of the polynucleotide-handling protein.

In view of this, in some embodiments of the disclosed methods the polynucleotide has a length at least as long as the portion of the target polypeptide to be characterised. In some embodiments the polynucleotide has a length longer than the portion of the target polypeptide to be characterised. This ensures that the length of the polypeptide portion which can be characterised is not limited by the amount of polynucleotide for the polynucleotide-handling protein to control the movement of.

The method can be understood by reference to Figure 1, which illustrates one non-limiting example of the disclosed method. A conjugate may comprise a polynucleotide and a polypeptide, and is contacted with a polynucleotide-handling protein such that the polypeptide threads the nanopore. In the illustrated embodiment a further polynucleotide is used to facilitate the threading of the polypeptide through the nanopore. Such use is within the scope of the disclosed methods, however this is not essential.

The polynucleotide-handling protein processes the polynucleotide conjugated to the polypeptide. As the polynucleotide-handling protein processes the polynucleotide, the conjugate is passed through the nanopore and so the polypeptide is passed through the nanopore. As the polypeptide is passed through the nanopore it is characterised.

In the example illustrated in Figure 1 the polynucleotide-handling protein moves the conjugate "into" the pore, from the "viewpoint" of the polynucleotide-handling protein. For example, as shown the polynucleotide-handling protein is located on the *cis* side of the nanopore and moves the conjugate into the pore, i.e. from the *cis* side to the *trans* side. The opposite setup could also be used.

In other words, in some embodiments, the polynucleotide-handling protein is located on the *cis* side of the nanopore and the polynucleotide-handling protein controls the movement of the conjugate from the *cis* side of the nanopore to the *trans* side of the nanopore. Thus, in some embodiments, the polynucleotide-handling protein is located on the *cis* side of the nanopore and the polynucleotide-handling protein controls the movement of the polynucleotide from the *cis* side of the nanopore to the *trans* side of the nanopore, thereby controlling the movement of the polypeptide through the nanopore.

In other embodiments, the polynucleotide-handling protein is located on the *trans* side of the nanopore and the polynucleotide-handling protein controls the movement of the conjugate from the *trans* side of the nanopore to the *cis* side of the nanopore. Thus, in some embodiments, the polynucleotide-handling protein is located on the *trans* side of the nanopore and the polynucleotide-handling protein controls the movement of the polynucleotide from the *trans* side of the nanopore to the *cis* side of the nanopore, thereby controlling the movement of the polypeptide through the nanopore.

As explained herein, the conjugate may comprise a leader. Any suitable leader may be used, as explained herein. Optionally, the leader may be a polynucleotide. The leader may be the same as the polynucleotide in the conjugate or may be different. As explained above, the leader may facilitate the threading of the conjugate through the nanopore.

In other words, in some embodiments the conjugate comprises one or more structures of the form L-{P-N}-P*ₘ*, wherein:
- L is a leader, wherein L is optionally an N moiety;
- P is a polypeptide;
- N comprises a polynucleotide; and
- *m* is 0 or 1;
and the method may comprise threading the leader (L) through the nanopore thereby contacting the polypeptide (P) with the nanopore.

In some such embodiments, the polynucleotide-handling protein is located on the *cis* side of the nanopore and the method comprises allowing the polynucleotide-handling protein to control the movement of the polynucleotide moiety (N) from the *cis* side of the nanopore to the *trans* side of the nanopore, thereby controlling the movement of the polypeptide (P) through the nanopore. In other embodiments, the polynucleotide-handling protein is located on the *trans* side of the nanopore and the method comprises allowing the polynucleotide-handling protein to control the movement of the polynucleotide moiety (N) from the *trans* side of the nanopore to the *cis* side of the nanopore, thereby controlling the movement of the polypeptide (P) through the nanopore.

As explained in more detail herein, the conjugate may comprise one or more adapters and/or anchors. A non-limiting example of such a setup is shown in Figure 2.

As explained in more detail herein, in some embodiments the conjugate comprises multiple polynucleotides and polypeptides. In such embodiments the polynucleotide-handling protein sequentially controls the movement of the polynucleotides with respect to the nanopore, thus sequentially moving the polypeptide with respect to the nanopore. In this way, each polypeptide within the conjugate can be sequentially characterised in the disclosed methods.

For example, the conjugate may comprise one or more structures of the form L-P₁-N-{P-N}*ₙ*-P*ₘ*, wherein:
- *n* is a positive integer;
- L is a leader, wherein L is optionally an N moiety;
- each P, which may be the same or different, is a polypeptide;
- each N, which may be the same or different, comprises a polynucleotide; and
- *m* is 0 or 1;
and the method may comprise threading the leader (L) through the nanopore thereby contacting polypeptide (P₁) with the nanopore.

Typically, in such embodiments, n is from 1 to about 1000, e.g. from 2 to about 100, such as from about 3 to about 10, e.g. 1, 2, 3, 4, 5, 6, 7, 8, 9 or 10.

In some such embodiments, the polynucleotide-handling protein is located on the *cis* side of the nanopore and the method comprises allowing the polynucleotide-handling protein to control the movement of each polynucleotide (N) sequentially from the *cis* side of the nanopore to the *trans* side of the nanopore, thereby controlling the movement of each polypeptide (P) sequentially through the nanopore. In other such embodiments, the polynucleotide-handling protein is located on the *trans* side of the nanopore and the method comprises allowing the polynucleotide-handling protein to control the movement of each polynucleotide (N) sequentially from the *trans* side of the nanopore to the *cis* side of the nanopore, thereby controlling the movement of each polypeptide (P) sequentially through the nanopore.

Those skilled in the art will appreciate that when the conjugate comprises more than one polypeptide, it may be advantageous that (as described in more detail herein) the polynucleotide-handling protein can remain bound to the conjugate when it contacts the polypeptide without dissociating. For example, as shown in Figure 3, this allows polynucleotide-handling protein to pass over portions of polypeptide in the conjugate as it contacts them, in order to move onto sequential portions of polynucleotide in order to control the movement of the conjugate with respect to the nanopore.

A non-limiting example of a more complex setup in accordance with the embodiment shown in Figure 3 is depicted in Figure 4, in which various adapters and tethers are used to facilitate the characterisation of the polypeptide. Just one polypeptide section is shown in Figure 4 although those skilled in the art will appreciate that multiple such sections could be incorporated, as shown schematically in Figure 5.

Another non-limiting embodiment of the disclosed methods is shown schematically in Figure 6. A conjugate may comprise a polynucleotide and a polypeptide, and is contacted with a polynucleotide-handling protein such that the polypeptide threads the nanopore. In the illustrated embodiment a leader (which is optionally a further polynucleotide) is used to facilitate the threading of the polypeptide through the nanopore. Such use is within the scope of the disclosed methods, however this is not essential.

The polynucleotide-handling protein processes the polynucleotide conjugated to the polypeptide. As the polynucleotide-handling protein processes the polynucleotide, the conjugate is passed through the nanopore and so the polypeptide is passed through the nanopore. As the polypeptide is passed through the nanopore it is characterised.

In the example illustrated in Figure 6 the polynucleotide-handling protein moves the conjugate "out" of the pore, from the "viewpoint" of the polynucleotide-handling protein. For example, as shown the polynucleotide-handling protein is located on the *cis* side of the nanopore and moves the conjugate into the pore, i.e. from the *trans* side to the *cis* side. The opposite setup could also be used.

In other words, in some embodiments, the polynucleotide-handling protein is located on the *cis* side of the nanopore and the polynucleotide-handling protein controls the movement of the conjugate from the *trans* side of the nanopore to the *cis* side of the nanopore. Thus, in some embodiments the polynucleotide-handling protein is located on the *cis* side of the nanopore and the polynucleotide-handling protein controls the movement of the polynucleotide from the *trans* side of the nanopore to the *cis* side of the nanopore, thereby controlling the movement of the polypeptide through the nanopore.

In other embodiments, the polynucleotide-handling protein is located on the *trans* side of the nanopore and the polynucleotide-handling protein controls the movement of the conjugate from the *cis* side of the nanopore to the *trans* side of the nanopore. Thus, in some embodiments the polynucleotide-handling protein is located on the *trans* side of the nanopore and the polynucleotide-handling protein controls the movement of the polynucleotide from the *cis* side of the nanopore to the *trans* side of the nanopore, thereby controlling the movement of the polypeptide through the nanopore.

Using similar notation as above, in some embodiments the conjugate comprises one or more structures of the form L-{P-N}- P*ₘ*, wherein:
- L is a leader, wherein L is optionally an N moiety;
- P is a polypeptide;
- N comprises a polynucleotide;
- *m* is 0 or 1;
and the method may comprise threading the leader (L) through the nanopore thereby contacting the polypeptide (P) with the nanopore.

In some such embodiments the polynucleotide-handling protein is located on the *cis* side of the nanopore and the method comprises allowing the polynucleotide-handling protein to control the movement of the polynucleotide (N) from the *trans* side of the nanopore to the *cis* side of the nanopore, thereby controlling the movement of the polypeptide (P) through the nanopore. In other such embodiments the polynucleotide-handling protein is located on the *trans* side of the nanopore and the method comprises allowing the polynucleotide-handling protein to control the movement of the polynucleotide (N) from the *cis* side of the nanopore to the *trans* side of the nanopore, thereby controlling the movement of the polypeptide (P) through the nanopore

In some embodiments, particularly embodiments where as discussed above the polynucleotide-handling protein controls the movement of the conjugate "out" of the nanopore, the conjugate may comprise a blocking moiety attached to the polypeptide via an optional linker. The blocking moiety is typically too large to pass through the nanopore and so when the movement of the conjugate with respect to the nanopore brings the blocking moiety into contact with the nanopore, the further movement of the conjugate through the nanopore is prevented. At such time the polynucleotide-handling protein may be allowed to transiently unbind from the conjugate. In embodiments of the disclosed methods in which the conjugate moves with respect to the nanopore under an applied force (e.g. a voltage potential or chemical potential) the conjugate may then move "back" through the pore in the opposite direction to the movement controlled by the polynucleotide-handling protein. The movement of the conjugate back through the pore allows the polypeptide portion of the conjugate to be re-characterised again.

The process can be repeated multiple times by sequentially allowing the polynucleotide-handling protein to bind and rebind to the conjugate. In such a manner, the conjugate may oscillate through the pore (i.e. it may be "flossed" through the nanopore). This "flossing" allows the polypeptide portion of the conjugate to be repeatedly characterised by the nanopore. In some embodiments this allows the accuracy of the characterisation information to be increased.

Any suitable blocking moiety can be used in such embodiments. For example, the conjugate may be modified with biotin and the blocking moiety may be e.g. streptavidin, avidin or neutravidin. The blocking moiety may be a large chemical group such as a dendrimer. The blocking moiety may be a nanoparticle or a bead. Other suitable blocking moieties will be apparent to those skilled in the art.

A non-limiting example of such a method is shown in Figure 7.

Accordingly, in some embodiments the method comprises
i) contacting the conjugate with the nanopore such that the blocking moiety is on the opposite side of the nanopore to the polynucleotide-handling protein;
ii) contacting the polynucleotide of the conjugate with the polynucleotide-handling protein;
iii) allowing the polynucleotide-handling protein to control the movement of the polynucleotide with respect to the nanopore thereby controlling the movement of the polypeptide through the nanopore;
iv) when the blocking moiety contacts the nanopore thereby preventing further movement of the conjugate through the nanopore, allowing the polynucleotide-handling protein to transiently unbind from the polynucleotide so that the conjugate moves through the nanopore under an applied force in a direction opposite to the direction of movement controlled by the polynucleotide-handling protein; and
v) optionally repeating steps (ii) to (iv) to oscillate the polypeptide through the nanopore.

### Displacer Units

As described above, and without being bound by theory, the inventors have found that the length of the polypeptide which can be characterised is typically improved when nanopores having a longer barrel or channel are used as compared to nanopores which have a shorter barrel or channel. As explained above, this is believed to be correlated with or determined by the "RED" distance, shown schematically in Figure 8A(A).

Accordingly, in some embodiments the nanopore is modified to extend the distance between the polynucleotide-handling protein and a constriction region of the nanopore. The nanopore is typically modified to extend the distance between the polynucleotide-handling protein and a constriction region of the nanopore as determined when the polynucleotide-handling protein is used to control the movement of the conjugate with respect to the nanopore. In some embodiments the polynucleotide-handling protein when used to control the movement of the conjugate with respect to the nanopore is in a "seating position" in contact with the nanopore, e.g. in contact with the *cis* or *trans* opening of the nanopore. This is described in more detail herein, and is shown schematically in Figure 8A(B).

In some embodiments, the distance between the active site of the polynucleotide-handling protein and the nanopore may be extended by using a displacer unit. The use of a displacer unit is shown schematically in Figure 8A(C). Accordingly, in some embodiments the methods provided herein comprise providing a displacer unit. In some embodiments the displacer unit is for separating the polypeptide-handling protein from the nanopore, thereby extending the distance between the polynucleotide-handling protein and the nanopore.

Any suitable displacer unit can be used in such embodiments. For example, a displacer unit can be provided as a protein.

Any suitable protein can be used as a displacer unit. Exemplary proteins include those which adopt a ring shaped conformation, e.g. as a multimer, and which thus can be readily positioned at the entrance of the nanopore. Many suitable ring shaped proteins are known in the art, including nanopores as described herein, helicases (e.g. T7 helicase) and variants thereof, etc. There is no requirement that the displacer has any activity of its own. In some embodiments the displacer unit does not provide any significant discrimination of either the polynucleotide or the peptide in the conjugate.

In some embodiments the displacer unit may comprise one or more polynucleotide-handling proteins or inactive variants thereof. This is shown schematically in Figure 8B. As shown, polynucleotide-handling protein (E₁) is used to control the movement of the conjugate with respect to the nanopore. Polynucleotide-handling proteins E₂...Eₙ are initially in contact with the polypeptide portion of the conjugate and thus do not control the movement of the conjugate with respect to the nanopore; however they displace polynucleotide-handling protein E₁ from the nanopore thus increasing the RED.

In such embodiments the polynucleotide-handling proteins used as displacer units may be the same or different to the polynucleotide-handling protein used to control the movement of the conjugate. In some embodiments the polynucleotide-handling proteins used as displacer units are formed of inactive variants of the same polynucleotide-handling protein used to control the movement of the conjugate.

One or more displacer units, e.g. one or more displacer units described herein, can be attached (e.g. coupled covalently or non-covalently) to the nanopore. Alternatively one or more displacer units can be associated with the nanopore, e.g. by using a polynucleotide to control their position with respect to the nanopore.

In other embodiments the polynucleotide-handling protein is modified to extend the distance from the active site of the polynucleotide-handling protein to the nanopore. The polynucleotide-handling protein is typically modified to extend the distance between the active site of the polynucleotide-handling protein and the nanopore as determined when the polynucleotide-handling protein is used to control the movement of the conjugate with respect to the nanopore. This is described in more detail herein.

### Polypeptide

As explained above, the disclosed methods comprise characterising a target polypeptide within a conjugate as the conjugate moves with respect to a nanopore.

Any suitable polypeptide can be characterised in the disclosed methods.

In some embodiments the target polypeptide is an unmodified protein or a portion thereof, or a naturally occurring polypeptide or a portion thereof.

In some embodiments the target polypeptide is secreted from cells. Alternatively, the target polypeptide can be produced inside cells such that it must be extracted from cells for characterisation by the disclosed methods. The polypeptide may comprise the products of cellular expression of a plasmid, e.g. a plasmid used in cloning of proteins in accordance with the methods described in Sambrook et al., Molecular Cloning: A Laboratory Manual, 4th ed., Cold Spring Harbor Press, Plainsview, New York (2012); and Ausubel et al., Current Protocols in Molecular Biology (Supplement 114), John Wiley & Sons, New York (2016).

The polypeptide may be obtained from or extracted from any organism or microorganism. The polypeptide may be obtained from a human or animal, e.g. from urine, lymph, saliva, mucus, seminal fluid or amniotic fluid, or from whole blood, plasma or serum. The polypeptide may be obtained from a plant e.g. a cereal, legume, fruit or vegetable.

The target polypeptide can be provided as an impure mixture of one or more polypeptides and one or more impurities. Impurities may comprise truncated forms of the target polypeptide which are distinct from the "target polypeptides" for characterisation in the disclosed methods. For example, the target polypeptide may be a full length protein and impurities may comprise fractions of the protein. Impurities may also comprise proteins other than the target protein e.g. which may be co-purified from a cell culture or obtained from a sample.

A polypeptide may comprise any combination of any amino acids, amino acid analogs and modified amino acids (i.e. amino acid derivatives). Amino acids (and derivatives, analogs etc) in the polypeptide can be distinguished by their physical size and charge.

The amino acids/derivatives/analogs can be naturally occurring or artificial.

In some embodiments the polypeptide may comprise any naturally occurring amino acid. Twenty amino acids are encoded by the universal genetic code. These are alanine (A), arginine (R), asparagine (N), aspartic acid (D), cysteine (C), glutamic acid/glutamate (E), glutamine (Q), glycine (G), histidine (H), isoleucine (I), leucine (L), lysine (K), methionine (M), phenylalanine (F), proline (P), serine (S), threonine (T), tryptophan (W), tyrosine (Y) and valine (V). Other naturally occurring amino acids include selenocysteine and pyrrolysine.

In some embodiments the polypeptide is modified. In some embodiments the polypeptide is modified for detection using the disclosed methods. In some embodiments the disclosed methods are for characterising modifications in the target polypeptide.

In some embodiments one or more of the amino acids/derivatives/analogs in the polypeptide is modified. In some embodiments one or more of the amino acids/derivatives/analogs in the polypeptide is post-translationally modified. As such, the methods disclosed herein can be used to detect the presence, absence, number of positions of post-translational modifications in a polypeptide. The disclosed methods can be used to characterise the extent to which a polypeptide has been post-translationally modified.

Any one or more post-translational modifications may be present in the polypeptide. Typical post-translational modifications include modification with a hydrophobic group, modification with a cofactor, addition of a chemical group, glycation (the non-enzymatic attachment of a sugar), biotinylation and pegylation. Post-translational modifications can also be non-natural, such that they are chemical modifications done in the laboratory for biotechnological or biomedical purposes. This can allow monitoring the levels of the laboratory made peptide, polypeptide or protein in contrast to the natural counterparts.

Examples of post-translational modification with a hydrophobic group include myristoylation, attachment of myristate, a C₁₄ saturated acid; palmitoylation, attachment of palmitate, a C₁₆ saturated acid; isoprenylation or prenylation, the attachment of an isoprenoid group; farnesylation, the attachment of a farnesol group; geranylgeranylation, the attachment of a geranylgeraniol group; and glypiation, and
glycosylphosphatidylinositol (GPI) anchor formation via an amide bond.

Examples of post-translational modification with a cofactor include lipoylation, attachment of a lipoate (C₈) functional group; flavination, attachment of a flavin moiety (e.g. flavin mononucleotide (FMN) or flavin adenine dinucleotide (FAD)); attachment of heme C, for instance via a thioether bond with cysteine; phosphopantetheinylation, the attachment of a 4'-phosphopantetheinyl group; and retinylidene Schiff base formation.

Examples of post-translational modification by addition of a chemical group include acylation, e.g. O-acylation (esters), N-acylation (amides) or S-acylation (thioesters); acetylation, the attachment of an acetyl group for instance to the N-terminus or to lysine; formylation; alkylation, the addition of an alkyl group, such as methyl or ethyl; methylation, the addition of a methyl group for instance to lysine or arginine; amidation; butyrylation; gamma-carboxylation; glycosylation, the enzymatic attachment of a glycosyl group for instance to arginine, asparagine, cysteine, hydroxylysine, serine, threonine, tyrosine or tryptophan; polysialylation, the attachment of polysialic acid; malonylation; hydroxylation; iodination; bromination; citrulination; nucleotide addition, the attachment of any nucleotide such as any of those discussed above, ADP ribosylation; oxidation; phosphorylation, the attachment of a phosphate group for instance to serine, threonine or tyrosine (O-linked) or histidine (N-linked); adenylylation, the attachment of an adenylyl moiety for instance to tyrosine (O-linked) or to histidine or lysine (N-linked); propionylation; pyroglutamate formation; S-glutathionylation; Sumoylation; S-nitrosylation; succinylation, the attachment of a succinyl group for instance to lysine; selenoylation, the incorporation of selenium; and ubiquitinilation, the addition of ubiquitin subunits (N-linked).

It is within the scope of the methods provided herein that the polypeptide is labelled with a molecular label. A molecular label may be a modification to the polypeptide which promotes the detection of the polypeptide in the methods provided herein. For example the label may be a modification to the polypeptide which alters the signal obtained as conjugate is characterised. For example, the label may interfere with a flux of ions through the nanopore. In such a manner, the label may improve the sensitivity of the methods.

In some embodiments the polypeptide contains one or more cross-linked sections, e.g. C-C bridges. In some embodiments the polypeptides is not cross-linked prior to being characterised using the disclosed methods.

In some embodiments the polypeptide comprises sulphide-containing amino acids and thus has the potential to form disulphide bonds. Typically, in such embodiments, the polypeptide is reduced using a reagent such as DTT (Dithiothreitol) or TCEP (tris(2-carboxyethyl)phosphine) prior to being characterised using the disclosed methods.

In some embodiments the polypeptide is a full length protein or naturally occurring polypeptide. In some embodiments a protein or naturally occurring polypeptide is fragmented prior to conjugation to the polynucleotide. In some embodiments the protein or polypeptide is chemically or enzymatically fragmented. In some embodiments polypeptides or polypeptide fragments can be conjugated to form a longer target polypeptide.

The polypeptide can be a polypeptide of any suitable length. In some embodiments the polypeptide has a length of from about 2 to about 300 peptide units. In some embodiments the polypeptide has a length of from about 2 to about 100 peptide units, for example from about 2 to about 50 peptide units, e.g. from about 2 to about 40 peptide units, such as from about 2 to about 30 peptide units, e.g. from about 2 to about 25 peptide units, e.g. from about 2 to about 20 peptide units; or from about 3 to about 50 peptide units, e.g. from about 3 to about 40 peptide units, such as from about 3 to about 30 peptide units, e.g. from about 3 to about 25 peptide units, e.g. from about 3 to about 20 peptide units; or from about 5 to about 50 peptide units, e.g. from about 5 to about 40 peptide units, such as from about 5 to about 30 peptide units, such as from about 5 to about 25 peptide units, e.g. from about 5 to about 20 peptide units; e.g. from about 7 to about 16 peptide units, such as from about 9 to about 12 peptide units; or from about 16 to about 25 peptide units, such as from about 18 to about 22 peptide units.

Any number of polypeptides can be characterised in the disclosed methods. For instance, the method may comprise characterising 2, 3, 4, 5, 6, 7, 8, 9, 10, 20, 30, 50, 100 or more polypeptides. If two or more polypeptides are used, they may be different polypeptides or two or more instances of the same polypeptide.

It will thus be apparent that the measurements taken in the disclosed methods are typically characteristic of one or more characteristics of the polypeptide selected from (i) the length of the polypeptide, (ii) the identity of the polypeptide, (iii) the sequence of the polypeptide, (iv) the secondary structure of the polypeptide and (v) whether or not the polypeptide is modified. In typical embodiments the measurements are characteristic of the sequence of the polypeptide or whether or not the polypeptide is modified, e.g. by one or more post-translational modifications. In some embodiments the measurements are characteristics of the sequence of the polypeptide.

In some embodiments the polypeptide is in a relaxed form. In some embodiments the polypeptide is held in a linearized form. Holding the polypeptide in a linearized form can facilitate the characterisation of the polypeptide on a residue-by-residue basis as "bunching up" of the polypeptide within the nanopore is prevented.

The polypeptide can be held in a linearized form using any suitable means.

For example, if the polypeptide is charged the polypeptide can be held in a linearized form by applying a voltage.

If the polypeptide is not charged or is only weakly charged then the charge can be altered or controlled by adjusting the pH. For example, the polypeptide can be held in a linearized form by using high pH to increase the relative negative charge of the polypeptide. Increasing the negative charge of the polypeptide allows it to be held in a linearized form under e.g. a positive voltage. Alternatively, the polypeptide can be held in a linearized form by using low pH to increase the relative positive charge of the polypeptide. Increasing the positive charge of the polypeptide allows it to be held in a linearized form under e.g. a negative voltage. In the disclosed methods a polynucleotide-handling protein is used to control the movement of a polynucleotide with respect to a nanopore. As a polynucleotide is typically negatively charged it is generally most suitable to increase the linearization of the polypeptide by increasing the pH thus making the polypeptide more negatively charged, in common with the polynucleotide. In this way, the conjugate retains an overall negative charge and thus can readily move e.g. under an applied voltage.

The polypeptide can be held in a linearized form by using suitable denaturing conditions. Suitable denaturing conditions include, for example, the presence of appropriate concentrations of denaturants such as guanidine HCl and/or urea. The concentration of such denaturants to use in the disclosed methods is dependent on the target polypeptide to be characterised in the methods and can be readily selected by those of skill in the art.

The polypeptide can be held in a linearized form by using suitable detergents. Suitable detergents for use in the disclosed methods include SDS (sodium dodecyl sulfate).

The polypeptide can be held in a linearized form by carrying out the disclosed methods at an elevated temperature. Increasing the temperature overcomes intra-strand bonding and allows the polypeptide to adopt a linearized form.

The polypeptide can be held in a linearized form by carrying out the disclosed methods under strong electro-osmotic forces. Such forces can be provided by using asymmetric salt conditions and/or providing suitable charge in the channel of the nanopore. The charge in the channel of a protein nanopore can be altered e.g. by mutagenesis. Altering the charge of a nanopore is well within the capacity of those skilled in the art. Altering the charge of a nanopore generates strong electro-osmotic forces from the unbalanced flow of cations and anions through the nanopore when a voltage potential is applied across the nanopore.

The polypeptide can be held in a linearized form by passing it through a structure such an array of nanopillars, through a nanoslit or across a nanogap. In some embodiments the physical constraints of such structures can force the polypeptide to adopt a linearized form.

### Formation of the conjugate

As explained in more detail herein, the conjugate comprises a polynucleotide conjugated to the target polypeptide.

The target polypeptide can be conjugate to the polynucleotide at any suitable position. For example, the polypeptide can be conjugated to the polynucleotide at the N-terminus or the C-terminus of the polypeptide. The polypeptide can be conjugated to the polynucleotide via a side chain group of a residue (e.g. an amino acid residue) in the polypeptide.

In some embodiments the target polypeptide has a naturally occurring reactive functional group which can be used to facilitate conjugation to the polynucleotide. For example, a cysteine residue can be used to form a disulphide bond to the polynucleotide or to a modified group thereon.

In some embodiments the target polypeptide is modified in order to facilitate its conjugation to the polynucleotide. For example, in some embodiments the polypeptide is modified by attaching a moiety comprising a reactive functional group for attaching to the polynucleotide. For example, in some embodiments the polypeptide can be extended at the N-terminus or the C-terminus by one or more residues (e.g. amino acid residues) comprising one or more reactive functional groups for reacting with a corresponding reactive functional group on the polynucleotide. For example, in some embodiments the polypeptide can be extended at the N-terminus and/or the C-terminus by one or more cysteine residues. Such residues can be used for attachment to the polynucleotide portion of the conjugate, e.g. by maleimide chemistry (e.g. by reaction of cysteine with an azido-maleimide compound such as azido-[Pol]-maleimide wherein [Pol] is typically a short chain polymer such as PEG, e.g. PEG2, PEG3, or PEG4; followed by coupling to appropriately functionalised polynucleotide e.g. polynucleotide carrying a BCN group for reaction with the azide). Such chemistry is described in Example 2. For avoidance of doubt, when the polypeptide comprises an appropriate naturally occurring residue at the N- and/or C-terminus (e.g. a naturally occurring cysteine residue at the N- and/or C-terminus) then such residue(s) can be used for attachment to the polynucleotide.

In some embodiments a residue in the target polypeptide is modified to facilitate attachment of the target polypeptide to the polynucleotide. In some embodiments a residue (e.g. an amino acid residue) in the polypeptide is chemically modified for attachment to the polynucleotide. In some embodiments a residue (e.g. an amino acid residue) in the polypeptide is enzymatically modified for attachment to the polynucleotide.

The conjugation chemistry between the polynucleotide and the polypeptide in the conjugate is not particularly limited. Any suitable combination of reactive functional groups can be used. Many suitable reactive groups and their chemical targets are known in the art. Some exemplary reactive groups and their corresponding targets include aryl azides which may react with amine, carbodiimides which may react with amines and carboxyl groups, hydrazides which may react with carbohydrates, hydroxmethyl phosphines which may react with amines, imidoesters which may react with amines, isocyanates which may react with hydroxyl groups, carbonyls which may react with hydrazines, maleimides which may react with sulfhydryl groups, NHS-esters which may react with amines, PFP-esters which may react with amines, psoralens which may react with thymine, pyridyl disulfides which may react with sulfhydryl groups, vinyl sulfones which may react with sulfhydryl amines and hydroxyl groups, vinylsulfonamides, and the like.

Other suitable chemistry for conjugating the polypeptide to the polynucleotide includes click chemistry. Many suitable click chemistry reagents are known in the art. Suitable examples of click chemistry include, but are not limited to, the following:
(a) copper(I)-catalyzed azide-alkyne cycloadditions (azide alkyne Huisgen cycloadditions);
(b) strain-promoted azide-alkyne cycloadditions; including alkene and azide [3+2] cycloadditions; alkene and tetrazine inverse-demand Diels-Alder reactions; and alkene and tetrazole photoclick reactions;
(c) copper-free variant of the 1,3 dipolar cycloaddition reaction, where an azide reacts with an alkyne under strain, for example in a cyclooctane ring such as in bicycle[6.1.0]nonyne (BCN);
(d) the reaction of an oxygen nucleophile on one linker with an epoxide or aziridine reactive moiety on the other; and
(e) the Staudinger ligation, where the alkyne moiety can be replaced by an aryl phosphine, resulting in a specific reaction with the azide to give an amide bond.

Any reactive group may be used to form the conjugate. Some suitable reactive groups include [1, 4-Bis[3-(2-pyridyldithio)propionamido]butane; 1,1 1-bis-maleimidotriethyleneglycol; 3,3'-dithiodipropionic acid di(N-hydroxysuccinimide ester); ethylene glycol-bis(succinic acid N-hydroxysuccinimide ester); 4,4'-diisothiocyanatostilbene-2,2'-disulfonic acid disodium salt; Bis[2-(4-azidosalicylamido)ethyl] disulphide; 3-(2-pyridyldithio)propionic acid N-hydroxysuccinimide ester; 4-maleimidobutyric acid N-hydroxysuccinimide ester; Iodoacetic acid N-hydroxysuccinimide ester; S-acetylthioglycolic acid N-hydroxysuccinimide ester; azide-PEG-maleimide; and alkyne-PEG-maleimide. The reactive group may be any of those disclosed in WO 2010/086602, particularly in Table 3 of that application.

In some embodiments the reactive functional group is comprised in the polynucleotide and the target functional group is comprised in the polypeptide prior to the conjugation step. In other embodiments the reactive functional group is comprised in the polypeptide and the target functional group is comprised in the polynucleotide prior to the conjugation step. In some embodiments the reactive functional group is attached directly to the polypeptide. In some embodiments the reactive functional group is attached to the polypeptide via a spacer. Any suitable spacer can be used. Suitable spacers include for example alkyl diamines such as ethyl diamine, etc.

As will be apparent from the above discussed, in some embodiments the conjugate comprises a plurality of polypeptide sections and/or a plurality of polynucleotide sections. For example the conjugate may comprise a structure of the form ...-P-N-P-N-P-N... wherein P is a polypeptide and N is a polynucleotide. In such embodiments the polynucleotide-handling protein sequentially controls the N portions of the conjugate with respect to the nanopore and thus sequentially controls the movement of the P sections with respect to the nanopore, thus allowing the sequential characterisation of the P sections. In such embodiments the plurality of polynucleotides and polypeptides may be conjugated together by the same or different chemistries.

As explained herein, the conjugate may comprise a leader. Any suitable leader may be used, as explained herein. In some embodiments the leader is a polynucleotide. In embodiments wherein the leader is a polynucleotide the leader may be the same sort of polynucleotide as the polynucleotide used in the conjugate, or it may be a different type of polynucleotide. For example, the polynucleotide in the conjugate may be DNA and the leader may be RNA or vice versa.

In some embodiments the leader is a charged polymer, e.g. a negatively charged polymer. In some embodiments the leader comprises a polymer such as PEG or a polysaccharide. In such embodiments the leader may be from 10 to 150 monomer units (e.g. ethylene glycol or saccharide units) in length, such as from 20 to 120, e.g. 30 to 100, for example 40 to 80 such as 50 to 70 monomer units (e.g. ethylene glycol or saccharide units) in length.

### Polynucleotide

As explained in more detail herein, the methods provided herein comprise conjugating a polypeptide to a polynucleotide and controlling the movement of the conjugate with respect to a nanopore using a polynucleotide-handling protein.

In the disclosed methods, any suitable polynucleotide can be used.

In some embodiments the polynucleotide is secreted from cells. Alternatively, polynucleotide can be produced inside cells such that it must be extracted from cells for use in the disclosed methods.

A polynucleotide may be provided as an impure mixture of one or more polynucleotides and one or more impurities. Impurities may comprise truncated forms of polynucleotides which are distinct from the polynucleotide for use in the formation of the conjugate. For example the polynucleotide for use in the formation of the conjugate may be genomic DNA and impurities may comprise fractions of genomic DNA, plasmids, etc. The target polynucleotide may be a coding region of genomic DNA and undesired polynucleotides may comprise non-coding regions of DNA.

Examples of polynucleotides include DNA and RNA. The bases in DNA and RNA may be distinguished by their physical size.

A polynucleotide or nucleic acid may comprise any combination of any nucleotides. The nucleotides can be naturally occurring or artificial. One or more nucleotides in the polynucleotide can be oxidized or methylated. One or more nucleotides in the polynucleotide may be damaged. For instance, the polynucleotide may comprise a pyrimidine dimer. Such dimers are typically associated with damage by ultraviolet light and are the primary cause of skin melanomas.

One or more nucleotides in the polynucleotide may be modified, for instance with a label or a tag, for which suitable examples are known by a skilled person. The polynucleotide may comprise one or more spacers. An adapter, for example a sequencing adapter, may be comprised in the polynucleotide. Adapters, tags and spacers are described in more detail herein.

Examples of modified bases are disclosed herein and can be incorporated into the polynucleotide by means known in the art, e.g. by polymerase incorporation of modified nucleotide triphosphates during strand copying (e.g. in PCR) or by polymerase fill-in methods. In some embodiments one or more bases can be modified by chemical means using reagents known in the art.

A nucleotide typically contains a nucleobase, a sugar and at least one phosphate group. The nucleobase and sugar form a nucleoside. The nucleobase is typically heterocyclic. Nucleobases include, but are not limited to, purines and pyrimidines and more specifically adenine (A), guanine (G), thymine (T), uracil (U) and cytosine (C). The sugar is typically a pentose sugar. Nucleotide sugars include, but are not limited to, ribose and deoxyribose. The sugar is preferably a deoxyribose. The polynucleotide preferably comprises the following nucleosides: deoxyadenosine (dA), deoxyuridine (dU) and/or thymidine (dT), deoxyguanosine (dG) and deoxycytidine (dC). The nucleotide is typically a ribonucleotide or deoxyribonucleotide. The nucleotide typically contains a monophosphate, diphosphate or triphosphate. The nucleotide may comprise more than three phosphates, such as 4 or 5 phosphates. Phosphates may be attached on the 5' or 3' side of a nucleotide. The nucleotides in the polynucleotide may be attached to each other in any manner. The nucleotides are typically attached by their sugar and phosphate groups as in nucleic acids. The nucleotides may be connected via their nucleobases as in pyrimidine dimers.

A polynucleotide may be double stranded or single stranded.

In some embodiments the polynucleotide is single stranded DNA. In some embodiments the polynucleotide is single stranded RNA. In some embodiments the polynucleotide is a single-stranded DNA-RNA hybrid. DNA-RNA hybrids can be prepared by ligating single stranded DNA to RNA or vice versa. The polynucleotide is most typically single stranded deoxyribonucleic acid (DNA) or single stranded ribonucleic nucleic acid (RNA).

In some embodiments the polynucleotide is double stranded DNA. In some embodiments the polynucleotide is double stranded RNA. In some embodiments the polynucleotide is a double-stranded DNA-RNA hybrid. Double-stranded DNA-RNA hybrids can be prepared from single-stranded RNA by reverse transcribing the cDNA complement.

The polynucleotide can be any length. For example, the polynucleotide can be at least 10, at least 50, at least 100, at least 150, at least 200, at least 250, at least 300, at least 400 or at least 500 nucleotides or nucleotide pairs in length. The polynucleotide can be 1000 or more nucleotides or nucleotide pairs, 5000 or more nucleotides or nucleotide pairs in length or 100000 or more nucleotides or nucleotide pairs in length.

More typically, the polynucleotide has a length of from about 1 to about 10,000 nucleotides or nucleotide pairs. such as from about 1 to about 1000 nucleotides or nucleotide pairs (e.g. from about 10 to about 1000 nucleotides or nucleotide pairs), e.g. from about 5 to about 500 nucleotides or nucleotide pairs, such as from about 10 to about 100 nucleotides or nucleotide pairs, e.g. from about 20 to about 80 nucleotides or nucleotide pairs such as from about 30 to about 50 nucleotides or nucleotide pairs.

Any number of polynucleotides can be used in the disclosed methods. For instance, the method may comprise using 2, 3, 4, 5, 6, 7, 8, 9, 10, 20, 30, 50, 100 or more polynucleotides. If two or more polynucleotides are used, they may be different polynucleotides or two instances of the same polynucleotide. The polynucleotide can be naturally occurring or artificial.

Nucleotides can have any identity, and include, but are not limited to, adenosine monophosphate (AMP), guanosine monophosphate (GMP), thymidine monophosphate (TMP), uridine monophosphate (UMP), 5-methylcytidine monophosphate, 5-hydroxymethylcytidine monophosphate, cytidine monophosphate (CMP), cyclic adenosine monophosphate (cAMP), cyclic guanosine monophosphate (cGMP), deoxyadenosine monophosphate (dAMP), deoxyguanosine monophosphate (dGMP), deoxythymidine monophosphate (dTMP), deoxyuridine monophosphate (dUMP), deoxycytidine monophosphate (dCMP) and deoxymethylcytidine monophosphate. The nucleotides are preferably selected from AMP, TMP, GMP, CMP, UMP, dAMP, dTMP, dGMP, dCMP and dUMP. A nucleotide may be abasic (i.e. lack a nucleobase). A nucleotide may also lack a nucleobase and a sugar (i.e. is a C3 spacer).

The polynucleotide may comprise the products of a PCR reaction, genomic DNA, the products of an endonuclease digestion and/or a DNA library. The polynucleotide may be obtained from or extracted from any organism or microorganism. The polynucleotide may be obtained from a human or animal, e.g. from urine, lymph, saliva, mucus, seminal fluid or amniotic fluid, or from whole blood, plasma or serum. The polynucleotide may be obtained from a plant e.g. a cereal, legume, fruit or vegetable. The polynucleotide may comprise genomic DNA. The genomic DNA may be fragmented. The DNA may be fragmented by any suitable method. For example, methods of fragmenting DNA are known in the art, Such methods may use a transposase, such as a MuA transposase. Often the genomic DNA is not fragmented.

It is within the scope of the methods provided herein that the polynucleotide is labelled with a molecular label. A molecular label may be a modification to the polynucleotide which promotes the detection of the polynucleotide or conjugate in the methods provided herein. For example the label may be a modification to the polynucleotide which alters the signal obtained as conjugate is characterised. For example, the label may interfere with a flux of ions through the nanopore. In such a manner, the label may improve the sensitivity of the methods.

### Adapters

In some embodiments of the methods provided herein, the polynucleotide has a polynucleotide adapter attached thereto. An adapter typically comprises a polynucleotide strand capable of being attached to the end of the polynucleotide.

In some embodiments the adapter is attached to the polynucleotide before the conjugate with the polypeptide is formed. In some embodiments the adapter is attached to the conjugate of the polynucleotide and the polypeptide.

Accordingly, in some embodiments the methods comprise attaching an adapter (e.g. an adapter as described herein) to the polynucleotide and forming the conjugate by conjugating the polynucleotide/adapter construct to the target polypeptide. In some embodiments the conjugate is formed by attaching an adapter (e.g. an adapter as described herein) to the polynucleotide and forming the conjugate by attaching the adapter to the target polypeptide.

In some embodiments the adapter may be chosen or modified in order to provide a specific site for the conjugation to the polynucleotide.

An adapter may be attached to just one end of the polynucleotide or conjugate. A polynucleotide adapter may be added to both ends of the polynucleotide or conjugate. Alternatively, different adapters may be added to the two ends of the polynucleotide or conjugate.

Adapters may be added to both strands of double stranded polynucleotides. Adapter may be added to single stranded polynucleotides. Methods of adding adapters to polynucleotides are known in the art. Adapters may be attached to polynucleotides, for example, by ligation, by click chemistry, by tagmentation, by topoisomerisation or by any other suitable method.

In one embodiment, the or each adapter is synthetic or artificial. Typically, the or each adapter comprises a polymer as described herein. In some embodiments, the or each adapter comprises a spacer as described herein. In some embodiments, the or each adapter comprises a polynucleotide. The or each polynucleotide adapter may comprise DNA, RNA, modified DNA (such as abasic DNA), RNA, PNA, LNA, BNA and/or PEG. Usually, the or each adapter comprises single stranded and/or double stranded DNA or RNA. The adapter may comprise the same type of polynucleotide as the polynucleotide strand to which it is attached. The adapter may comprise a different type of polynucleotide to the polynucleotide strand to which it is attached. In some embodiments the polynucleotide strand used in the disclosed methods is a single stranded DNA strand and the adapter comprises DNA or RNA, typically single stranded DNA. In some embodiments the polynucleotide is a double stranded DNA strand and the adapter comprises DNA or RNA, e.g. double or single stranded DNA.

In some embodiments, an adapter may be a bridging moiety. A bridging moiety may be used to connect the two strands of a double-stranded polynucleotide. For example, in some embodiments a bridging moiety is used to connect the template strand of a double stranded polynucleotide to the complement strand of the double stranded polynucleotide.

A bridging moiety typically covalently links the two strands of a double-stranded polynucleotide. The bridging moiety can be anything that is capable of linking the two strands of a double-stranded polynucleotide, provided that the bridging moiety does not interfere with movement of the polynucleotide with respect to the nanopore. Suitable bridging moieties include, but are not limited to a polymeric linker, a chemical linker, a polynucleotide or a polypeptide. Preferably, the bridging moiety comprises DNA, RNA, modified DNA (such as abasic DNA), RNA, PNA, LNA or PEG. The bridging moiety is more preferably DNA or RNA.

In some embodiments a bridging moiety is a hairpin adapter. A hairpin adapter is an adapter comprising a single polynucleotide strand, wherein the ends of the polynucleotide strand are capable of hybridising to each other, or are hybridized to each other, and wherein the middle section of the polynucleotide forms a loop. Suitable hairpin adapters can be designed using methods known in the art. In some embodiments a hairpin loop is typically 4 to 100 nucleotides in length, e.g. from 4 to 50 such as from 4 to 20 e.g. from 4 to 8 nucleotides in length. In some embodiments the bridging moiety (e.g. hairpin adapter) is attached at one end of a double-stranded polynucleotide. A bridging moiety (e.g. hairpin adapter) is typically not attached at both ends of a double-stranded polynucleotide.

In some embodiments, an adapter is a linear adapter. A linear adapter may be bound to either or both ends of a single stranded polynucleotide. When the polynucleotide is a double stranded polynucleotide, a linear adapter may be bound to either or both ends of either or both strands of the double stranded polynucleotide. A linear adapter may comprise a leader sequence as described herein. A linear adapter may comprise a portion for hybridisation with a tag (such as a pore tag) as described herein. A linear adapter may be 10 to 150 nucleotides in length, such as from 20 to 120, e.g. 30 to 100, for example 40 to 80 such as 50 to 70 nucleotides in length. A linear adapter may be single stranded. A linear adapter may be double stranded.

In some embodiments, an adapter may be a Y adapter. A Y adapter is typically a polynucleotide adapter. A Y adapter is typically double stranded and comprises (a) at one end, a region where the two strands are hybridised together and (b), at the other end, a region where the two strands are not complementary. The non-complementary parts of the strands typically form overhangs. The presence of a non-complementary region in the Y adapter gives the adapter its Y shape since the two strands typically do not hybridise to each other unlike the double stranded portion. The two single-stranded portions of the Y adapter may be the same length, or may be different lengths. For example, one single-stranded portion of the Y adapter may be 10 to 150 nucleotides in length, such as from 20 to 120, e.g. 30 to 100, for example 40 to 80 such as 50 to 70 nucleotides in length and the other single stranded portion of the Y adapter may independently by 10 to 150 nucleotides in length, such as from 20 to 120, e.g. 30 to 100, for example 40 to 80 such as 50 to 70 nucleotides in length. The double-stranded "stem" portion of the Y adapter may be e.g. from 10 to 150 nucleotides in length, such as from 20 to 120, e.g. 30 to 100, for example 40 to 80 such as 50 to 70 nucleotides in length.

An adapter may be linked to the target polynucleotide by any suitable means known in the art. The adapter may be synthesized separately and chemically attached or enzymatically ligated to the target polynucleotide. Alternatively, the adapter may be generated in the processing of the target polynucleotide. In some embodiments, the adapter is linked to the target polynucleotide at or near one end of the target polynucleotide. In some embodiments, the adapter is linked to the target polynucleotide within 50, e.g. within 20 for example within 10 nucleotides of an end of the target polynucleotide. In some embodiments the adapter is linked to the target polynucleotide at a terminus of the target polynucleotide. When a adapter is linked to the target polynucleotide the adapter may comprise the same type of nucleotides as the target polynucleotide or may comprise different nucleotides to the target polynucleotide.

Adapters which are particularly suitable for use in the disclosed methods may comprise linear homopolymeric regions (e.g. from about 5 to about 20 nucleotides, such as from about 10 to about 30 nucleotides, for example thymine or cytidine) and/or hybridisation sites for hybridising to one or more tethers or anchors (as described in more detail herein). Such adapters may also comprise reactive functional groups for binding to the target polypeptide. Click chemistry groups are particularly suitable in this regard. For example, exemplary groups for inclusion in an adapter include groups which can particulate in copper-free click chemistry, for example groups based on BCN (bicyclo[6.1.0]nonyne) and its derivatives, dibenzocyclooctyne (DBCO) groups, and the like. The reaction of such groups is well known in the art. For example, BCN groups typically react with groups such as azides, tetrazines and nitrones, which can for example incorporated in the polypeptide. DBCO groups have high reactivity toward azide groups. Other chemical groups which are particularly suitable include 2-pyridinecarboxyaldehyde (2-PCA) groups and their derivatives. For example, 6-(azidomethyl)-2-pyridinecarboxyaldehyde can react with N-terminal amino groups of peptides.

### Spacers

In some embodiments of the methods provided herein, the polynucleotide, a conjugate formed by the reaction thereof with a polypeptide, or an adapter as described herein, may comprise a spacer. For example, one or more spacers may be present in the polynucleotide adapter. For example, the polynucleotide adapter may comprise from one to about 20 spacers, e.g. from about 1 to about 10, e.g. from 1 to about 5 spacers, e.g. 1, 2, 3, 4 or 5 spacers. The spacer may comprise any suitable number of spacer units. A spacer may provide an energy barrier which impedes movement of a polynucleotide-handling protein. For example, a spacer may stall a polynucleotide-handling protein by reducing the traction of the polynucleotide-handling protein on the polynucleotide. This may be achieved for instance by using an abasic spacer i.e. a spacer in which the bases are removed from one or more nucleotides in the polynucleotide adapter. A spacer may physically block movement of a polynucleotide-handling protein, for instance by introducing a bulky chemical group to physically impede the movement of the polynucleotide-handling protein.

In some embodiments, one or more spacers are included in the polynucleotide or conjugate or in an adapter as used in the methods claimed herein in order to provide a distinctive signal when they pass through or across the nanopore, i.e. as they move with respect to the nanopore.

In some embodiments, a spacer may comprise a linear molecule, such as a polymer. Typically, such a spacer has a different structure from the polynucleotide used in the conjugate. For instance, if the polynucleotide is DNA, the or each spacer typically does not comprise DNA. In particular, if the polynucleotide is deoxyribonucleic acid (DNA) or ribonucleic acid (RNA), the or each spacer preferably comprises peptide nucleic acid (PNA), glycerol nucleic acid (GNA), threose nucleic acid (TNA), locked nucleic acid (LNA) or a synthetic polymer with nucleotide side chains. In some embodiments, a spacer may comprise one or more nitroindoles, one or more inosines, one or more acridines, one or more 2-aminopurines, one or more 2-6-diaminopurines, one or more 5-bromo-deoxyuridines, one or more inverted thymidines (inverted dTs), one or more inverted dideoxy-thymidines (ddTs), one or more dideoxy-cytidines (ddCs), one or more 5-methylcytidines, one or more 5-hydroxymethylcytidines, one or more 2'-O-Methyl RNA bases, one or more Iso-deoxycytidines (Iso-dCs), one or more Iso-deoxyguanosines (Iso-dGs), one or more C3 (OC₃H₆OPO₃) groups, one or more photo-cleavable (PC) [OC₃H₆-C(O)NHCH₂-C₆H₃NO₂-CH(CH₃)OPO₃] groups, one or more hexandiol groups, one or more spacer 9 (iSp9) [(OCH₂CH₂)₃OPO₃] groups, or one or more spacer 18 (iSp18) [(OCH₂CH₂)₆OPO₃] groups; or one or more thiol connections. A spacer may comprise any combination of these groups. Many of these groups are commercially available from IDT^{®} (Integrated DNA Technologies^{®}). For example, C3, iSp9 and iSp18 spacers are all available from IDT^{®}. A spacer may comprise any number of the above groups as spacer units.

In some embodiments, a spacer may comprise one or more chemical groups which cause a polynucleotide-handling protein to stall. In some embodiments, suitable chemical groups are one or more pendant chemical groups. The one or more chemical groups may be attached to one or more nucleobases in the polynucleotide, construct or adapter. The one or more chemical groups may be attached to the backbone of the polynucleotide adapter. Any number of appropriate chemical groups may be present, such as 2, 3, 4, 5, 6, 7, 8, 9, 10, 11, 12 or more. Suitable groups include, but are not limited to, fluorophores, streptavidin and/or biotin, cholesterol, methylene blue, dinitrophenols (DNPs), digoxigenin and/or anti-digoxigenin and dibenzylcyclooctyne groups. In some embodiments, a spacer may comprise a polymer. In some embodiments the spacer may comprise a polymer which is a polypeptide or a polyethylene glycol (PEG).

In some embodiments, a spacer may comprise one or more abasic nucleotides (i.e. nucleotides lacking a nucleobase), such as 2, 3, 4, 5, 6, 7, 8, 9, 10, 11, 12 or more abasic nucleotides. The nucleobase can be replaced by -H (idSp) or -OH in the abasic nucleotide. Abasic spacers can be inserted into target polynucleotides by removing the nucleobases from one or more adjacent nucleotides. For instance, polynucleotides may be modified to include 3-methyladenine, 7-methylguanine, 1,N6-ethenoadenine inosine or hypoxanthine and the nucleobases may be removed from these nucleotides using Human Alkyladenine DNA Glycosylase (hAAG). Alternatively, polynucleotides may be modified to include uracil and the nucleobases removed with Uracil-DNA Glycosylase (UDG). In one embodiment, the one or more spacers do not comprise any abasic nucleotides.

Methods of stalling a polynucleotide-handling protein such as a helicase on a polynucleotide adapter using a spacer are described in WO 2014/135838.

### Anchors

In some embodiments, a polynucleotide, conjugate thereof with a polypeptide, or an adapter attached thereto may comprise a membrane anchor or a transmembrane pore anchor e.g. attached to the adapter. In one embodiment the anchor aids in characterisation of the conjugate in accordance with the methods disclosed herein. For example, a membrane anchor or transmembrane pore anchor may promote localisation of the conjugate around a nanopore in a membrane.

The anchor may be a polypeptide anchor and/or a hydrophobic anchor that can be inserted into the membrane. In one embodiment, the hydrophobic anchor is a lipid, fatty acid, sterol, carbon nanotube, polypeptide, protein or amino acid, for example cholesterol, palmitate or tocopherol. The anchor may comprise thiol, biotin or a surfactant.

In one aspect the anchor may be biotin (for binding to streptavidin), amylose (for binding to maltose binding protein or a fusion protein), Ni-NTA (for binding to poly-histidine or poly-histidine tagged proteins) or peptides (such as an antigen).

In one embodiment, the anchor comprises a linker, or 2, 3, 4 or more linkers. Preferred linkers include, but are not limited to, polymers, such as polynucleotides, polyethylene glycols (PEGs), polysaccharides and polypeptides. These linkers may be linear, branched or circular. For instance, the linker may be a circular polynucleotide. The adapter may hybridise to a complementary sequence on a circular polynucleotide linker. The one or more anchors or one or more linkers may comprise a component that can be cut or broken down, such as a restriction site or a photolabile group. The linker may be functionalised with maleimide groups to attach to cysteine residues in proteins. Suitable linkers are described in WO 2010/086602.

In one embodiment, the anchor is cholesterol or a fatty acyl chain. For example, any fatty acyl chain having a length of from 6 to 30 carbon atom, such as hexadecanoic acid, may be used. Examples of suitable anchors and methods of attaching anchors to adapters are disclosed in WO 2012/164270 and WO 2015/150786.

### Controlling movement of the conjugate with respect to a nanopore

As explained in more detail above, the methods provided herein comprise contacting the conjugate with a polynucleotide-handling protein capable of controlling the movement of the polynucleotide with respect to a nanopore, wherein the polynucleotide-handling protein is modified by covalently closing a polynucleotide-unbinding opening through which a polynucleotide strand may unbind; and taking one or more measurements characteristic of the polypeptide as the conjugate moves with respect to the nanopore.

The movement of the conjugate with respect to the nanopore may be driven by any suitable means. In some embodiments, the movement of the conjugate is driven by a physical or chemical force (potential). In some embodiments the physical force is provided by an electrical (e.g. voltage) potential or a temperature gradient, etc.

In some embodiments, the conjugate moves with respect to the nanopore as an electrical potential is applied across the nanopore. Polynucleotides are negatively charged, and so applying a voltage potential across a nanopore will cause the polynucleotides to move with respect to the nanopore under the influence of the applied voltage potential. For example, if a positive voltage potential is applied to the *trans* side of the nanopore relative to the *cis* side of the nanopore, then this will induce a negatively charged analyte to move from the *cis* side of the nanopore to the *trans* side of the nanopore. Similarly, if a positive voltage potential is applied to the *trans* side of the nanopore relative to the *cis* side of the nanopore then this will impede the movement of a negatively charged analyte from the *trans* side of the nanopore to the *cis* side of the nanopore. The opposite will occur if a negative voltage potential is applied to the *trans* side of the nanopore relative to the *cis* side of the nanopore. Apparatuses and methods of applying appropriate voltages are described in more detail herein.

In some embodiments the chemical force is provided by a concentration (e.g. pH) gradient.

In some embodiments the polynucleotide-handling protein controls the movement of the conjugate in the same direction as the physical or chemical force (potential). For example, in some embodiments a positive voltage potential is applied to the *trans* side of the nanopore relative to the *cis* side of the nanopore, and the polynucleotide-handling protein controls the movement of the conjugate from the *cis* side of the nanopore to the *trans* side of the nanopore. In some embodiments a positive voltage potential is applied to the *cis* side of the nanopore relative to the *trans* side of the nanopore, and the polynucleotide-handling protein controls the movement of the conjugate from the *trans* side of the nanopore to the *cis* side of the nanopore.

In some embodiments the polynucleotide-handling protein controls the movement of the conjugate in the opposite direction to the physical or chemical force (potential). For example, in some embodiments a positive voltage potential is applied to the *trans* side of the nanopore relative to the *cis* side of the nanopore, and the polynucleotide-handling protein controls the movement of the conjugate from the *trans* side of the nanopore to the *cis* side of the nanopore. In some embodiments a positive voltage potential is applied to the *cis* side of the nanopore relative to the *trans* side of the nanopore, and the polynucleotide-handling protein controls the movement of the conjugate from the *cis* side of the nanopore to the *trans* side of the nanopore.

In some embodiments the movement of the conjugate is driven by the polynucleotide-handling protein in the absence of an applied potential.

In the disclosed methods, the polynucleotide-handling protein is capable of controlling the movement of the polynucleotide with respect to a nanopore. In other words, the polynucleotide-handling protein is capable of controlling the movement of the conjugate. In some embodiments the polynucleotide-handling protein is capable of controlling the movement of the polynucleotide and the polypeptide.

Suitable polynucleotide-handling proteins are also known as motor proteins or polynucleotide-handling enzymes. Suitable polynucleotide-handling proteins are known in the art and some exemplary polynucleotide-handling proteins are described in more detail below.

In one embodiment, a motor protein is or is derived from a polynucleotide handling enzyme. A polynucleotide handling enzyme is a polypeptide that is capable of interacting with and modifying at least one property of a polynucleotide. The enzyme may modify the polynucleotide by cleaving it to form individual nucleotides or shorter chains of nucleotides, such as di- or trinucleotides. The enzyme may modify the polynucleotide by orienting it or moving it to a specific position.

In some embodiments, a polynucleotide-handling protein can be present on the conjugate prior to its contact with a nanopore. For example, a polynucleotide-handling protein can be present on the polynucleotide in the conjugate. In some embodiments the polynucleotide-handling protein is present on an adapter comprising part of the conjugate, or can be otherwise present on a portion of the conjugate.

In the provided methods, the polynucleotide-handling protein is modified by covalently closing a polynucleotide-unbinding opening through which a polynucleotide strand may unbind, thereby preventing the polynucleotide binding protein from disengaging from the conjugate when the polynucleotide-handling protein contacts a portion of the conjugate comprising a polypeptide.

In some embodiments the polynucleotide-handling protein is capable of remaining bound to the conjugate when the portion of the conjugate in contact with the active site of the polynucleotide-handling protein comprises a polypeptide. In other words, in some embodiments the polynucleotide-handling protein does not dissociate from the conjugate when the polynucleotide-handling protein contacts the polypeptide portion of the conjugate. In some embodiments the polynucleotide-handling protein moves freely with respect to the polypeptide portion until one or more subsequent polynucleotide portions of the conjugate are contacted.

The polynucleotide-handling protein is modified to prevent it from disengaging from the conjugate, polynucleotide or adapter (other than by passing off the end of the conjugate, polynucleotide or adapter) when the polynucleotide-handling protein contacts a portion of the conjugate comprising a polypeptide. Such modified polynucleotide-handling proteins are particularly suitable for use in the disclosed methods.

The polynucleotide-handling protein can be adapted in any suitable way. For example, the polynucleotide-handling protein can be loaded onto the polynucleotide, conjugate or adapter and then modified in order to prevent it from disengaging. Alternatively, the polynucleotide-handling protein can be modified to prevent it from disengaging before it is loaded onto the polynucleotide, conjugate or adapter. Modification of a polynucleotide-handling protein in order to prevent it from disengaging from a polynucleotide, conjugate or adapter can be achieved using methods known in the art, such as those discussed in WO 2014/013260, and with particular reference to passages describing the modification of polynucleotide-handling proteins (polynucleotide binding proteins) such as helicases in order to prevent them from disengaging with polynucleotide strands.

In the provided methods, the polynucleotide-handling protein is modified by covalently closing a polynucleotide-unbinding opening; e.g. a cavity, cleft or void through which a polynucleotide strand may pass when the polynucleotide-handling protein disengages from the strand, thereby preventing the polynucleotide binding protein from disengaging from the conjugate when the polynucleotide-handling protein contacts a portion of the conjugate comprising a polypeptide.

In some embodiments, the polynucleotide-unbinding opening for a given motor protein (polynucleotide-handling protein) can be determined by reference to its structure, e.g. by reference to its X-ray crystal structure. The X-ray crystal structure may be obtained in the presence and/or the absence of a polynucleotide substrate. In some embodiments, the location of a polynucleotide-unbinding opening in a given polynucleotide-handling protein may be deduced or confirmed by molecular modelling using standard packages known in the art. In some embodiments, the polynucleotide-unbinding opening may be transiently produced by movement of one or more parts e.g. one or more domains of the polynucleotide-handling protein.

The polynucleotide-handling protein (motor protein) is modified by closing the polynucleotide-unbinding opening. Closing the polynucleotide-unbinding opening may therefore prevent the polynucleotide-handling protein from disengaging from the polypeptide portion of the conjugate as well as preventing it from disengaging from the polynucleotide or adapter. The motor protein is modified by covalently closing the polynucleotide-unbinding opening. In some embodiments, a motor protein for addressing in this way is a helicase, as described herein.

The polynucleotide-handling protein may be chosen or selected according to the polynucleotide to be used in the conjugate characterised in the methods disclosed herein. Alternatively, the polynucleotide may be chosen or selected according to the polynucleotide-handling protein used to control the movement of the conjugate. For example, typically DNA motor proteins can be used when the polynucleotide is DNA. RNA motor protein can be used when the polynucleotide is RNA. Motor proteins which can process both DNA and RNA can be used when the polynucleotide is a hybrid of DNA and RNA.

In one embodiment, the motor protein is derived from a member of any of the Enzyme Classification (EC) groups 3.1.11, 3.1.13, 3.1.14, 3.1.15, 3.1.16, 3.1.21, 3.1.22, 3.1.25, 3.1.26, 3.1.27, 3.1.30 and 3.1.31.

In some embodiments of the claimed methods, the motor protein is a helicase, a polymerase, an exonuclease, a topoisomerase, or a variant thereof.

In one embodiment, the motor protein is an exonuclease. Suitable enzymes include, but are not limited to, exonuclease I from *E. coli* (SEQ ID NO: 1), exonuclease III enzyme from *E. coli* (SEQ ID NO: 2), RecJ from *T. thermophilus* (SEQ ID NO: 3) and bacteriophage lambda exonuclease (SEQ ID NO: 4), TatD exonuclease and variants thereof. Three subunits comprising the sequence shown in SEQ ID NO: 3 or a variant thereof interact to form a trimer exonuclease.

In one embodiment, the motor protein is a polymerase. The polymerase may be PyroPhage^{®} 3173 DNA Polymerase (which is commercially available from Lucigen^{®} Corporation), SD Polymerase (commercially available from Bioron^{®}), Klenow from NEB or variants thereof. In one embodiment, the enzyme is Phi29 DNA polymerase (SEQ ID NO: 5) or a variant thereof. Modified versions of Phi29 polymerase that may be used in the disclosed methods are disclosed in US Patent No. 5,576,204.

In embodiments of the methods provided herein which comprise controlling the movement of the conjugate by synthesizing a strand complementary to the polynucleotide, the polynucleotide-handling protein is typically a polymerase, e.g. a polymerase as described herein.

In one embodiment the polynucleotide-handling protein is a topoisomerase. In one embodiment, the topoisomerase is a member of any of the Moiety Classification (EC) groups 5.99.1.2 and 5.99.1.3. The topoisomerase may be a reverse transcriptase, which are enzymes capable of catalysing the formation of cDNA from a RNA template. They are commercially available from, for instance, New England Biolabs^{®} and Invitrogen^{®}.

In one embodiment, the polynucleotide-handling protein is a helicase. Any suitable helicase can be used in accordance with the methods provided herein. For example, the or each motor protein used in accordance with the present disclosure may be independently selected from a Hel308 helicase, a RecD helicase, a TraI helicase, a TrwC helicase, an XPD helicase, and a Dda helicase, or a variant thereof. Monomeric helicases may comprise several domains attached together. For instance, TraI helicases and TraI subgroup helicases may contain two RecD helicase domains, a relaxase domain and a C-terminal domain. The domains typically form a monomeric helicase that is capable of functioning without forming oligomers. Particular examples of suitable helicases include Hel308, NS3, Dda, UvrD, Rep, PcrA, Pif1 and TraI. These helicases typically work on single stranded DNA. Examples of helicases that can move along both strands of a double stranded DNA include FtfK and hexameric enzyme complexes, or multisubunit complexes such as RecBCD. NS3 helicases are particularly suitable for use in the disclosed methods as they are capable of processing both DNA and RNA and so can be used in embodiments of the disclosed methods in which the target double stranded nucleic acid is a DNA-RNA hybrid.

Hel308 helicases are described in publications such as WO 2013/057495. RecD helicases are described in publications such as WO 2013/098562. XPD helicases are described in publications such as WO 2013/098561. Dda helicases are described in publications such as WO 2015/055981 and WO 2016/055777.

In one embodiment the helicase comprises the sequence shown in SEQ ID NO: 6 (Trwc Cba) or a variant thereof, the sequence shown in SEQ ID NO: 7 (Hel308 Mbu) or a variant thereof or the sequence shown in SEQ ID NO: 8 (Dda) or a variant thereof. Variants may differ from the native sequences in any of the ways discussed herein. An example variant of SEQ ID NO: 8 comprises E94C/A360C. A further example variant of SEQ ID NO: 8 comprises E94C/A360C and then (ΔM1)G1G2 (i.e. deletion of M1 and then addition of G1 and G2).

In some embodiments a motor protein (e.g. a helicase) can control the movement of the conjugate in at least two active modes of operation (when the motor protein is provided with all the necessary components to facilitate movement, e.g. fuel and cofactors such as ATP and Mg²⁺ discussed herein) and one inactive mode of operation (when the motor protein is not provided with the necessary components to facilitate movement).

When provided with all the necessary components to facilitate movement (i.e. in the active modes), the motor protein (e.g. helicase) moves along the polynucleotide in a 5' to 3' or a 3' to 5' direction (depending on the motor protein). The motor protein can be used to either move the conjugate away from (e.g. out of) the pore (e.g. against an applied force) or the conjugate towards (e.g. into) the pore (e.g. with an applied force). For example, when the end of the conjugate towards which the motor protein moves is captured by a pore, the motor protein works against the direction of the force and pulls the threaded conjugate out of the pore (e.g. into the *cis* chamber). However, when the end away from which the motor protein moves is captured in the pore, the motor protein works with the direction of the force and pushes the threaded conjugate into the pore (e.g. into the *trans* chamber).

When the motor protein (e.g. helicase) is not provided with the necessary components to facilitate movement (i.e. in the inactive mode) it can bind to the conjugate and act as a brake slowing the movement of the construct when it is moved with respect to a nanopore, e.g. by being pulled into the pore by a force. In the inactive mode, it does not matter which end of the conjugate is captured, it is the applied force which determines the movement of the conjugate with respect to the pore, and the polynucleotide binding protein acts as a brake. When in the inactive mode, the movement control of the conjugate by the polynucleotide binding protein can be described in a number of ways including ratcheting, sliding and braking.

A motor protein typically requires fuel in order to handle the processing of polynucleotides. Fuel is typically free nucleotides or free nucleotide analogues. The free nucleotides may be one or more of, but are not limited to, adenosine monophosphate (AMP), adenosine diphosphate (ADP), adenosine triphosphate (ATP), guanosine monophosphate (GMP), guanosine diphosphate (GDP), guanosine triphosphate (GTP), thymidine monophosphate (TMP), thymidine diphosphate (TDP), thymidine triphosphate (TTP), uridine monophosphate (UMP), uridine diphosphate (UDP), uridine triphosphate (UTP), cytidine monophosphate (CMP), cytidine diphosphate (CDP), cytidine triphosphate (CTP), cyclic adenosine monophosphate (cAMP), cyclic guanosine monophosphate (cGMP), deoxyadenosine monophosphate (dAMP), deoxyadenosine diphosphate (dADP), deoxyadenosine triphosphate (dATP), deoxyguanosine monophosphate (dGMP), deoxyguanosine diphosphate (dGDP), deoxyguanosine triphosphate (dGTP), deoxythymidine monophosphate (dTMP), deoxythymidine diphosphate (dTDP), deoxythymidine triphosphate (dTTP), deoxyuridine monophosphate (dUMP), deoxyuridine diphosphate (dUDP), deoxyuridine triphosphate (dUTP), deoxycytidine monophosphate (dCMP), deoxycytidine diphosphate (dCDP) and deoxycytidine triphosphate (dCTP). The free nucleotides are usually selected from AMP, TMP, GMP, CMP, UMP, dAMP, dTMP, dGMP or dCMP. The free nucleotides are typically adenosine triphosphate (ATP).

A cofactor for the motor protein is a factor that allows the motor protein to function. The cofactor is preferably a divalent metal cation. The divalent metal cation is preferably Mg²⁺, Mn²⁺, Ca²⁺ or Co²⁺. The cofactor is most preferably Mg²⁺.

As explained herein, in some embodiments the polynucleotide-handling protein is modified in order to extend the distance between the polynucleotide-handling protein and the nanopore when the polynucleotide-handling protein is used to control the movement of the conjugate with respect to the nanopore.

The polynucleotide-handling protein may be modified in any suitable way. Modification of proteins such as polynucleotide-handling proteins is within the knowledge of one of skill in the art.

A polynucleotide-handling protein may be modified by introducing additional amino acids into the protein structure. In some embodiments a polynucleotide-handling protein is modified by introducing one or more loop regions which extend beyond the natural extent of the protein. The one or more loop regions can be introduced into one or more subunits of the polynucleotide-handling protein, in embodiments wherein the polynucleotide-handling protein comprises multiple subunits.

A polynucleotide-handling protein may be modified by fusion of one or more additional domains, to displace the nanopore when the polynucleotide-handling protein is in a "seating position" relative to the nanopore.

### Nanopore

As explained above, the methods disclosed herein comprise using a polynucleotide-handling protein to control the movement of the conjugate with respect to a nanopore.

In the disclosed methods, any suitable nanopore can be used. In one embodiment a nanopore is a transmembrane pore.

A transmembrane pore is a structure that crosses the membrane to some degree. It permits hydrated ions driven by an applied potential to flow across or within the membrane. The transmembrane pore typically crosses the entire membrane so that hydrated ions may flow from one side of the membrane to the other side of the membrane. However, the transmembrane pore does not have to cross the membrane. It may be closed at one end. For instance, the pore may be a well, gap, channel, trench or slit in the membrane along which or into which hydrated ions may flow.

Any transmembrane pore may be used in the methods provided herein. The pore may be biological or artificial. Suitable pores include, but are not limited to, protein pores, polynucleotide pores, and solid state pores. A solid state pore may, in one embodiment, comprise a nanochannel. The pore may be a DNA origami pore (Langecker et al., Science, 2012; 338: 932-936). Suitable DNA origami pores are disclosed in WO2013/083983.

In one embodiment, the nanopore is a transmembrane protein pore. A transmembrane protein pore is a polypeptide or a collection of polypeptides that permits hydrated ions, such as polynucleotide, to flow from one side of a membrane to the other side of the membrane. In the methods provided herein, the transmembrane protein pore is capable of forming a pore that permits hydrated ions driven by an applied potential to flow from one side of the membrane to the other. The transmembrane protein pore preferably permits polynucleotides to flow from one side of the membrane, such as a triblock copolymer membrane, to the other. The transmembrane protein pore allows a polynucleotide to be moved through the pore.

In one embodiment, the nanopore is a transmembrane protein pore which is a monomer or an oligomer. The pore is preferably made up of several repeating subunits, such as at least 6, at least 7, at least 8, at least 9, at least 10, at least 11, at least 12, at least 13, at least 14, at least 15, or at least 16 subunits. The pore is preferably a hexameric, heptameric, octameric or nonameric pore. The pore may be a homo-oligomer or a hetero-oligomer.

In one embodiment, the transmembrane protein pore comprises a barrel or channel through which the ions may flow. The subunits of the pore typically surround a central axis and contribute strands to a transmembrane β-barrel or channel or a transmembrane α-helix bundle or channel.

Typically, the barrel or channel of the transmembrane protein pore comprises amino acids that facilitate interaction with an analyte, such as a target polynucleotide (as described herein). These amino acids are preferably located near a constriction of the barrel or channel. The transmembrane protein pore typically comprises one or more positively charged amino acids, such as arginine, lysine or histidine, or aromatic amino acids, such as tyrosine or tryptophan. These amino acids typically facilitate the interaction between the pore and nucleotides, polynucleotides or nucleic acids.

In one embodiment, the nanopore is a transmembrane protein pore derived from β-barrel pores or α-helix bundle pores. β-barrel pores comprise a barrel or channel that is formed from β-strands. Suitable β-barrel pores include, but are not limited to, β-toxins, such as α-hemolysin, anthrax toxin and leukocidins, and outer membrane proteins/porins of bacteria, such as *Mycobacterium smegmatis* porin (Msp), for example MspA, MspB, MspC or MspD, CsgG, outer membrane porin F (OmpF), outer membrane porin G (OmpG), outer membrane phospholipase A and *Neisseria* autotransporter lipoprotein (NalP) and other pores, such as lysenin. α-helix bundle pores comprise a barrel or channel that is formed from α-helices. Suitable α-helix bundle pores include, but are not limited to, inner membrane proteins and α outer membrane proteins, such as WZA and ClyA toxin.

In one embodiment the nanopore is a transmembrane pore derived from or based on Msp, α-hemolysin (α-HL), lysenin, CsgG, ClyA, Sp1 or haemolytic protein fragaceatoxin C (FraC).

In one embodiment, the nanopore is a transmembrane protein pore derived from CsgG, e.g. from CsgG from *E. coli* Str. K-12 substr. MC4100. Such a pore is oligomeric and typically comprises 7, 8, 9 or 10 monomers derived from CsgG. The pore may be a homo-oligomeric pore derived from CsgG comprising identical monomers. Alternatively, the pore may be a hetero-oligomeric pore derived from CsgG comprising at least one monomer that differs from the others. Examples of suitable pores derived from CsgG are disclosed in WO 2016/034591.

In one embodiment, the nanopore is a transmembrane pore derived from lysenin. Examples of suitable pores derived from lysenin are disclosed in WO 2013/153359.

In one embodiment, the nanopore is a transmembrane pore derived from or based on α-hemolysin (α-HL). The wild type α-hemolysin pore is formed of 7 identical monomers or sub-units (i.e., it is heptameric). An α-hemolysin pore may be α-hemolysin-NN or a variant thereof. The variant preferably comprises N residues at positions E111 and K147.

In one embodiment, the nanopore is a transmembrane protein pore derived from Msp, e.g. from MspA. Examples of suitable pores derived from MspA are disclosed in WO 2012/107778.

In one embodiment, the nanopore is a transmembrane pore derived from or based on ClyA.

As explained above, in some embodiments the nanopore comprises a constriction. The constriction is typically a narrowing in the channel which runs through the nanopore which may determine or control the signal obtained when the conjugate moves with respect to the nanopore. As used herein, both protein and solid state nanopores typically comprise a "constriction".

In some embodiments, the nanopore is modified to extend the distance between the polynucleotide-handling protein and a constriction region of the nanopore. In some embodiments the nanopore is modified to extend the distance between the polynucleotide-handling protein and a constriction region of the nanopore when the polynucleotide-handling protein is being used to control the movement of the conjugate with respect to the nanopore. In some embodiments the nanopore is modified to extend the distance between the polynucleotide-handling protein and a constriction region of the nanopore when the polynucleotide-handling protein is in contact with the nanopore.

In some embodiments the nanopore is modified to extend the distance between the active site of the polynucleotide-handling protein and the constriction region of the nanopore. In such embodiments the distance may be the distance between the active site of the polynucleotide-handling protein and the constriction of the nanopore when the polynucleotide-handling protein is being used to control the movement of the conjugate with respect to the nanopore and/or when the polynucleotide-handling protein is in contact with the nanopore.

The nanopore may be modified in any suitable way. Modification of nanopores such as protein nanopores is within the knowledge of one of skill in the art. Modification of solid-state nanopores is routine and can be achieved by controlling the substrate in which the nanopore is formed (e.g. its thickness) or the components from which the nanopore is formed.

For example, the nanopore may be modified to extend the length of the channel running through the pore.

A protein nanopore may be modified by introducing additional amino acids into the pore structure. In some embodiments a protein nanopore is modified by introducing one or more loop regions which extend beyond the natural extent of the nanopore. The one or more loop regions can be introduced into one or more subunits of the nanopore, in embodiments wherein the nanopore comprises multiple subunits. The loop regions may for example extend beyond the *cis* entrance of the nanopore.

A protein nanopore may be modified to extend the length of the barrel or channel running through the pore. For example, a beta-barrel pore can be modified by introducing additional amino acids into the protein sequence in the portion which forms the barrel thereby extending the length of the barrel. Rational design of relevant positions for such modifications can be made e.g. by reference to the structure (e.g. the X-ray) structure of the protein and/or monomer subunits thereof.

A protein nanopore may be modified by fusion of one or more additional domains to raise the "seating position" of the polynucleotide-handling protein relative to the nanopore.

In some embodiments it is possible to modify a protein nanopore by fusing it to another protein nanopore. In this way a chain of nanopores can be made with a single channel running therethrough, to extend the distance between a constriction in the channel and a polynucleotide-handling protein. In such cases the multiple nanopores can be the same or different.

### Tags

In some embodiments of the methods provided herein, a tag on the nanopore can be used, e.g. to promote the capture of the conjugate by the nanopore.

The interaction between a tag on a nanopore and the binding site on a polynucleotide (*e.g*., the binding site present in the polynucleotide portion of the conjugate, or in an adaptor attached to the conjugate, wherein the binding site can be provided by an anchor or a leader sequence of an adaptor or by a capture sequence within the duplex stem of an adaptor) may be reversible. For example, a polynucleotide can bind to a tag on a nanopore, *e.g.,* via its adaptor, and release at some point, *e.g.,* during characterization of the polynucleotide by the nanopore and/or during processing by a motor protein. A strong non-covalent bond (*e.g*., biotin/avidin) is still reversible and can be useful in some embodiments of the methods described herein. For example, a pair of pore tag and polynucleotide adaptor can be designed to provide a sufficient interaction between the complement of a double stranded polynucleotide (or a portion of an adaptor that is attached to the complement) and the nanopore such that the complement is held close to the nanopore (without detaching from the nanopore and diffusing away) but is able to release from the nanopore as it is processed.

A pore tag and polynucleotide adaptor can be configured such that the binding strength or affinity of a binding site on the polynucleotide (*e.g*., a binding site provided by an anchor or a leader sequence of an adaptor or by a capture sequence within the duplex stem of an adaptor) to a tag on a nanopore is sufficient to maintain the coupling between the nanopore and polynucleotide until an applied force is placed on it to release the bound polynucleotide from the nanopore.

In some embodiments, the tags or tethers are uncharged. This can ensure that the tags or tethers are not drawn into the nanopore under the influence of a potential difference if present.

One or more molecules that attract or bind the conjugate, polynucleotide or adaptor may be linked to the nanopore. Any molecule that hybridizes to the conjugate, adaptor and/or polynucleotide may be used. The molecule attached to the pore may be selected from a PNA tag, a PEG linker, a short oligonucleotide, a positively charged amino acid and an aptamer. Pores having such molecules linked to them are known in the art. For example, pores having short oligonucleotides attached thereto are disclosed in Howarka et al (2001) Nature Biotech. 19: 636-639 and WO 2010/086620, and pores comprising PEG attached within the lumen of the pore are disclosed in Howarka et al (2000) J. Am. Chem. Soc. 122(11): 2411-2416.

A short oligonucleotide attached to the nanopore, which comprises a sequence complementary to a sequence in the conjugate (e.g. in a leader sequence or another single stranded sequence in an adaptor) may be used to enhance capture of the conjugate in the methods described herein.

In some embodiments, the tag or tether may comprise or be an oligonucleotide *(e.g.,* DNA, RNA, LNA, BNA, PNA, or morpholino). The oligonucleotide can have about 10-30 nucleotides in length or about 10-20 nucleotides in length. In some embodiments, the oligonucleotide can have at least one end (*e.g.,* 3'- or 5'-end) modified for conjugation to other modifications or to a solid substrate surface including, *e.g.,* a bead. The end modifiers may add a reactive functional group which can be used for conjugation. Examples of functional groups that can be added include, but are not limited to amino, carboxyl, thiol, maleimide, aminooxy, and any combinations thereof. The functional groups can be combined with different length of spacers (*e.g.,* C3, C9, C12, Spacer 9 and 18) to add physical distance of the functional group from the end of the oligonucleotide sequence.

Examples of modifications on the 3' and/or 5' end of oligonucleotides include, but are not limited to 3' affinity tag and functional groups for chemical linkage (including, *e.g*., 3'-biotin, 3'-primary amine, 3'-disulfide amide, 3'-pyridyl dithio, and any combinations thereof); 5' end modifications (including, *e.g*., 5'-primary ammine, and/or 5'-dabcyl), modifications for click chemistry (including, *e.g*., 3'-azide, 3'-alkyne, 5'-azide, 5'-alkyne), and any combinations thereof.

In some embodiments, the tag or tether may further comprise a polymeric linker, *e.g.,* to facilitate coupling to the nanopore. An exemplary polymeric linker includes, but is not limited to polyethylene glycol (PEG). The polymeric linker may have a molecular weight of about 500 Da to about 10 kDa (inclusive), or about 1 kDa to about 5 kDa (inclusive). The polymeric linker (*e.g.,* PEG) can be functionalized with different functional groups including, *e.g*., but not limited to maleimide, NHS ester, dibenzocyclooctyne (DBCO), azide, biotin, amine, alkyne, aldehyde, and any combinations thereof.

Other examples of a tag or tether include, but are not limited to His tags, biotin or streptavidin, antibodies that bind to analytes, aptamers that bind to analytes, analyte binding domains such as DNA binding domains (including, i., peptide zippers such as leucine zippers, single-stranded DNA binding proteins (SSB)), and any combinations thereof.

The tag or tether may be attached to the external surface of a nanopore, *e.g.,* on the *cis* side of a membrane, using any methods known in the art. For example, one or more tags or tethers can be attached to the nanopore via one or more cysteines (cysteine linkage), one or more primary amines such as lysines, one or more non-natural amino acids, one or more histidines (His tags), one or more biotin or streptavidin, one or more antibody-based tags, one or more enzyme modification of an epitope (including, *e.g*., acetyl transferase), and any combinations thereof. Suitable methods for carrying out such modifications are well-known in the art. Suitable non-natural amino acids include, but are not limited to, 4-azido-L-phenylalanine (Faz) and any one of the amino acids numbered 1-71 in Figure 1 of Liu C. C. and Schultz P. G., Annu. Rev. Biochem., 2010, 79, 413-444.

In some embodiments where one or more tags or tethers are attached to a nanopore via cysteine linkage(s), the one or more cysteines can be introduced to one or more monomers that form the nanopore by substitution. In some embodiments, the nanopore may be chemically modified by attachment of (i) Maleimides including diabromomaleimides such as: 4-phenylazomaleinanil, 1.N-(2-Hydroxyethyl)maleimide, N-Cyclohexylmaleimide, 1.3-Maleimidopropionic Acid, 1.1-4-Aminophenyl-1H-pyrrole,2,5,dione, 1.1-4-Hydroxyphenyl-1H-pyrrole,2,5,dione, N-Ethylmaleimide, N-Methoxycarbonylmaleimide, N-tert-Butylmaleimide, N-(2-Aminoethyl)maleimide , 3-Maleimido-PROXYL , N-(4-Chlorophenyl)maleimide, 1-[4-(dimethylamino)-3,5-dinitrophenyl]-1H-pyrrole-2,5-dione, N-[4-(2-Benzimidazolyl)phenyl]maleimide, N-[4-(2-benzoxazolyl)phenyl]maleimide, N-(1-naphthyl)-maleimide, N-(2,4-xylyl)maleimide, N-(2,4-difluorophenyl)maleimide , N-(3-chloro-para-tolyl)-maleimide, 1-(2-amino-ethyl)-pyrrole-2,5-dione hydrochloride, 1-cyclopentyl-3-methyl-2,5-dihydro-1H-pyrrole-2,5-dione, 1-(3-aminopropyl)-2,5-dihydro-1H-pyrrole-2,5-dione hydrochloride, 3-methyl-1-[2-oxo-2-(piperazin-1-yl)ethyl]-2,5-dihydro-1H-pyrrole-2,5-dione hydrochloride, 1-benzyl-2,5-dihydro-1H-pyrrole-2,5-dione, 3-methyl-1-(3,3,3-trifluropropyl)-2,5-dihydro-1H-pyrrole-2,5-dione, 1-[4-(methylamino)cyclohexyl]-2,5-dihydro-1H-pyrrole-2,5-dione trifluroacetic acid, SMILES O=C1C=CC(=O)N1CC=2C=CN=CC2, SMILES O=C1C=CC(=O)N1CN2CCNCC2, 1-benzyl-3-methyl-2,5-dihydro-1H-pyrrole-2,5-dione, 1-(2-fluorophenyl)-3-methyl-2,5-dihydro 1H-pyrrole-2,5-dione, N-(4-phenoxyphenyl)maleimide , N-(4-nitrophenyl)maleimide (ii) Iodocetamides such as :3-(2-Iodoacetamido)-proxyl, N-(cyclopropylmethyl)-2-iodoacetamide, 2-iodo-N-(2-phenylethyl)acetamide, 2-iodo-N-(2,2,2-trifluoroethyl)acetamide, N-(4-acetylphenyl)-2-iodoacetamide, N-(4-(aminosulfonyl)phenyl)-2-iodoacetamide, N-(1,3-benzothiazol-2-yl)-2-iodoacetamide, N-(2,6-diethylphenyl)-2-iodoacetamide, N-(2-benzoyl-4-chlorophenyl)-2-iodoacetamide, (iii) Bromoacetamides: such as N-(4-(acetylamino)phenyl)-2-bromoacetamide , N-(2-acetylphenyl)-2-bromoacetamide , 2-bromo-n-(2-cyanophenyl)acetamide, 2-bromo-N-(3-(trifluoromethyl)phenyl)acetamide, N-(2-benzoylphenyl)-2-bromoacetamide , 2-bromo-N-(4-fluorophenyl)-3-methylbutanamide, N-Benzyl-2-bromo-N-phenylpropionamide, N-(2-bromo-butyryl)-4-chloro-benzenesulfonamide, 2-Bromo-N-methyl-N-phenylacetamide, 2-bromo-N-phenethyl-acetamide,2-adamantan-1-yl-2-bromo-N-cyclohexyl-acetamide, 2-bromo-N-(2-methylphenyl)butanamide, Monobromoacetanilide, (iv) Disulphides such as: aldrithiol-2 , aldrithiol-4 , isopropyl disulfide, 1-(Isobutyldisulfanyl)-2-methylpropane, Dibenzyl disulfide, 4-aminophenyl disulfide, 3-(2-Pyridyldithio)propionic acid, 3-(2-Pyridyldithio)propionic acid hydrazide, 3-(2-Pyridyldithio)propionic acid N-succinimidyl ester, am6amPDP1-βCD and (v) Thiols such as: 4-Phenylthiazole-2-thiol, Purpald, 5,6,7,8-tetrahydro-quinazoline-2-thiol.

In some embodiments, the tag or tether may be attached directly to a nanopore or via one or more linkers. The tag or tether may be attached to the nanopore using the hybridization linkers described in WO 2010/086602. Alternatively, peptide linkers may be used. Peptide linkers are amino acid sequences. The length, flexibility and hydrophilicity of the peptide linker are typically designed such that it does not to disturb the functions of the monomer and pore. Preferred flexible peptide linkers are stretches of 2 to 20, such as 4, 6, 8, 10 or 16, serine and/or glycine amino acids. More preferred flexible linkers include (SG)₁, (SG)₂, (SG)₃, (SG)₄, (SG)₅ and (SG)₈ wherein S is serine and G is glycine. Preferred rigid linkers are stretches of 2 to 30, such as 4, 6, 8, 16 or 24, proline amino acids. More preferred rigid linkers include (P)₁₂ wherein P is proline.

### Membrane

Typically, in the disclosed methods, the nanopore is typically present in a membrane. Any suitable membrane may be used in the system.

The membrane is preferably an amphiphilic layer. An amphiphilic layer is a layer formed from amphiphilic molecules, such as phospholipids, which have both hydrophilic and lipophilic properties. The amphiphilic molecules may be synthetic or naturally occurring. Non-naturally occurring amphiphiles and amphiphiles which form a monolayer are known in the art and include, for example, block copolymers (Gonzalez-Perez et al., Langmuir, 2009, 25, 10447-10450). Block copolymers are polymeric materials in which two or more monomer sub-units that are polymerized together to create a single polymer chain. Block copolymers typically have properties that are contributed by each monomer sub-unit. However, a block copolymer may have unique properties that polymers formed from the individual sub-units do not possess. Block copolymers can be engineered such that one of the monomer sub-units is hydrophobic (i.e. lipophilic), whilst the other sub-unit(s) are hydrophilic whilst in aqueous media. In this case, the block copolymer may possess amphiphilic properties and may form a structure that mimics a biological membrane. The block copolymer may be a diblock (consisting of two monomer sub-units), but may also be constructed from more than two monomer sub-units to form more complex arrangements that behave as amphipiles. The copolymer may be a triblock, tetrablock or pentablock copolymer. The membrane is preferably a triblock copolymer membrane.

Archaebacterial bipolar tetraether lipids are naturally occurring lipids that are constructed such that the lipid forms a monolayer membrane. These lipids are generally found in extremophiles that survive in harsh biological environments, thermophiles, halophiles and acidophiles. Their stability is believed to derive from the fused nature of the final bilayer. It is straightforward to construct block copolymer materials that mimic these biological entities by creating a triblock polymer that has the general motif hydrophilic-hydrophobic-hydrophilic. This material may form monomeric membranes that behave similarly to lipid bilayers and encompass a range of phase behaviours from vesicles through to laminar membranes. Membranes formed from these triblock copolymers hold several advantages over biological lipid membranes. Because the triblock copolymer is synthesised, the exact construction can be carefully controlled to provide the correct chain lengths and properties required to form membranes and to interact with pores and other proteins.

Block copolymers may also be constructed from sub-units that are not classed as lipid sub-materials; for example a hydrophobic polymer may be made from siloxane or other non-hydrocarbon based monomers. The hydrophilic sub-section of block copolymer can also possess low protein binding properties, which allows the creation of a membrane that is highly resistant when exposed to raw biological samples. This head group unit may also be derived from non-classical lipid head-groups.

Triblock copolymer membranes also have increased mechanical and environmental stability compared with biological lipid membranes, for example a much higher operational temperature or pH range. The synthetic nature of the block copolymers provides a platform to customise polymer based membranes for a wide range of applications.

In some embodiments, the membrane is one of the membranes disclosed in International Application No. WO2014/064443 or WO2014/064444.

The amphiphilic molecules may be chemically-modified or functionalised to facilitate coupling of the polynucleotide. The amphiphilic layer may be a monolayer or a bilayer. The amphiphilic layer is typically planar. The amphiphilic layer may be curved. The amphiphilic layer may be supported.

Amphiphilic membranes are typically naturally mobile, essentially acting as two dimensional fluids with lipid diffusion rates of approximately 10⁻⁸ cm s⁻¹. This means that the pore and coupled polynucleotide can typically move within an amphiphilic membrane.

The membrane may be a lipid bilayer. Lipid bilayers are models of cell membranes and serve as excellent platforms for a range of experimental studies. For example, lipid bilayers can be used for *in vitro* investigation of membrane proteins by single-channel recording. Alternatively, lipid bilayers can be used as biosensors to detect the presence of a range of substances. The lipid bilayer may be any lipid bilayer. Suitable lipid bilayers include, but are not limited to, a planar lipid bilayer, a supported bilayer or a liposome. The lipid bilayer is preferably a planar lipid bilayer. Suitable lipid bilayers are disclosed in WO 2008/102121, WO 2009/077734 and WO 2006/100484.

Methods for forming lipid bilayers are known in the art. Lipid bilayers are commonly formed by the method of Montal and Mueller (Proc. Natl. Acad. Sci. USA., 1972; 69: 3561-3566), in which a lipid monolayer is carried on aqueous solution/air interface past either side of an aperture which is perpendicular to that interface. The lipid is normally added to the surface of an aqueous electrolyte solution by first dissolving it in an organic solvent and then allowing a drop of the solvent to evaporate on the surface of the aqueous solution on either side of the aperture. Once the organic solvent has evaporated, the solution/air interfaces on either side of the aperture are physically moved up and down past the aperture until a bilayer is formed. Planar lipid bilayers may be formed across an aperture in a membrane or across an opening into a recess.

The method of Montal & Mueller is popular because it is a cost-effective and relatively straightforward method of forming good quality lipid bilayers that are suitable for protein pore insertion. Other common methods of bilayer formation include tip-dipping, painting bilayers and patch-clamping of liposome bilayers.

Tip-dipping bilayer formation entails touching the aperture surface (for example, a pipette tip) onto the surface of a test solution that is carrying a monolayer of lipid. Again, the lipid monolayer is first generated at the solution/air interface by allowing a drop of lipid dissolved in organic solvent to evaporate at the solution surface. The bilayer is then formed by the Langmuir-Schaefer process and requires mechanical automation to move the aperture relative to the solution surface.

For painted bilayers, a drop of lipid dissolved in organic solvent is applied directly to the aperture, which is submerged in an aqueous test solution. The lipid solution is spread thinly over the aperture using a paintbrush or an equivalent. Thinning of the solvent results in formation of a lipid bilayer. However, complete removal of the solvent from the bilayer is difficult and consequently the bilayer formed by this method is less stable and more prone to noise during electrochemical measurement.

Patch-clamping is commonly used in the study of biological cell membranes. The cell membrane is clamped to the end of a pipette by suction and a patch of the membrane becomes attached over the aperture. The method has been adapted for producing lipid bilayers by clamping liposomes which then burst to leave a lipid bilayer sealing over the aperture of the pipette. The method requires stable, giant and unilamellar liposomes and the fabrication of small apertures in materials having a glass surface.

Liposomes can be formed by sonication, extrusion or the Mozafari method (Colas et al. (2007) Micron 38:841-847).

In some embodiments, a lipid bilayer is formed as described in International Application No. WO 2009/077734. Advantageously in this method, the lipid bilayer is formed from dried lipids. In a most preferred embodiment, the lipid bilayer is formed across an opening as described in WO2009/077734.

A lipid bilayer is formed from two opposing layers of lipids. The two layers of lipids are arranged such that their hydrophobic tail groups face towards each other to form a hydrophobic interior. The hydrophilic head groups of the lipids face outwards towards the aqueous environment on each side of the bilayer. The bilayer may be present in a number of lipid phases including, but not limited to, the liquid disordered phase (fluid lamellar), liquid ordered phase, solid ordered phase (lamellar gel phase, interdigitated gel phase) and planar bilayer crystals (lamellar sub-gel phase, lamellar crystalline phase).

Any lipid composition that forms a lipid bilayer may be used. The lipid composition is chosen such that a lipid bilayer having the required properties, such surface charge, ability to support membrane proteins, packing density or mechanical properties, is formed. The lipid composition can comprise one or more different lipids. For instance, the lipid composition can contain up to 100 lipids. The lipid composition preferably contains 1 to 10 lipids. The lipid composition may comprise naturally-occurring lipids and/or artificial lipids.

The lipids typically comprise a head group, an interfacial moiety and two hydrophobic tail groups which may be the same or different. Suitable head groups include, but are not limited to, neutral head groups, such as diacylglycerides (DG) and ceramides (CM); zwitterionic head groups, such as phosphatidylcholine (PC), phosphatidylethanolamine (PE) and sphingomyelin (SM); negatively charged head groups, such as phosphatidylglycerol (PG); phosphatidylserine (PS), phosphatidylinositol (PI), phosphatic acid (PA) and cardiolipin (CA); and positively charged headgroups, such as trimethylammonium-Propane (TAP). Suitable interfacial moieties include, but are not limited to, naturally-occurring interfacial moieties, such as glycerol-based or ceramide-based moieties. Suitable hydrophobic tail groups include, but are not limited to, saturated hydrocarbon chains, such as lauric acid (*n*-Dodecanolic acid), myristic acid (*n-*Tetradecononic acid), palmitic acid (*n*-Hexadecanoic acid), stearic acid (*n*-Octadecanoic) and arachidic (*n*-Eicosanoic); unsaturated hydrocarbon chains, such as oleic acid (*cis*-9-Octadecanoic); and branched hydrocarbon chains, such as phytanoyl. The length of the chain and the position and number of the double bonds in the unsaturated hydrocarbon chains can vary. The length of the chains and the position and number of the branches, such as methyl groups, in the branched hydrocarbon chains can vary. The hydrophobic tail groups can be linked to the interfacial moiety as an ether or an ester. The lipids may be mycolic acid.

The lipids can also be chemically-modified. The head group or the tail group of the lipids may be chemically-modified. Suitable lipids whose head groups have been chemically-modified include, but are not limited to, PEG-modified lipids, such as 1,2-Diacyl-sn-Glycero-3-Phosphoethanolamine-N -[Methoxy(Polyethylene glycol)-2000]; functionalised PEG Lipids, such as 1,2-Distearoyl-sn-Glycero-3 Phosphoethanolamine-N-[Biotinyl(Polyethylene Glycol)2000]; and lipids modified for conjugation, such as 1,2-Dioleoyl-sn-Glycero-3-Phosphoethanolamine-N-(succinyl) and 1,2-Dipalmitoyl-sn-Glycero-3-Phosphoethanolamine-N-(Biotinyl). Suitable lipids whose tail groups have been chemically-modified include, but are not limited to, polymerisable lipids, such as 1,2-bis(10,12-tricosadiynoyl)-sn-Glycero-3-Phosphocholine; fluorinated lipids, such as 1-Palmitoyl-2-(16-Fluoropalmitoyl)-sn-Glycero-3-Phosphocholine; deuterated lipids, such as 1,2-Dipalmitoyl-D62-sn-Glycero-3-Phosphocholine; and ether linked lipids, such as 1,2-Di-O-phytanyl-sn-Glycero-3-Phosphocholine. The lipids may be chemically-modified or functionalised to facilitate coupling of the polynucleotide.

The amphiphilic layer, for example the lipid composition, typically comprises one or more additives that will affect the properties of the layer. Suitable additives include, but are not limited to, fatty acids, such as palmitic acid, myristic acid and oleic acid; fatty alcohols, such as palmitic alcohol, myristic alcohol and oleic alcohol; sterols, such as cholesterol, ergosterol, lanosterol, sitosterol and stigmasterol; lysophospholipids, such as 1-Acyl-2-Hydroxy-sn- Glycero-3-Phosphocholine; and ceramides.

In another embodiment, the membrane comprises a solid state layer. Solid state layers can be formed from both organic and inorganic materials including, but not limited to, microelectronic materials, insulating materials such as Si₃N₄, Al₂O₃, and SiO, organic and inorganic polymers such as polyamide, plastics such as Teflon^{®} or elastomers such as two-component addition-cure silicone rubber, and glasses. The solid state layer may be formed from graphene. Suitable graphene layers are disclosed in WO 2009/035647. If the membrane comprises a solid state layer, the pore is typically present in an amphiphilic membrane or layer contained within the solid state layer, for instance within a hole, well, gap, channel, trench or slit within the solid state layer. The skilled person can prepare suitable solid state/amphiphilic hybrid systems. Suitable systems are disclosed in WO 2009/020682 and WO 2012/005857. Any of the amphiphilic membranes or layers discussed above may be used.

The methods disclosed herein are typically carried out using (i) an artificial amphiphilic layer comprising a pore, (ii) an isolated, naturally-occurring lipid bilayer comprising a pore, or (iii) a cell having a pore inserted therein. The methods are typically carried out using an artificial amphiphilic layer, such as an artificial triblock copolymer layer. The layer may comprise other transmembrane and/or intramembrane proteins as well as other molecules in addition to the pore. Suitable apparatus and conditions are discussed below. The disclosed methods are typically carried out *in vitro.*

### Conditions

As explained above, the disclosed methods comprise characterising a polypeptide as the conjugate within which the polypeptide is comprised moves with respect to a nanopore.

The characterisation methods may be carried out using any apparatus that is suitable for investigating a membrane/pore system in which a pore is inserted into a membrane. The characterisation method may be carried out using any apparatus that is suitable for transmembrane pore sensing. For example, the apparatus may comprise a chamber comprising an aqueous solution and a barrier that separates the chamber into two sections. The barrier may have an aperture in which a membrane containing a transmembrane pore is formed. Transmembrane pores are described herein.

The characterisation methods may be carried out using the apparatus described in WO 2008/102120, WO 2010/122293 or WO 00/28312.

The characterisation methods may involve measuring the ion current flow through the pore, typically by measurement of a current. Alternatively, the ion flow through the pore may be measured optically, such as disclosed by Heron et al: J. Am. Chem. Soc. 9 Vol. 131, No. 5, 2009. Therefore the apparatus may also comprise an electrical circuit capable of applying a potential and measuring an electrical signal across the membrane and pore. The characterisation methods may be carried out using a patch clamp or a voltage clamp. The characterisation methods preferably involve the use of a voltage clamp. The characterisation methods may be carried out on a silicon-based array of wells where each array comprises 128, 256, 512, 1024, 2000, 3000, 4000, 6000, 10000, 12000, 15000 or more wells.

The characterisation methods may involve the measuring of a current flowing through the pore. The method is typically carried out with a voltage applied across the membrane and pore. The voltage used is typically from +2 V to -2 V, typically -400 mV to +400mV. The voltage used is preferably in a range having a lower limit selected from -400 mV, -300 mV, -200 mV, -150 mV, -100 mV, -50 mV, -20mV and 0 mV and an upper limit independently selected from +10 mV, + 20 mV, +50 mV, +100 mV, +150 mV, +200 mV, +300 mV and +400 mV. The voltage used is more preferably in the range 100 mV to 240mV and most preferably in the range of 120 mV to 220 mV. It is possible to increase discrimination between different nucleotides by a pore by using an increased applied potential.

The characterisation methods are typically carried out in the presence of any charge carriers, such as metal salts, for example alkali metal salts, halide salts, for example chloride salts, such as alkali metal chloride salt. Charge carriers may include ionic liquids or organic salts, for example tetramethyl ammonium chloride, trimethylphenyl ammonium chloride, phenyltrimethyl ammonium chloride, or 1-ethyl-3-methyl imidazolium chloride. In the exemplary apparatus discussed above, the salt is present in the aqueous solution in the chamber. Potassium chloride (KCl), sodium chloride (NaCl) or caesium chloride (CsCl) is typically used. KCl is preferred. The salt may be an alkaline earth metal salt such as calcium chloride (CaCl₂). The salt concentration may be at saturation. The salt concentration may be 3M or lower and is typically from 0.1 to 2.5 M, from 0.3 to 1.9 M, from 0.5 to 1.8 M, from 0.7 to 1.7 M, from 0.9 to 1.6 M or from 1 M to 1.4 M. The salt concentration is preferably from 150 mM to 1 M. The characterisation method is preferably carried out using a salt concentration of at least 0.3 M, such as at least 0.4 M, at least 0.5 M, at least 0.6 M, at least 0.8 M, at least 1.0 M, at least 1.5 M, at least 2.0 M, at least 2.5 M or at least 3.0 M. High salt concentrations provide a high signal to noise ratio and allow for currents indicative of binding/no binding to be identified against the background of normal current fluctuations.

The characterisation methods are typically carried out in the presence of a buffer. In the exemplary apparatus discussed above, the buffer is present in the aqueous solution in the chamber. Any suitable buffer may be used. Typically, the buffer is HEPES. Another suitable buffer is Tris-HCl buffer. The methods are typically carried out at a pH of from 4.0 to 12.0, from 4.5 to 10.0, from 5.0 to 9.0, from 5.5 to 8.8, from 6.0 to 8.7 or from 7.0 to 8.8 or 7.5 to 8.5. The pH used is preferably about 7.5.

The characterisation methods may be carried out at from 0 °C to 100 °C, from 15 °C to 95 °C, from 16 °C to 90 °C, from 17 °C to 85 °C, from 18 °C to 80 °C, 19 °C to 70 °C, or from 20 °C to 60 °C. The characterisation methods are typically carried out at room temperature. The characterisation methods are optionally carried out at a temperature that supports enzyme function, such as about 37 °C.

### Kit

Also provided is a kit comprising:
- a nanopore comprising a constriction region, wherein the nanopore is a transmembrane protein pore;
- a polynucleotide comprising a reactive functional group for conjugating to a target polypeptide; and
- a polynucleotide-handling protein capable of controlling the movement of the polynucleotide with respect to the nanopore;
   wherein the polynucleotide-handling protein is modified by covalently closing a polynucleotide-unbinding opening through which a polynucleotide strand may unbind, thereby preventing the polynucleotide binding protein to prevent it from disengaging from the conjugate when the polynucleotide-handling protein contacts a portion of the conjugate comprising a polypeptide.

In some embodiments, said nanopore is modified to increase the distance between the constriction region and the polynucleotide-handling protein when the polynucleotide-handling enzyme is in contact with the nanopore.

In some embodiments, said kit further comprises one or more displacer units for extending the distance between the nanopore and the active site of the polynucleotide-handling protein.

In some embodiments the nanopore, polynucleotide and/or polynucleotide-handling protein, and optionally the one or more displacer units if present are as described herein.

The kit may be configured for use with an algorithm, also provided herein, adapted to be run on a computer system. The algorithm may be adapted to detect information characteristic of a polypeptide (e.g. characteristic of the sequence of the polypeptide and/or whether the polypeptide is modified), and to selectively process the signal obtained as a conjugate comprising the polypeptide conjugated to a polynucleotide moves with respect to the nanopore.
The preceding embodiments and following examples are provided for illustration only, and should not be considered limiting the application. The application is limited only by the claims.

### EXAMPLES

### Example 1

This example demonstrates controlled translocation of a conjugate comprising a polypeptide flanked by two pieces of polynucleotide; a dsDNA Y adapter (DNA1) and a dsDNA tail (DNA2). A polynucleotide-handling protein at the *cis* side of the nanopore controls the movement of the conjugate by first unwinding DNA1 and translocating 5'-3' on ssDNA, then sliding across the polypeptide section to finally unwind the DNA2 segment. As this construct moves from the *cis* to the *trans* side of the nanopore, passing through the RED, the polypeptide section can be visualized on a current vs time plot enabling characterization.

A Y-adapter was prepared by annealing DNA oligonucleotides (SEQ ID NO:11, SEQ ID NO: 12, SEQ ID NO:13). A DNA motor (Dda helicase) was loaded and closed on the adapter as described in WO 2014/013260. The subsequent material was HPLC purified. The Y adapter contains 30 C3 leader section for easier capture by the nanopore and a side arm for tethering to the membrane. The DNA tail was made by annealing DNA oligonucleotides (SEQ ID NO:14, SEQ ID NO:16).

In this example the model polypeptide analytes (SEQ ID NOs: 20, 21, 22) were obtained with azide moieties pre-synthesized at the N-terminus, and directly after the C-terminus using an ethyl diamine spacer in line with the peptide backbone. Each analyte was then conjugated to the Y-adapter and DNA tail via copper-free Click Chemistry reaction between an azide and BCN (bicyclo[6.1.0]nonyne) moieties. A schematic of the resulting construct is shown in Figure 4A. The sample was purified using Agencourt AMPure XP (Beckman Coulter) beads, with two washes with LNB from Oxford Nanopore Technologies sequencing kit (SQK-LSK109). The conjugated substrate was eluted into 10 mM Tris-Cl, 50 mM NaCl (pH 8.0).

Electrical measurements were acquired using MinION Mk1b from Oxford Nanopore Technologies and a custom MinION flow cell with MspA nanopores. Flow cells were flushed with a tether mix containing 50 nM of DNA tether and sequencing buffer lacking ATP. Initially 800 µL of tether mix was added for 5 minutes, then a further 200 µL of mix were flowed through the system with the SpotON port open. DNA-peptide constructs were prepared at 0.5nM concentration in sequencing buffer lacking ATP, and LB from Oxford Nanopore Technologies sequencing kit (SQK-LSK109), yielding "sequencing mix". 75 µL of the sequencing mix were added to a MinION flowcell via the SpotON flow cell port. The mixture was incubated on the flowcell for 5-10 minutes to allow for construct tethering and subsequent capture by the nanopores. In the absence of ATP, the DNA motor remains stalled on the spacer region of the Y-adapter, the conjugates are captured by the nanopores but there is no translocation. After the incubation, 200 µL of sequencing buffer containing ATP was added; in the presence of ATP the captured DNA-peptide conjugate is moved across the nanopore by the helicase resulting in a reproducible current footprint.

A standard sequencing script at 180mV was run for 30 minutes to 1 hour, with static flips every 1 minute to remove extended nanopore blocks. Raw data was collected in a bulk FAST5 file using MinKNOW software (Oxford Nanopore Technologies).

Exemplary current vs time traces for one of the model peptides (SEQ ID NO: 20) conjugated to the DNA Y adapter and tail can be seen in Figure 9. The Y-adapter section and dsDNA tail can be separated from the peptide portion of the "squiggle" (the trace) to enable characterization of the peptide.

High throughput was achieved with multiple translocation events observed per second. An example current vs time trace showing multiple capture and translocation events is shown in Figure 10 for the same construct as used in Figure 9 (i.e. containing the peptide region of SEQ ID NO: 20)

Characterisation of other conjugated polynucleotide-polypeptide constructs was carried out as described above. Figures 11 to 13 show reproducible current vs. time traces enabling characterisation of constructs incorporating peptide regions containing positively charged amino acids (SEQ ID NO: 21; Figure 11); aromatic amino acids (SEQ ID NO: 22, Figure 12) and negatively charged amino acids (SEQ ID NO: 20, Figure 13).

For ease of reference a schematic structure of the construct obtained using peptide of SEQ ID NO: 22 is shown in Figure 14.

### Example 2

This example demonstrates the utility of the disclosed methods in characterising polynucleotide-polypeptide constructs obtained from peptides which are not pre-synthesized to contain attachment groups.

In this example the Y adapter is the same as in Example 1 and dsDNA tail was prepared by annealing DNA oligonucleotides (SEQ ID NO: 15, SEQ ID NO: 16). The data collection was carried out on MinION Mk1b from Oxford Nanopore Technologies and a custom MinION flow cell with MspA nanopores using the protocol established in Example 1.

The peptide analyte used in this example was nearly identical to the model peptide used in Example 1 (GGSGDDSGSG, SEQ ID NO: 20 for Example 1; SEQ ID NO: 23 for Example 2) but lacked pre-synthesised azide molecules for the click chemistry conjugation of the polynucleotide adapter and tail. An additional C-terminal cysteine was included to enable maleimide chemistry. The N-terminus of the peptide was functionalized with a tetrazine-NHS ester compound (BroadPharm, product code: BP-22946). Unconjugated tetrazine was removed with amino functionalized magnetic particles (Sigma Aldrich, product code: 53572).

The peptide was then incubated with DNA tail (SEQ ID NO: 15, SEQ ID NO: 16) overnight at 4°C to facilitate the clicking reaction between tetrazine and TCO (trans-cyclooctene). Following the incubation, possible disulfide bonds between the C-terminal peptide cysteines were reduced with 5mM DTT for 30 minutes at room temperature and the peptide-DNA conjugate was purified using Agencourt AMPure XP beads (Beckman Coulter) to remove unreacted peptide and DTT. The exposed cysteines were then reacted with an azido-PEG3-maleimide (BroadPharm, product code: BP-22468). Excess maleimide linker was removed with Agencourt AMPure XP beads and the construct was reacted with the Y adapter overnight at 4°C via click chemistry between BCN and azide. The resulting construct formed by conjugation between the peptide C-terminus with the Y adapter, and the N-terminus with the DNA tail, was purified using Agencourt AMPure XP beads to separate the full construct from the peptide-DNA tail.

The final construct was assessed as set out for Example 1, with exemplary current traces presented in Figure 15. As can be seen, characterisation of the peptide was possible without requiring pre-synthesis of attachment points.

### Example 3

This example compares the disclosed methods in characterising 21-amino acid peptides compared to 10-amino acid peptides.

A polynucleotide-polypeptide conjugate of a 21-amino acid peptide was prepared and analysed according to the method described in Example 1. The current vs time trace obtained with the 21-amino acid construct was compared to that obtained with a 10-amino acid construct from Example 2. The peptide sequences used were GDDDGSASGDDDGSASGDDDG (21aa; SEQ ID NO: 24) and GGSGDDSGSG (10aa; SEQ ID NO: 20).

Data showing current vs time traces for translocation of polynucleotide-peptide conjugates of the 10-amino acid peptide and 21-amino acid peptide are shown in Figure 16. The two traces placed on the same time scale highlight that the current section for the 21-amino acid polypeptide is roughly twice as long as for the 10-amino acid polypeptide.

This example thus confirms that the disclosed methods can be used to characterise polypeptides of varying and extended length.

### Description of the Sequence Listing

SEQ ID NO: 1 shows the amino acid sequence of (hexa-histidine tagged) exonuclease I (EcoExo I) from *E. coli.*
SEQ ID NO: 2 shows the amino acid sequence of the exonuclease III enzyme from *E. coli.*
SEQ ID NO: 3 shows the amino acid sequence of the RecJ enzyme from *T. thermophilus* (TthRecJ-cd).
SEQ ID NO: 4 shows the amino acid sequence of bacteriophage lambda exonuclease. The sequence is one of three identical subunits that assemble into a trimer. (http://www.neb.com/nebecomm/products/productM0262.asp).
SEQ ID NO: 5 shows the amino acid sequence of Phi29 DNA polymerase from *Bacillus subtilis.*
SEQ ID NO: 6 shows the amino acid sequence of Trwc Cba (*Citromicrobium bathyomarinum*) helicase.
SEQ ID NO: 7 shows the amino acid sequence of Hel308 Mbu (*Methanococcoides burtonii*) helicase.
SEQ ID NO: 8 shows the amino acid sequence of the Dda helicase 1993 from Enterobacteria phage T4.
SEQ ID NO: 11 shows the sequence of a first polynucleotide strand used in the production of a Y adapter as described in Example 1 (DNA1-top with a C3 [(OC₃H₆OPO₃)) leader, 3' BCN click attachment, and an enzyme stalling chemistry; 8 = iSp18 [(OCH₂CH₂)₆OPO₃).
SEQ ID NO: 12 shows the sequence of a second polynucleotide strand used in the production of a Y adapter as described in Example 1 (DNA1-back with sidearm for tether).
SEQ ID NO: 13 shows the sequence of a third polynucleotide strand used in the production of a Y adapter as described in Example 1 (DNA1-bottom).
SEQ ID NO: 14 shows the sequence of a first polynucleotide strand used in the production of a dsDNA tail as described in Example 1 (DNA2-top strand, 5' BCN click chemistry).
SEQ ID NO: 15 shows the sequence of a second polynucleotide strand used in the production of a dsDNA tail as described in Example 1 (DNA2-top strand, 5' TCO (orthogonal click chemistry)).
SEQ ID NO: 16 shows the sequence of a polynucleotide strand used in the production of a dsDNA tail as described in Example 2 (DNA2-bottom strand, without sidearm).
SEQ ID NO: 20 shows the amino acid sequence of a first peptide fragment used in the production of a first polynucleotide-polypeptide construct as described in Example 1.
SEQ ID NO: 21 shows the amino acid sequence of a second peptide fragment used in the production of a second polynucleotide-polypeptide construct as described in Example 1.
SEQ ID NO: 22 shows the amino acid sequence of a third peptide fragment used in the production of a third polynucleotide-polypeptide construct as described in Example 1.
SEQ ID NO: 23 shows the amino acid sequence of a peptide fragment used in the production of a polynucleotide-polypeptide construct as described in Example 2.
SEQ ID NO: 24 shows the amino acid sequence of a 21 amino acid peptide fragment used in the production of a polynucleotide-polypeptide construct as described in Example 3.

### SEQUENCE LISTING

**SEQ ID NO: 1 - exonuclease I from *E. coli*** 
MMNDGKQQSTFLFHDYETFGTHPALDRPAQFAAIRTDSEFNVIGEPEVFYCKPADDYLPQ
PGAVLITGITPQEARAKGENEAAFAARIHSLFTVPKTCILGYNNVRFDDEVTRNIFYRNF
YDPYAWSWQHDNSRWDLLDVMRACYALRPEGINWPENDDGLPSFRLEHLTKANGIEHSNA
HDAMADVYATIAMAKLVKTRQPRLFDYLFTHRNKHKLMALIDVPQMKPLVHVSGMFGAWR
GNTSWVAPLAWHPENRNAVIMVDLAGDISPLLELDSDTLRERLYTAKTDLGDNAAVPVKL
VHINKCPVLAQANTLRPEDADRLGINRQHCLDNLKILRENPQVREKVVAIFAEAEPFTPS
DNVDAQLYNGFFSDADRAAMKIVLETEPRNLPALDITFVDKRIEKLLFNYRARNFPGTLD
YAEQQRWLEHRRQVFTPEFLQGYADELQMLVQQYADDKEKVALLKALWQYAEEIVSGSGH
HHHHH
**SEQ ID NO: 2 - exonuclease III enzyme from *E. coli*** 
MKFVSFNINGLRARPHQLEAIVEKHQPDVIGLQETKVHDDMFPLEEVAKLGYNVFYHGQK
GHYGVALLTKETPIAVRRGFPGDDEEAQRRIIMAEIPSLLGNVTVINGYFPQGESRDHPI
KFPAKAQFYQNLQNYLETELKRDNPVLIMGDMNISPTDLDIGIGEENRKRWLRTGKCSFL
PEEREWMDRLMSWGLVDTFRHANPQTADRFSWFDYRSKGFDDNRGLRIDLLLASQPLAEC
CVETGIDYEIRSMEKPSDHAPVWATFRR
**SEQ ID NO: 3 - RecJ enzyme from T. thermophilus** 
MFRRKEDLDPPLALLPLKGLREAAALLEEALRQGKRIRVHGDYDADGLTGTAILVRGLAA
LGADVHPFIPHRLEEGYGVLMERVPEHLEASDLFLTVDCGITNHAELRELLENGVEVIVT
DHHTPGKTPPPGLVVHPALTPDLKEKPTGAGVAFLLLWALHERLGLPPPLEYADLAAVGT
IADVAPLWGWNRALVKEGLARIPASSWVGLRLLAEAVGYTGKAVEVAFRIAPRINAASRL
GEAEKALRLLLTDDAAEAQALVGELHRLNARRQTLEEAMLRKLLPQADPEAKAIVLLDPE
GHPGVMGIVASRILEATLRPVFLVAQGKGTVRSLAPISAVEALRSAEDLLLRYGGHKEAA
GFAMDEALFPAFKARVEAYAARFPDPVREVALLDLLPEPGLLPQVFRELALLEPYGEGNP
EPLFL
**SEQ ID NO: 4 - bacteriophage lambda exonuclease** 
MTPDIILQRTGIDVRAVEQGDDAWHKLRLGVITASEVHNVIAKPRSGKKWPDMKMSYFHT
LLAEVCTGVAPEVNAKALAWGKQYENDARTLFEFTSGVNVTESPIIYRDESMRTACSPDG
LCSDGNGLELKCPFTSRDFMKFRLGGFEAIKSAYMAQVQYSMWVTRKNAWYFANYDPRMK
REGLHYVVIERDEKYMASFDEIVPEFIEKMDEALAEIGFVFGEQWR
**SEQ ID NO: 5 - Phi29 DNA polymerase** 
MKHMPRKMYSCAFETTTKVEDCRVWAYGYMNIEDHSEYKIGNSLDEFMAWVLKVQADLYF
HNLKFDGAFIINWLERNGFKWSADGLPNTYNTIISRMGQWYMIDICLGYKGKRKIHTVIY
DSLKKLPFPVKKIAKDFKLTVLKGDIDYHKERPVGYKITPEEYAYIKNDIQIIAEALLIQ
FKQGLDRMTAGSDSLKGFKDIITTKKFKKVFPTLSLGLDKEVRYAYRGGFTWLNDRFKEK
EIGEGMVFDVNSLYPAQMYSRLLPYGEPIVFEGKYVWDEDYPLHIQHIRCEFELKEGYIP
TIQIKRSRFYKGNEYLKSSGGEIADLWLSNVDLELMKEHYDLYNVEYISGLKFKATTGLF
KDFIDKWTYIKTTSEGAIKQLAKLMLNSLYGKFASNPDVTGKVPYLKENGALGFRLGEEE
TKDPVYTPMGVFITAWARYTTITAAQACYDRIIYCDTDSIHLTGTEIPDVIKDIVDPKKL
GYWAHESTFKRAKYLRQKTYIQDIYMKEVDGKLVEGSPDDYTDIKFSVKCAGMTDKIKKE
VTFENFKVGFSRKMKPKPVQVPGGVVLVDDTFTIKSGGSAWSHPQFEKGGGSGGGSGGSA
WSHPQFEK
**SEQ ID NO: 6 - Trwc Cba helicase** 
MLSVANVRSPSAAASYFASDNYYASADADRSGQWIGDGAKRLGLEGKVEARAFDALLRGE
LPDGSSVGNPGQAHRPGTDLTFSVPKSWSLLALVGKDERIIAAYREAVVEALHWAEKNAA
ETRVVEKGMVVTQATGNLAIGLFQHDTNRNQEPNLHFHAVIANVTQGKDGKWRTLKNDRL
WQLNTTLNSIAMARFRVAVEKLGYEPGPVLKHGNFEARGISREQVMAFSTRRKEVLEARR
GPGLDAGRIAALDTRASKEGIEDRATLSKQWSEAAQSIGLDLKPLVDRARTKALGQGMEA
TRIGSLVERGRAWLSRFAAHVRGDPADPLVPPSVLKQDRQTIAAAQAVASAVRHLSQREA
AFERTALYKAALDFGLPTTIADVEKRTRALVRSGDLIAGKGEHKGWLASRDAVVTEQRIL
SEVAAGKGDSSPAITPQKAAASVQAAALTGQGFRLNEGQLAAARLILISKDRTIAVQGIA
GAGKSSVLKPVAEVLRDEGHPVIGLAIQNTLVQMLERDTGIGSQTLARFLGGWNKLLDDP
GNVALRAEAQASLKDHVLVLDEASMVSNEDKEKLVRLANLAGVHRLVLIGDRKQLGAVDA
GKPFALLQRAGIARAEMATNLRARDPVVREAQAAAQAGDVRKALRHLKSHTVEARGDGAQ
 
VAAETWLALDKETRARTSIYASGRAIRSAVNAAVQQGLLASREIGPAKMKLEVLDRVNTT
REELRHLPAYRAGRVLEVSRKQQALGLFIGEYRVIGQDRKGKLVEVEDKRGKRFRFDPAR
IRAGKGDDNLTLLEPRKLEIHEGDRIRWTRNDHRRGLFNADQARVVEIANGKVTFETSKG
DLVELKKDDPMLKRIDLAYALNVHMAQGLTSDRGIAVMDSRERNLSNQKTFLVTVTRLRD
HLTLVVDSADKLGAAVARNKGEKASAIEVTGSVKPTATKGSGVDQPKSVEANKAEKELTR
SKSKTLDFGI
**SEQ ID NO: 7 - Hel308 Mbu helicase** 
MMIRELDIPRDIIGFYEDSGIKELYPPQAEAIEMGLLEKKNLLAAIPTASGKTLLAELAM
IKAIREGGKALYIVPLRALASEKFERFKELAPFGIKVGISTGDLDSRADWLGVNDIIVAT
SEKTDSLLRNGTSWMDEITTVVVDEIHLLDSKNRGPTLEVTITKLMRLNPDVQVVALSAT
VGNAREMADWLGAALVLSEWRPTDLHEGVLFGDAINFPGSQKKIDRLEKDDAVNLVLDTI
KAEGQCLVFESSRRNCAGFAKTASSKVAKILDNDIMIKLAGIAEEVESTGETDTAIVLAN
CIRKGVAFHHAGLNSNHRKLVENGFRQNLIKVISSTPTLAAGLNLPARRVIIRSYRRFDS
NFGMQPIPVLEYKQMAGRAGRPHLDPYGESVLLAKTYDEFAQLMENYVEADAEDIWSKLG
TENALRTHVLSTIVNGFASTRQELFDFFGATFFAYQQDKWMLEEVINDCLEFLIDKAMVS
ETEDIEDASKLFLRGTRLGSLVSMLYIDPLSGSKIVDGFKDIGKSTGGNMGSLEDDKGDD
ITVTDMTLLHLVCSTPDMRQLYLRNTDYTIVNEYIVAHSDEFHEIPDKLKETDYEWFMGE
VKTAMLLEEWVTEVSAEDITRHFNVGEGDIHALADTSEWLMHAAAKLAELLGVEYSSHAY
SLEKRIRYGSGLDLMELVGIRGVGRVRARKLYNAGFVSVAKLKGADISVLSKLVGPKVAY
NILSGIGVRVNDKHFNSAPISSNTLDTLLDKNQKTFNDFQ
**SEQ ID NO: 8 - Dda helicase** 
MTFDDLTEGQKNAFNIVMKAIKEKKHHVTINGPAGTGKTTLTKFIIEALISTGETGIILA
APTHAAKKILSKLSGKEASTIHSILKINPVTYEENVLFEQKEVPDLAKCRVLICDEVSMY
DRKLFKILLSTIPPWCTIIGIGDNKQIRPVDPGENTAYISPFFTHKDFYQCELTEVKRSN
APIIDVATDVRNGKWIYDKVVDGHGVRGFTGDTALRDFMVNYFSIVKSLDDLFENRVMAF
TNKSVDKLNSIIRKKIFETDKDFIVGEIIVMQEPLFKTYKIDGKPVSEIIFNNGQLVRII
EAEYTSTFVKARGVPGEYLIRHWDLTVETYGDDEYYREKIKIISSDEELYKFNLFLGKTA
ETYKNWNKGGKAPWSDFWDAKSQFSKVKALPASTFHKAQGMSVDRAFIYTPCIHYADVEL
AQQLLYVGVTRGRYDVFYV
**SEQ ID NO: 11** 
333333333333333333333333333333GCAATCGTCGAATGCGTACGTCGTTTTTTTTTT8AATGTACTT
CGTTCAGTTACGTATTGC-BCN
**SEQ ID NO: 12** CGACGTACGCATTCGACGATTGCTTTGAGGCGAGCGGTCAA
**SEQ ID NO: 13** GCAATACGTAACTGAACGAAGT/iBNA-A//iBNA-meC//iBNA-A//iBNAT//3BNA-T/
**SEQ ID NO: 14** BCN-AATGTACTTCGTTCAGTTACGTATTGCTGCTTGGGTGTTTAACC
**SEQ ID NO: 15** TCO-AATGTACTTCGTTCAGTTACGTATTGCTGCTTGGGTGTTTAACC
**SEQ ID NO: 16** GGTTAAACACCCAAGCAGCAATACGTAACTGAACGAAGTACATT
**SEQ ID NO: 20** 
X-GGSGDDSGSG-ed-X
(X = azidoacetyl; ed = ethylene diamine)
**SEQ ID NO: 21** 
X-GGSGRRSGSG-ed-X
(X = azidoacetyl; ed = ethylene diamine)
**SEQ ID NO: 22** 
X-GGSGYYSGSG-ed-X
(X = azidoacetyl; ed = ethylene diamine)
**SEQ ID NO: 23** GGSGDDSGSC
**SEQ ID NO: 24** GDDDGSASGDDDGSASGDDDG

## Claims

1. A method of characterising a target polypeptide, comprising
- conjugating the target polypeptide to a polynucleotide to form a polynucleotide-polypeptide conjugate;
- contacting the conjugate with a polynucleotide-handling protein capable of controlling the movement of the polynucleotide with respect to a nanopore; and
- taking one or more measurements characteristic of the polypeptide as the conjugate moves with respect to the nanopore,
thereby characterising the polypeptide;
wherein the polynucleotide-handling protein is modified by covalently closing a polynucleotide-unbinding opening through which a polynucleotide strand may unbind, thereby preventing the polynucleotide binding protein from disengaging from the conjugate when the polynucleotide-handling protein contacts a portion of the conjugate comprising a polypeptide.

2. A method according to claim 1, wherein the polynucleotide-handling protein remains bound to the conjugate when the portion of the conjugate in contact with the active site of the polynucleotide-handling protein comprises a polypeptide.

3. A method according to claim 1 or 2, wherein the polynucleotide-handling protein is a helicase.

4. A method according to any one of the preceding claims, wherein the conjugate further comprises a leader that facilitates the threading of the conjugate through the nanopore; preferably wherein the leader comprises a polynucleotide or a charged polymer.

5. A method according to any one of the preceding claims, wherein the conjugate comprises a plurality of polypeptide sections and/or a plurality of polynucleotide sections.

6. A method according to any one of the preceding claims, wherein the polypeptide has a length of from 2 to about 50 peptide units, and/or wherein the polynucleotide has a length of from about 10 to about 1000 nucleotides.

7. A method according to any one of the preceding claims, wherein one or more adapters are attached to the polynucleotide in the conjugate.

8. A method according to any one of the preceding claims, wherein the conjugate comprises one or more structures of the form L-{P-N}-P*ₘ*, wherein:
- L is a leader, wherein L is optionally an N moiety;
- P is a polypeptide;
- N comprises a polynucleotide; and
- *m* is 0 or 1;
and wherein the method comprises threading the leader (L) through the nanopore thereby contacting the polypeptide (P) with the nanopore.

9. A method according to any one of the preceding claims, wherein:
i) the polynucleotide-handling protein is located on the *cis* side of the nanopore and the polynucleotide-handling protein controls the movement of the conjugate from the *cis* side of the nanopore to the *trans* side of the nanopore; or
ii) the polynucleotide-handling protein is located on the *trans* side of the nanopore and the polynucleotide-handling protein controls the movement of the conjugate from the *trans* side of the nanopore to the *cis* side of the nanopore.

10. A method according to any one of claims 1 to 9, wherein the conjugate comprises one or more structures of the form L-{P-N}-P*ₘ*, wherein:
- L is a leader, wherein L is optionally an N moiety;
- P is a polypeptide;
- N comprises a polynucleotide; and
- *m* is 0 or 1;
and wherein the method comprises threading the leader (L) through the nanopore thereby contacting the polypeptide (P) with the nanopore; and
i) the polynucleotide-handling protein is located on the *cis* side of the nanopore and the method comprises allowing the polynucleotide-handling protein to control the movement of the polynucleotide moiety (N) from the *cis* side of the nanopore to the *trans* side of the nanopore, thereby controlling the movement of the polypeptide (P) through the nanopore; or
ii) the polynucleotide-handling protein is located on the *trans* side of the nanopore and the method comprises allowing the polynucleotide-handling protein to control the movement of the polynucleotide moiety (N) from the *trans* side of the nanopore to the *cis* side of the nanopore, thereby controlling the movement of the polypeptide (P) through the nanopore.

11. A method according to claim 10, wherein the conjugate comprises one or more structures of the form L-P₁-N-{P-N}*ₙ*-P*ₘ*, wherein:
- *n* is a positive integer;
- L is a leader, wherein L is optionally an N moiety;
- each P, which may be the same or different, is a polypeptide;
- each N, which may be the same or different, comprises a polynucleotide; and
- *m* is 0 or 1;
and wherein the method comprises threading the leader (L) through the nanopore thereby contacting polypeptide (P₁) with the nanopore, and
i) the polynucleotide-handling protein is located on the *cis* side of the nanopore and the method comprises allowing the polynucleotide-handling protein to control the movement of each polynucleotide (N) sequentially from the *cis* side of the nanopore to the *trans* side of the nanopore, thereby controlling the movement of each polypeptide (P) sequentially through the nanopore; or
ii) the polynucleotide-handling protein is located on the *trans* side of the nanopore and the method comprises allowing the polynucleotide-handling protein to control the movement of each polynucleotide (N) sequentially from the *trans* side of the nanopore to the *cis* side of the nanopore, thereby controlling the movement of each polypeptide (P) sequentially through the nanopore

12. A method according to any one of claims 1 to 8, wherein:
i) the polynucleotide-handling protein is located on the *cis* side of the nanopore and the polynucleotide-handling protein controls the movement of the conjugate from the *trans* side of the nanopore to the *cis* side of the nanopore; or
ii) the polynucleotide-handling protein is located on the *trans* side of the nanopore and the polynucleotide-handling protein controls the movement of the conjugate from the *cis* side of the nanopore to the *trans* side of the nanopore.

13. A method according to any one of claims 1 to 8 or 12, wherein the conjugate comprises one or more structures of the form L-{P-N}- P*ₘ*, wherein:
- L is a leader, wherein L is optionally an N moiety;
- P is a polypeptide;
- N comprises a polynucleotide;
- *m* is 0 or 1;
and wherein the method comprises threading the leader (L) through the nanopore thereby contacting the polypeptide (P) with the nanopore, and
i) the polynucleotide-handling protein is located on the *cis* side of the nanopore and the method comprises allowing the polynucleotide-handling protein to control the movement of the polynucleotide (N) from the *trans* side of the nanopore to the *cis* side of the nanopore, thereby controlling the movement of the polypeptide (P) through the nanopore; or
i) the polynucleotide-handling protein is located on the *trans* side of the nanopore and the method comprises allowing the polynucleotide-handling protein to control the movement of the polynucleotide (N) from the *cis* side of the nanopore to the *trans* side of the nanopore, thereby controlling the movement of the polypeptide (P) through the nanopore.

14. A method according to any one of the preceding claims, wherein the one or more measurements are characteristic of one or more characteristics of the polypeptide selected from (i) the length of the polypeptide, (ii) the identity of the polypeptide, (iii) the sequence of the polypeptide, (iv) the secondary structure of the polypeptide and (v) whether or not the polypeptide is modified.

15. A method according to any one of the preceding claims, wherein the step of taking one or more measurements characteristic of the polypeptide as the conjugate moves with respect to the nanopore comprises measuring an ion current flow through the nanopore.

16. A method according to any one of the preceding claims, wherein the nanopore is a transmembrane protein pore;
preferably wherein the transmembrane protein pore is a transmembrane protein pore derived from or based on Msp, α-hemolysin, CsgG, ClyA, Sp1, or FraC.

17. A method according to claim 16, (i) wherein the transmembrane protein pore is a transmembrane protein pore derived from Msp, preferably MspA; or (ii) wherein the transmembrane protein pore is a transmembrane protein pore derived from CsgG.

18. A method according to any one of the preceding claims, wherein the nanopore comprises a constriction region.

19. A kit comprising:
- a nanopore comprising a constriction region, wherein the nanopore is a transmembrane protein pore;
- a polynucleotide comprising a reactive functional group for conjugating to a target polypeptide; and
- a polynucleotide-handling protein capable of controlling the movement of the polynucleotide with respect to the nanopore;
wherein the polynucleotide-handling protein is modified by covalently closing a polynucleotide-unbinding opening through which a polynucleotide strand may unbind, thereby preventing the polynucleotide binding protein to prevent it from disengaging from the conjugate when the polynucleotide-handling protein contacts a portion of the conjugate comprising a polypeptide.

## Patentansprüche

1. Verfahren zum Charakterisieren eines Zielpolypeptids, umfassend:
- Konjugieren des Zielpolypeptids an ein Polynukleotid zur Bildung eines Polynukleotidpolypeptidkonjugats;
- Kontaktieren des Konjugats mit einem Polynukleotidhandhabungsprotein, das in der Lage ist, die Bewegung des Polynukleotids in Bezug auf eine Nanopore zu steuern; und
- Durchführen einer oder mehrerer Messungen, die für das Polypeptid charakteristisch sind, wenn sich das Konjugat in Bezug auf die Nanopore bewegt,
wodurch das Polypeptid charakterisiert wird;
wobei das Polynukleotidhandhabungsprotein durch kovalentes Verschließen einer Polynukleotidunbindungsöffnung, durch die sich ein Polynukleotidstrang lösen kann, modifiziert ist, wodurch das Polynukleotidhandhabungsprotein daran gehindert wird, sich von dem Konjugat zu lösen, wenn das Polynukleotidhandhabungsprotein einen Abschnitt des Konjugats kontaktiert, der ein Polypeptid umfasst.

2. Verfahren nach Anspruch 1, wobei das Polynukleotidhandhabungsprotein an das Konjugat gebunden bleibt, wenn der Abschnitt des Konjugats, der mit der aktiven Stelle des Polynukleotidhandhabungsproteins in Kontakt steht, ein Polypeptid umfasst.

3. Verfahren nach Anspruch 1 oder 2, wobei das Polynukleotidhandhabungsprotein eine Helikase ist.

4. Verfahren nach einem der vorstehenden Ansprüche, wobei das Konjugat ferner einen Leitfaden umfasst, der das Einfädeln des Konjugats durch die Nanopore erleichtert, wobei der Leitfaden vorzugsweise ein Polynukleotid oder ein geladenes Polymer umfasst.

5. Verfahren nach einem der vorstehenden Ansprüche, wobei das Konjugat eine Vielzahl von Polypeptidabschnitten und/oder eine Vielzahl von Polynukleotidabschnitten umfasst.

6. Verfahren nach einem der vorstehenden Ansprüche, wobei das Polypeptid eine Länge von 2 bis etwa 50 Peptideinheiten aufweist, und/oder wobei das Polynucleotid eine Länge von etwa 10 bis etwa 1000 Nucleotiden aufweist.

7. Verfahren nach einem der vorstehenden Ansprüche, wobei ein oder mehrere Adapter an das Polynukleotid in dem Konjugat gebunden sind.

8. Verfahren nach einem der vorstehenden Ansprüche, wobei das Konjugat eine oder mehrere Strukturen der Form L-{P-N}-P*ₘ* umfasst, wobei:
- L ein Leiter ist, wobei L optional eine N-Einheit ist;
- P ein Polypeptid ist;
- N ein Polynukleotid umfasst; und
- *m* 0 oder 1 ist;
und wobei das Verfahren das Einfädeln des Leiters (L) durch die Nanopore umfasst, wodurch das Polypeptid (P) mit der Nanopore in Kontakt gebracht wird.

9. Verfahren nach einem der vorstehenden Ansprüche, wobei:
i) das Polynukleotidhandhabungsprotein sich auf der *cis*-Seite der Nanopore befindet und das Polynukleotidhandhabungsprotein die Bewegung des Konjugats von der *cis*-Seite der Nanopore zur *trans*-Seite der Nanopore steuert; oder
ii) das Polynukleotidhandhabungsprotein sich auf der *trans*-Seite der Nanopore befindet und das Polynukleotidhandhabungsprotein die Bewegung des Konjugats von der trans-Seite der Nanopore zur cis-Seite der Nanopore steuert.

10. Verfahren nach einem der Ansprüche 1 bis 9, wobei das Konjugat eine oder mehrere Strukturen der Form L-{P-N}-P*ₘ* umfasst, wobei:
- L ein Leiter ist, wobei L optional eine N-Einheit ist;
- P ein Polypeptid ist;
- N ein Polynukleotid umfasst; und
- *m* 0 oder 1 ist;
und wobei das Verfahren das Einfädeln des Leiters (L) durch die Nanopore umfasst, wodurch das Polypeptid (P) mit der Nanopore in Kontakt gebracht wird; und
i) das Polynukleotidhandhabungsprotein sich auf der cis-Seite der Nanopore befindet und das Verfahren umfasst, dass dem Polynukleotidhandhabungsprotein ermöglicht wird, die Bewegung der Polynukleotideinheit (N) von der *cis*-Seite der Nanopore zur *trans*-Seite der Nanopore zu steuern, wodurch die Bewegung des Polypeptids (P) durch die Nanopore gesteuert wird; oder
ii) das Polynukleotidhandhabungsprotein sich auf der *trans*-Seite der Nanopore befindet und das Verfahren umfasst, dass die Steuerung der Bewegung der Polynukleotideinheit (N) von der *trans*-Seite der Nanopore zur *cis*-Seite der Nanopore durch das Polynukleotidhandhabungsprotein erfolgt, wodurch die Bewegung des Polypeptids (P) durch die Nanopore gesteuert wird.

11. Verfahren nach Anspruch 10, wobei das Konjugat eine oder mehrere Strukturen der Form L-P₁-N-{P-N}*ₙ*-P*ₘ* umfasst, wobei:
- *n* eine positive ganze Zahl ist;
- L ein Leiter ist, wobei L optional eine N-Einheit ist;
- jedes P, das gleich oder unterschiedlich sein kann, ein Polypeptid ist;
- jedes N, das gleich oder unterschiedlich sein kann, ein Polynukleotid umfasst; und
- *m* 0 oder 1 ist;
und wobei das Verfahren das Einfädeln des Leiters (L) durch die Nanopore umfasst, wodurch das Polypeptid (P₁) mit der Nanopore in Kontakt gebracht wird; und
i) das Polynukleotidhandhabungsprotein sich auf der *cis*-Seite der Nanopore befindet und das Verfahren umfasst, dass dem Polynukleotidhandhabungsprotein ermöglicht wird, die Bewegung jedes Polynukleotids (N) sequentiell von der cis-Seite der Nanopore zur *trans-*Seite der Nanopore zu steuern, wodurch die Bewegung jedes Polypeptids (P) sequentiell durch die Nanopore gesteuert wird; oder
ii) das Polynukleotidhandhabungsprotein sich auf der *trans*-Seite der Nanopore befindet und das Verfahren umfasst, dass das Polynukleotidhandhabungsprotein die Bewegung jedes Polynukleotids (N) sequenziell von der *trans*-Seite der Nanopore zur *cis*-Seite der Nanopore steuert, wodurch die Bewegung jedes Polypeptids (P) sequenziell durch die Nanopore gesteuert wird.

12. Verfahren nach einem der Ansprüche 1 bis 8, wobei:
i) das Polynukleotidhandhabungsprotein sich auf der *cis*-Seite der Nanopore befindet und das Polynukleotidhandhabungsprotein die Bewegung des Konjugats von der *trans*-Seite der Nanopore zur *cis*-Seite der Nanopore steuert; oder
ii) das Polynukleotidhandhabungsprotein sich auf der *trans*-Seite der Nanopore befindet und das Polynukleotidhandhabungsprotein die Bewegung des Konjugats von der *cis*-Seite der Nanopore zur *trans*-Seite der Nanopore steuert.

13. Verfahren nach einem der Ansprüche 1 bis 8 oder 12, wobei das Konjugat eine oder mehrere Strukturen der Form L-{P-N}-P*ₘ* umfasst, wobei:
- L ein Leiter ist, wobei L optional eine N-Einheit ist;
- P ein Polypeptid ist;
- N ein Polynukleotid umfasst;
- *m* 0 oder 1 ist;
und wobei das Verfahren das Einfädeln des Leiters (L) durch die Nanopore umfasst, wodurch das Polypeptid (P) mit der Nanopore in Kontakt gebracht wird und
i) das Polynukleotidhandhabungsprotein sich auf der cis-Seite der Nanopore befindet und das Verfahren umfasst, dass dem Polynukleotidhandhabungsprotein ermöglicht wird, die Bewegung des Polynukleotids (N) von der *trans*-Seite der Nanopore zur *cis*-Seite der Nanopore zu steuern, wodurch die Bewegung des Polypeptids (P) durch die Nanopore gesteuert wird; oder
i) das Polynukleotidhandhabungsprotein sich auf der *trans*-Seite der Nanopore befindet und das Verfahren umfasst, dass dem Polynukleotidhandhabungsprotein ermöglicht wird, die Bewegung des Polynukleotids (N) von der *cis*-Seite der Nanopore zur *trans*-Seite der Nanopore zu steuern, wodurch die Bewegung des Polypeptids (P) durch die Nanopore gesteuert wird.

14. Verfahren nach einem der vorstehenden Ansprüche, wobei die eine oder mehreren Messungen Eigenschaft für eine oder mehrere Eigenschaften des Polypeptids sind, ausgewählt aus (i) der Länge des Polypeptids, (ii) der Identität des Polypeptids, (iii) der Sequenz des Polypeptids, (iv) der Sekundärstruktur des Polypeptids und (v) der Frage, ob das Polypeptid modifiziert ist oder nicht.

15. Verfahren nach einem der vorstehenden Ansprüche, wobei der Schritt des Durchführens einer oder mehrerer Messungen, die für das Polypeptid eine Eigenschaft sind, während sich das Konjugat relativ zu der Nanopore bewegt, das Messen eines Ionenstroms durch die Nanopore umfasst.

16. Verfahren nach einem der vorstehenden Ansprüche, wobei die Nanopore eine Transmembranproteinpore ist; vorzugsweise wobei das Transmembranproteinporen ein Transmembranproteinporen ist, das von Msp, α-Hämolysin, CsgG, ClyA, Sp1 oder FraC abgeleitet ist oder darauf basiert.

17. Verfahren nach Anspruch 16, (i) wobei die Transmembranproteinpore eine Transmembranproteinpore ist, die von Msp, vorzugsweise MspA, abgeleitet ist; oder (ii) wobei die Transmembranproteinpore eine Transmembranproteinpore ist, die von CsgG abgeleitet ist.

18. Verfahren nach einem der vorstehenden Ansprüche, wobei die Nanopore eine Verengungsregion umfasst.

19. Kit, umfassend:
- eine Nanopore, umfassend eine Verengungsregion, wobei die Nanopore eine Transmembranproteinpore ist;
- ein Polynucleotid, umfassend eine reaktive funktionelle Gruppe zur Konjugation mit einem Zielpolypeptid; und
- ein Polynukleotidhandhabungsprotein, das in der Lage ist, die Bewegung des Polynukleotids in Bezug auf die Nanopore zu steuern;
wobei das Polynukleotidhandhabungsprotein durch kovalentes Verschließen einer Polynukleotidunbindungsöffnung, durch die sich ein Polynukleotidstrang lösen kann, modifiziert wird, wodurch verhindert wird, dass sich das Polynukleotidbindungsprotein vom Konjugat löst, wenn das Polynukleotidhandhabungsprotein mit einem Abschnitt des Konjugats in Kontakt kommt, der ein Polypeptid umfasst.

## Revendications

1. Procédé de caractérisation d'un polypeptide cible, comprenant
- la conjugaison du polypeptide cible à un polynucléotide pour former un conjugué polynucléotide-polypeptide ;
- la mise en contact du conjugué avec une protéine de manipulation de polynucléotides capable de commander le mouvement du polynucléotide par rapport à un nanopore ; et
- la prise d'une ou de plusieurs mesures caractéristiques du polypeptide lorsque le conjugué se déplace par rapport au nanopore,
caractérisant ainsi le polypeptide ;
dans lequel la protéine de manipulation de polynucléotides est modifiée en fermant de manière covalente une ouverture de dissociation de polynucléotides à travers laquelle un brin de polynucléotide peut se dissocier, empêchant ainsi la protéine de liaison de polynucléotides de se désengager du conjugué lorsque la protéine de manipulation de polynucléotides entre en contact avec une partie du conjugué comprenant un polypeptide.

2. Procédé selon la revendication 1, dans lequel la protéine de manipulation de polynucléotides reste liée au conjugué lorsque la partie du conjugué en contact avec le site actif de la protéine de manipulation de polynucléotides comprend un polypeptide.

3. Procédé selon la revendication 1 ou 2, dans lequel la protéine de manipulation de polynucléotides est une hélicase.

4. Procédé selon l'une quelconque des revendications précédentes, dans lequel le conjugué comprend en outre un groupement leader qui facilite l'enfilage du conjugué à travers le nanopore ; de préférence dans lequel le groupement leader comprend un polynucléotide ou un polymère chargé.

5. Procédé selon l'une quelconque des revendications précédentes, dans lequel le conjugué comprend une pluralité de sections polypeptidiques et/ou une pluralité de sections polynucléotidiques.

6. Procédé selon l'une quelconque des revendications précédentes, dans lequel le polypeptide a une longueur de 2 à environ 50 unités peptidiques, et/ou dans lequel le polynucléotide a une longueur d'environ 10 à environ 1000 nucléotides.

7. Procédé selon l'une quelconque des revendications précédentes, dans lequel un ou plusieurs adaptateurs sont attachés au polynucléotide dans le conjugué.

8. Procédé selon l'une quelconque des revendications précédentes, dans lequel le conjugué comprend une ou plusieurs structures de la forme L-{PN}-Pₘ, dans lequel :
- L est un groupement leader, dans lequel L est éventuellement une fraction N ;
- P est un polypeptide ;
- N comprend un polynucléotide ; et
- m vaut 0 ou 1 ;
et dans lequel le procédé comprend l'enfilage du groupement leader (L) à travers le nanopore, mettant ainsi en contact le polypeptide (P) avec le nanopore.

9. Procédé selon l'une quelconque des revendications précédentes, dans lequel :
i) la protéine de manipulation de polynucléotides est située sur le côté cis du nanopore et la protéine de manipulation de polynucléotides commande le mouvement du conjugué du côté cis du nanopore au côté trans du nanopore ; ou
ii) la protéine de manipulation de polynucléotides est située sur le côté trans du nanopore et la protéine de manipulation de polynucléotides commande le mouvement du conjugué du côté trans du nanopore au côté cis du nanopore.

10. Procédé selon l'une quelconque des revendications 1 à 9, dans lequel le conjugué comprend une ou plusieurs structures de la forme L-{P-N}-Pₘ, dans lequel :
- L est un groupement leader, dans lequel L est éventuellement une fraction N ;
- P est un polypeptide ;
- N comprend un polynucléotide ; et
- m vaut 0 ou 1 ;
et dans lequel le procédé comprend l'enfilage du groupement leader (L) à travers le nanopore, mettant ainsi en contact le polypeptide (P) avec le nanopore ; et
i) la protéine de manipulation de polynucléotides est située sur le côté cis du nanopore et le procédé comprend le fait de permettre à la protéine de manipulation de polynucléotides de commander le mouvement de la fraction polynucléotidique (N) du côté cis du nanopore au côté trans du nanopore, commandant ainsi le mouvement du polypeptide (P) à travers le nanopore ; ou
ii) la protéine de manipulation de polynucléotides est située sur le côté trans du nanopore et le procédé comprend le fait de permettre à la protéine de manipulation de polynucléotides de commander le mouvement de la fraction polynucléotidique (N) du côté trans du nanopore au côté cis du nanopore, commandant ainsi le mouvement du polypeptide (P) à travers le nanopore.

11. Procédé selon la revendication 10, dans lequel le conjugué comprend une ou plusieurs structures de la forme L-P₁-N-{P-N}ₙ-Pₘ, dans lequel :
- n est un entier positif ;
- L est un groupement leader, dans lequel L est éventuellement une fraction N ;
- chaque P, qui peut être identique ou différent, est un polypeptide ;
- chaque N, qui peut être identique ou différent, comprend un polynucléotide ; et
- m vaut 0 ou 1 ;
et dans lequel le procédé comprend l'enfilage du groupement leader (L) à travers le nanopore, mettant ainsi en contact le polypeptide (P₁) avec le nanopore, et
i) la protéine de manipulation de polynucléotides est située sur le côté cis du nanopore et le procédé comprend le fait de permettre à la protéine de manipulation de polynucléotides de commander le mouvement de chaque polynucléotide (N) séquentiellement du côté cis du nanopore au côté trans du nanopore, commandant ainsi le mouvement de chaque polypeptide (P) séquentiellement à travers le nanopore ; ou
ii) la protéine de manipulation de polynucléotides est située sur le côté trans du nanopore et le procédé comprend le fait de permettre à la protéine de manipulation de polynucléotides de commander le mouvement de chaque polynucléotide (N) séquentiellement du côté trans du nanopore au côté cis du nanopore, commandant ainsi le mouvement de chaque polypeptide (P) séquentiellement à travers le nanopore.

12. Procédé selon l'une quelconque des revendications 1 à 8, dans lequel :
i) la protéine de manipulation de polynucléotides est située sur le côté cis du nanopore et la protéine de manipulation de polynucléotides commande le mouvement du conjugué du côté trans du nanopore au côté cis du nanopore ; ou
ii) la protéine de manipulation de polynucléotides est située sur le côté trans du nanopore et la protéine de manipulation de polynucléotides commande le mouvement du conjugué du côté cis du nanopore au côté trans du nanopore.

13. Procédé selon l'une quelconque des revendications 1 à 8 ou 12, dans lequel le conjugué comprend une ou plusieurs structures de la forme L-{P-N}-Pₘ, dans lequel :
- L est un groupement leader, dans lequel L est éventuellement une fraction N ;
- P est un polypeptide ;
- N comprend un polynucléotide ;
- m vaut 0 ou 1 ;
et dans lequel le procédé comprend l'enfilage du groupement leader (L) à travers le nanopore, mettant ainsi en contact le polypeptide (P) avec le nanopore, et
i) la protéine de manipulation de polynucléotides est située sur le côté cis du nanopore et le procédé comprend le fait de permettre à la protéine de manipulation de polynucléotides de commander le mouvement du polynucleotide (N) du côté trans du nanopore au côté cis du nanopore, commandant ainsi le mouvement du polypeptide (P) à travers le nanopore ; ou
i) la protéine de manipulation de polynucléotides est située sur le côté trans du nanopore et le procédé comprend le fait de permettre à la protéine de manipulation de polynucléotides de commander le mouvement du polynucleotide (N) du côté cis du nanopore au côté trans du nanopore, commandant ainsi le mouvement du polypeptide (P) à travers le nanopore.

14. Procédé selon l'une quelconque des revendications précédentes, dans lequel les une ou plusieurs mesures sont caractéristiques d'une ou de plusieurs caractéristiques du polypeptide choisies parmi (i) la longueur du polypeptide, (ii) l'identité du polypeptide, (iii) la séquence du polypeptide, (iv) la structure secondaire du polypeptide et (v) si le polypeptide est modifié ou non.

15. Procédé selon l'une quelconque des revendications précédentes, dans lequel l'étape de prise d'une ou de plusieurs mesures caractéristiques du polypeptide lorsque le conjugué se déplace par rapport au nanopore comprend la mesure d'un flux de courant ionique à travers le nanopore.

16. Procédé selon l'une quelconque des revendications précédentes, dans lequel le nanopore est un pore protéique transmembranaire ;
de préférence dans lequel le pore protéique transmembranaire est un pore protéique transmembranaire dérivé ou basé sur Msp, l'α-hémolysine, CsgG, ClyA, Sp1 ou FraC.

17. Procédé selon la revendication 16, (i) dans lequel le pore protéique transmembranaire est un pore protéique transmembranaire dérivé de Msp, de préférence MspA ; ou (ii) dans lequel le pore protéique transmembranaire est un pore protéique transmembranaire dérivé de CsgG.

18. Procédé selon l'une quelconque des revendications précédentes, dans lequel le nanopore comprend une région de constriction.

19. Kit comprenant :
- un nanopore comprenant une région de constriction, dans lequel le nanopore est un pore protéique transmembranaire ;
- un polynucléotide comprenant un groupe fonctionnel réactif pour la conjugaison à un polypeptide cible ; et
- une protéine de manipulation de polynucléotides capable de commander le mouvement du polynucléotide par rapport au nanopore ;
dans lequel la protéine de manipulation de polynucléotides est modifiée en fermant de manière covalente une ouverture de dissociation de polynucléotides à travers laquelle un brin de polynucléotide peut se dissocier, empêchant ainsi la protéine de liaison de polynucléotides de se désengager du conjugué lorsque la protéine de manipulation de polynucléotides entre en contact avec une partie du conjugué comprenant un polypeptide.
